(19) Europäisches Patentamt
European Patent Office
Office européen des brevets

(11) EP 3 959 293 B1

(12) EUROPEAN PATENT SPECIFICATION

(45) Date of publication and mention of the grant of the patent:
**13.11.2024 Bulletin 2024/46**

(21) Application number: **20810806.8**

(22) Date of filing: **19.05.2020**

(51) International Patent Classification (IPC):
***C09K 19/54*** (2006.01) ***G02F 1/1335*** (2006.01)
***H05B 33/14*** (2006.01) ***C09K 11/06*** (2006.01)
***C09K 19/60*** (2006.01)

(52) Cooperative Patent Classification (CPC):
**C09K 19/542; C09K 11/06; C09K 19/60**

(86) International application number:
**PCT/US2020/033606**

(87) International publication number:
**WO 2020/236818 (26.11.2020 Gazette 2020/48)**

# (54) NARROW EMISSION DYES, COMPOSITIONS COMPRISING SAME, AND METHODS FOR MAKING AND USING SAME

FARBSTOFFE MIT GERINGER EMISSION, ZUSAMMENSETZUNGEN DAMIT UND VERFAHREN ZU IHRER HERSTELLUNG UND VERWENDUNG

COLORANTS À ÉMISSION ÉTROITE, COMPOSITIONS CONTENANT CES DERNIERS, ET MÉTHODES DE PRODUCTION ET D’UTILISATION CORRESPONDANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.05.2019 US 201962850442 P**

(43) Date of publication of application:
**02.03.2022 Bulletin 2022/09**

(73) Proprietor: **NIRvana Sciences Inc.**
**Durham NC 27709 (US)**

(72) Inventors:
- **MACNEVIN, Christopher, J.**
  **Durham, NC 27709 (US)**
- **PITNER, James, Bruce**
  **Durham, NC 27709 (US)**
- **CHEN, Chih-Yuan**
  **Durham, NC 27709 (US)**

(74) Representative: **Isarpatent**
**Patent- und Rechtsanwälte Barth**
**Charles Hassa Peckmann & Partner mbB**
**Friedrichstrasse 31**
**80801 München (DE)**

(56) References cited:
**WO-A1-2017/214637**

- **KEE HOOI LING ET AL: "Effects of Substituents on Synthetic Analogs of Chlorophylls. Part 1: Synthesis, Vibrational Properties and Excited-state Decay Characteristics", PHOTOCHEMISTRY AND PHOTOBIOLOGY, vol. 83, no. 5, 1 September 2007 (2007-09-01), US, pages 1110 - 1124, XP055979817, ISSN: 0031-8655, DOI: 10.1111/j.1751-1097.2007.00150.x**
- **KEE HOOI LING ET AL: "Chlorin-Bacteriochlorin Energy-transfer Dyads as Prototypes for Near-infrared Molecular Imaging Probes: Controlling Charge-transfer and Fluorescence Properties in Polar Media", PHOTOCHEMISTRY AND PHOTOBIOLOGY, vol. 85, no. 4, 1 July 2009 (2009-07-01), US, pages 909 - 920, XP055979811, ISSN: 0031-8655, DOI: 10.1111/j.1751-1097.2008.00532.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 959 293 B1

- LIU SIJIA ET AL: "Single-Fluorophore Single-Chain Nanoparticle Undergoes Fluorophore-Driven Assembly with Fluorescence Features Retained in Physiological Milieu", ACS APPLIED POLYMER MATERIALS, vol. 3, no. 4, 10 March 2007 (2007-03-10), pages 1767 - 1776, XP055979806, ISSN: 2637-6105, Retrieved from the Internet <URL:https://pubs.acs.org/doi/pdf/10.1021/acsapm.0c01313> DOI: 10.1021/acsapm.0c01313
- DATABASE PubChem Compound 14 November 2007 (2007-11-14), "10-Phynyl-18, 18-Dimethyl-17, 18-Dihydro-21H, 23H-Porphyrin", XP055761879, retrieved from NCBI Database accession no. 17747864
- LINDSEY JONATHAN S.: "De Novo Synthesis of Gem-Dialkyl Chlorophyll Analogues for Probing and Emulating Our Green World", CHEM REV., vol. 115, no. 13, 2015, pages 6534 - 6620, XP055761865
- DATABASE PubChem Compound 1 December 2016 (2016-12-01), "2,2-Dimethyl-10-[4-(Methoxycarbonyl)Phenyl]-17-(Phenylethynyl)-2,3-Dihydro-21H,23H-Porphyrin", XP055761883, retrieved from NCBI Database accession no. 122395954

## Description

TECHNICAL FIELD

The presently disclosed subject matter relates generally to chlorin derivatives, in some embodiments water-soluble chlorin derivatives, with narrow emission wavelengths, to conjugates thereof, and to methods of making and using the chlorin derivatives and conjugates thereof.

BACKGROUND

A large and growing number of applications require fluorescent dyes that are water-soluble and suited for conjugation to other substances, ranging from nanoparticles to biological targeting agents. Such applications encompass, for example, flow cytometry, cellular and whole-organism imaging, sensing, and photodynamic therapy. Chlorin molecules are particularly of interest in these applications, as chlorins typically emit in the red (i.e., 600-700 nm) spectral range, making them one of the few chromophores available for photochemical studies in the red spectral region.

The success of the applications described above relies on a host of factors, including (1) significant solubility in aqueous saline solutions, thereby avoiding intermolecular aggregation (and excited-state quenching), (2) minimal non-specific binding to cellular components, (3) incorporation of a single reactive group for conjugation, thereby avoiding crosslinking and mixtures of products, and (4) robust synthesis affording ample quantities for experimentation. However, the large hydrophobic face of chlorins can present a challenge to water-solubilization.

Accordingly, there is an ongoing need to provide additional chlorin derivatives, including but not limited to those with improved aqueous solubility (e.g., aqueous solubility above 1 mg/mL), particularly those that can also be easily conjugated to a wide variety of substances and/or that are characterized by narrow absorption and emission bands.

KEE HOOI LING ET AL, PHOTOCHEMISTRY AND PHOTOBIOLOGY (2007), vol. 83, no. 5, pages 1110 - 1124, investigates the effects of substituents on the spectra of chlorins.

KEE HOOI LING ET AL, PHOTOCHEMISTRY AND PHOTOBIOLOGY, (2009), vol. 85, no. 4, pages 909 - 920, describes chlorin-bacteriochlorin energy-transfer dyads as prototypes for near-infrared molecular imaging probes.

WO 2017/214637 A1 describes compositions comprising one or more porphyrinic macrocycles, optionally one or more chlorins, bacteriochlorins, and/or isobacteriochlorins, and methods for using the same to label and/or otherwise detect biological molecules and/or cells.

SUMMARY

In some embodiments, the presently disclosed subject matter provides compounds of Formula (I):

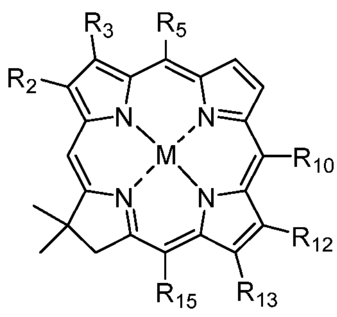

(I)

,

wherein M is a metal or is -H, -H; $R_5$, and $R_{15}$ are independently selected from H, alkoxy, and a linker group having the formula $-L_1-(X_1-L_2)_p-G$, wherein p is 0 or 1; $L_1$ is alkylidene; $X_1$ is -C(=O)NH- or -NHC(=O)-; $L_2$ is $-(CH_2CH_2O)_q$-alkylene wherein q is an integer between 1 and 24, alkylene, or substituted alkylene, optionally wherein substituted alkylene is alkylene substituted by a one or more groups comprising a polyoxyethylene chain and/or an amide group; and G is a bioconjugatable group; $R_{10}$ is

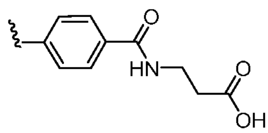

;

and $R_2$, $R_3$, $R_{12}$, and $R_{13}$ are independently selected from H, cyano, halo, perhaloalkyl, sulfonate, sulfonamide, ester, carboxylic acid, formyl, acetyl, a linker group having the formula $-L_1-(X_1-L_2)_p-G$, and a solubilizing group, wherein the solubilizing group is selected from -aryl-$(R_s)_w$ and -alkynyl-aryl-$(R_s)_w$, wherein w is an integer between 0 and 5 inclusive, and $R_S$ is a group having the formula $-X_2-(L_3)_z-R_{17}$, wherein z is 0 or 1; $X_2$ is $-CH_2NHC(=O)-$, - C(=O)NH-alkylene-NH-, or triazolyl; $L_3$ is -C(=O)-alkylene-C(=O)-NH-, and $R_{17}$ is selected from $-(C_2H_4O)_m-R_{18}$, $-C(=O)C_2H_4-(OC_2H_4)_mOR_{18}$, and $-(C_2H_4O)_n-C_2H_4-C(=O)NH-C(R_{19})_3$, wherein m is an integer of 4 or more, optionally wherein m is an integer of 8 or more; n is an integer between 1 and 5; $R_{18}$ is loweralkyl, optionally methyl; and $R_{19}$ is $-CH_2O-C_2H_4-C(=O)NH-(C_2H_4O)_mR_{18}$; subject to the proviso that at least one of $R_2$, $R_3$, $R_{12}$, and $R_{13}$ is -aryl-$(R_s)_w$ or -alkynyl-aryl-$(R_s)_w$.

In some embodiments, $R_5$, and $R_{15}$ are independently selected from H, methoxy, and a linker group having the formula $-L_1-(X_1-L_2)_p-G$.

In some embodiments, $R_3$ and $R_{13}$ are each independently -aryl-$(R_s)_w$, optionally wherein $R_3$ and $R_{13}$ are each -phenyl-$(R_s)_2$. In some embodiments, each $R_s$ has a formula - $X_2-(L_3)_z-R_{17}$, wherein z is 0, $X_2$ is $-CH_2NHC(=O)-$, and $R_{17}$ is $-(C_2H_4O)_m-R_{18}$, wherein m is an integer between 12 and 24. In some embodiments, $R_3$ and $R_{13}$ are each:

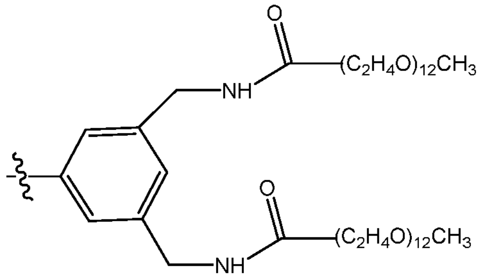

.

In some embodiments, the compound is:

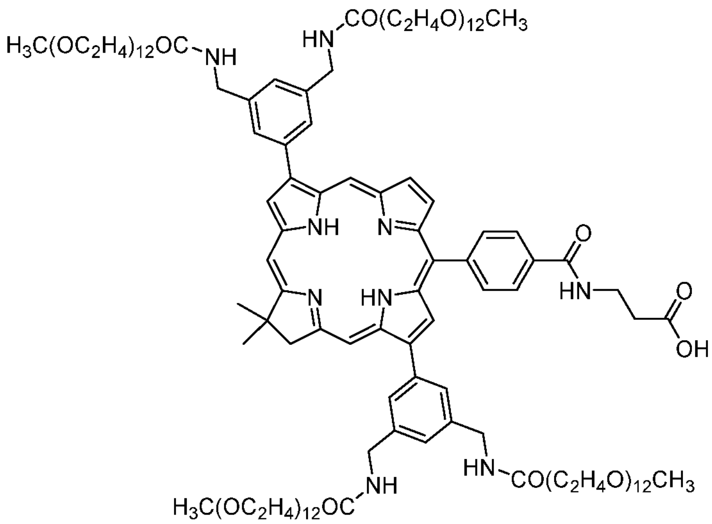

.

In some embodiments, the presently disclosed subject matter provides compositions comprising a covalent conjugate formed between (a) a compound of Formula (I), subject to the proviso that at least one of $R_2$, $R_3$, $R_5$, $R_{10}$, $R_{12}$, $R_{13}$, and $R_{15}$ is a linker group; and (b) one or more of a group chosen from a small molecule, an antigen, a microparticle, a nanoparticle, a polymer, a peptide, a protein, an antibody or an antibody fragment, a nucleic acid, a hormone, and a growth factor.

In some embodiments, the presently disclosed subject matter provides pharmaceutical compositions comprising a compound of Formula (I) or a conjugate formed between a compound of Formula (I), subject to the proviso that at least one of $R_2$, $R_3$, $R_5$, $R_{10}$, $R_{12}$, $R_{13}$, and $R_{15}$ is a linker group; and one or more of the group comprising a small molecule, an antigen, a microparticle, a nanoparticle, a polymer, a peptide, a protein, an antibody or an antibody fragment, a nucleic acid, a hormone, and a growth factor; and a pharmaceutically acceptable carrier.

In some embodiments, the presently disclosed subject matter provides methods for detecting a target. In some embodiments, the target is a compound, cell, or particle, and further wherein the method comprises labelling the target with a conjugate formed between a compound of Formula (I), subject to the proviso that at least one of $R_2$, $R_3$, $R_5$, $R_{10}$,

$R_{12}$, $R_{13}$, and $R_{15}$ is a linker group; and one or more of the group comprising a small molecule, an antigen, a microparticle, a nanoparticle, a polymer, a peptide, a protein, an antibody or an antibody fragment, a nucleic acid, a hormone, and a growth factor. In some embodiments, the method comprises the use of flow cytometry.

In some embodiments, the presently disclosed subject matter provides methods for imaging cells, tissues, and/or organisms. In some embodiments, the methods comprise the use of a compound of Formula (I) or a conjugate formed between a compound of Formula (I), subject to the proviso that at least one of $R_2$, $R_3$, $R_5$, $R_{10}$, $R_{12}$, $R_{13}$, and $R_{15}$ is a linker group; and one or more of the group comprising a small molecule, an antigen, a microparticle, a nanoparticle, a polymer, a peptide, a protein, an antibody or an antibody fragment, a nucleic acid, a hormone, and a growth factor.

In some embodiments, the presently disclosed subject matter provides methods for treating diseases in subjects in need of treatment thereof. In some embodiments, the methods comprise administering to a subject a compound of Formula (I), a conjugate formed between a compound of Formula (I), subject to the proviso that at least one of $R_2$, $R_3$, $R_5$, $R_{10}$, $R_{12}$, $R_{13}$, and $R_{15}$ is a linker group; and one or more of the group comprising a small molecule, an antigen, a microparticle, a nanoparticle, a polymer, a peptide, a protein, an antibody or an antibody fragment, a nucleic acid, a hormone, and a growth factor, or a pharmaceutical composition of said compound or conjugate; and irradiating at least a portion of the subject with light, optionally wherein said disease is a hyperproliferative disease, further optionally wherein the disease is cancer.

In some embodiments, the presently disclosed subject matter provides compounds of Formula (I) having a solubility of above about 1 mg/ml in an aqueous solution, optionally having a solubility of about 2.5 mg/ml or more in an aqueous solution; further optionally having a solubility of about 10 mg/ml or more in an aqueous solution.

In some embodiments, the presently disclosed subject matter provides methods for preparing synthetic intermediates of the compounds of Formula (I):

(I)

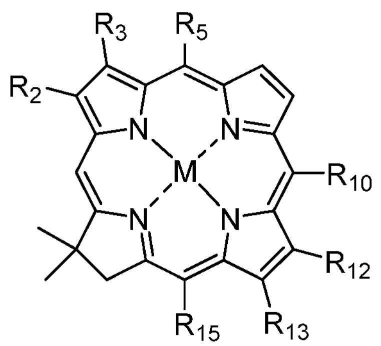

,

wherein M is a metal or is two hydrogens (i.e., -H, -H); $R_5$, and $R_{15}$ are independently selected from H, alkoxy, and a linker group having the formula $-L_1-(X_1-L_2)_p-G$, wherein p is 0 or 1; $L_1$ is alkylidene; $X_1$ is -C(=O)NH- or -NHC(=O)-; $L_2$ is $-(CH_2CH_2O)_q$-alkylene, wherein q is an integer between 1 and 24, alkylene, or substituted alkylene, optionally wherein the substituted alkylene is an alkylene substituted by a one or more groups comprising a polyoxyethylene chain and/or an amide group; and G is a bioconjugatable group; $R_{10}$ is

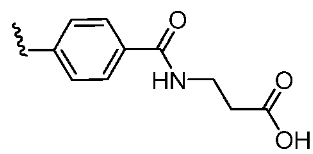

;

and $R_2$, $R_3$, $R_{12}$, and $R_{13}$ are independently selected from H, halo, cyano, perhaloalkyl, sulfonate, sulfonamide, ester, carboxylic acid, formyl, acetyl, a linker group having the formula $-L_1-(X_1-L_2)_p-G$, and a solubilizing group, wherein the solubilizing group is selected from $-aryl-(R_s)_w$ and $-alkynyl-aryl-(R_s)_w$, wherein w is an integer between 0 and 5 inclusive, and $R_S$ is a group having the formula $-X_2-(L_3)_z-R_{17}$, wherein: z is 0 or 1; $X_2$ is $-CH_2NHC(=O)-$, -C(=O)NH-alkylene-NH-, or triazolyl; $L_3$ is -C(=O)-alkylene-C(=O)-NH-, and $R_{17}$ is selected from $-(C_2H_4O)_m-R_{18}$, $-C(=O)C_2H_4-(OC_2H_4)_mOR_{18}$, and $-(C_2H_4O)_n-C_2H_4-C(=O)NH-C(R_{19})_3$, wherein m is an integer of 4 or more, optionally wherein m is an integer of 8 or more; n is an integer between 1 and 5; $R_{18}$ is loweralkyl, optionally methyl; and $R_{19}$ is $-CH_2O-C_2H_4-C(=O)NH-(C_2H_4O)_mR_{18}$; subject to the proviso that at least one of $R_2$, $R_3$, $R_{12}$, and $R_{13}$ is $-aryl-(R_s)_w$ or $-alkynyl-aryl-(R_s)_w$; wherein the method comprises (a) providing a compound having the formula (I'):

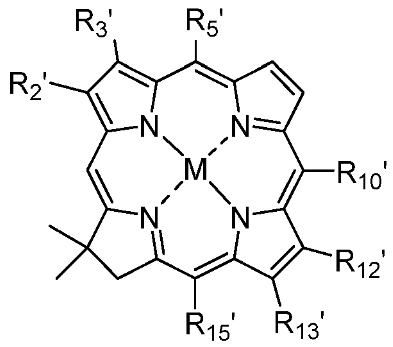

,

wherein M is a metal or is -H, -H; $R_5$', and $R_{15}$' are independently selected from H, alkoxy,

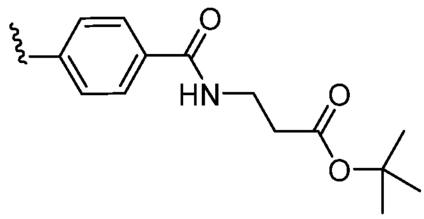, and 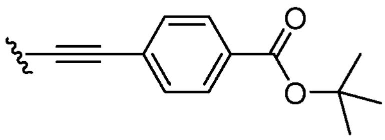;

$R_{10}$' is

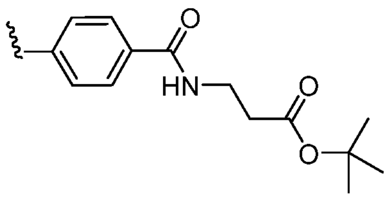;

and

$R_2$', $R_3$', $R_{12}$', and $R_{13}$' are each independently selected from H, ester, carboxylic acid, formyl, acetyl,

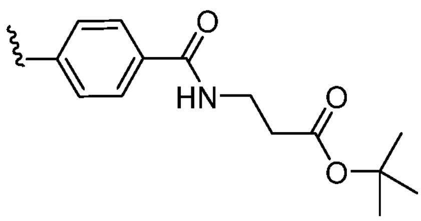, 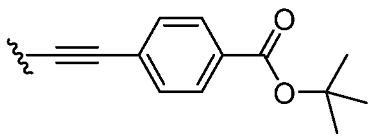,

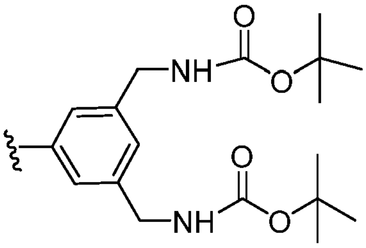, and 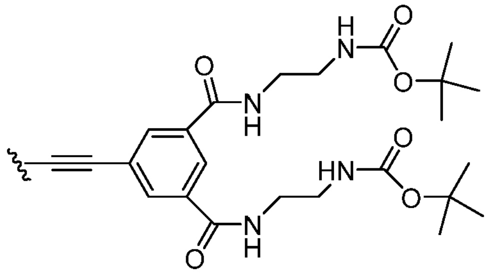;

subject to the proviso that at least one of $R_2$', $R_3$', $R_{12}$', and $R_{13}$' is:

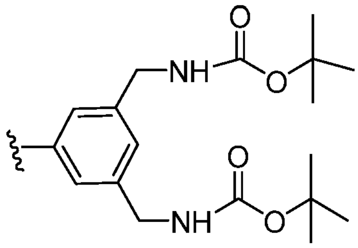 or 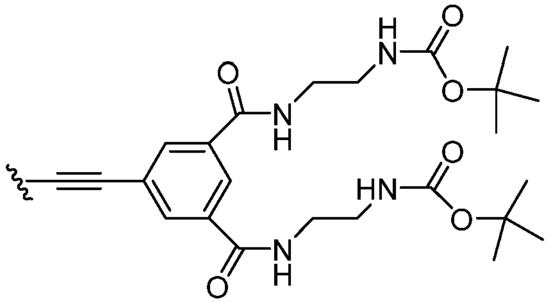 ;

and

(b) contacting the compound provided in step (a) with a solution comprising 4 molar (M) HCl in dioxane to provide a compound of the formula (I"):

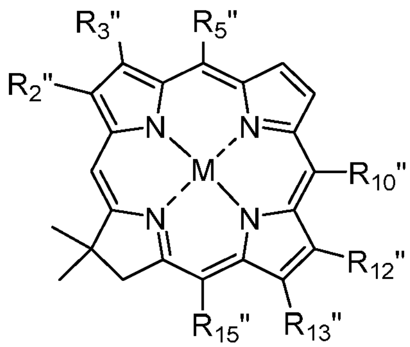 ,

wherein M is a metal or is -H, -H; $R_5$", and $R_{15}$" are independently selected from H, alkoxy,

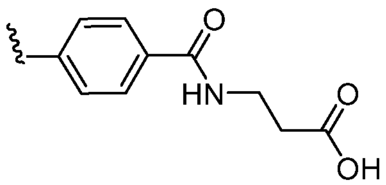 , and 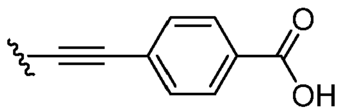 ;

$R_{10}$" is

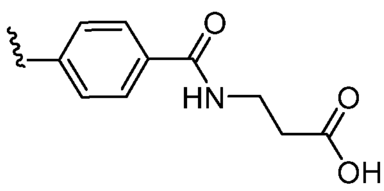 ; and

$R_2$", $R_3$", $R_{12}$", and $R_{13}$" are independently selected from H, ester, carboxylic acid, formyl, acetyl,

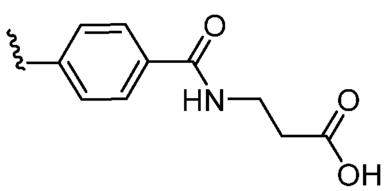 , 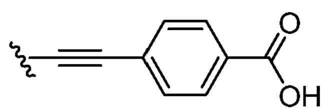 ,

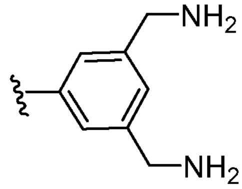

and

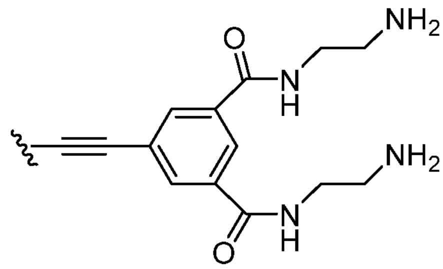

;

subject to the proviso that at least one of $R_2$", $R_3$", $R_{12}$", and $R_{13}$" is

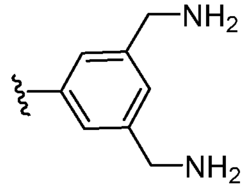

or

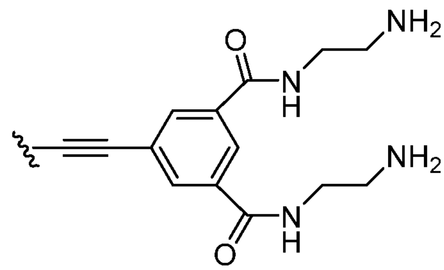

.

In some embodiments, a compound of the presently disclosed subject matter is:

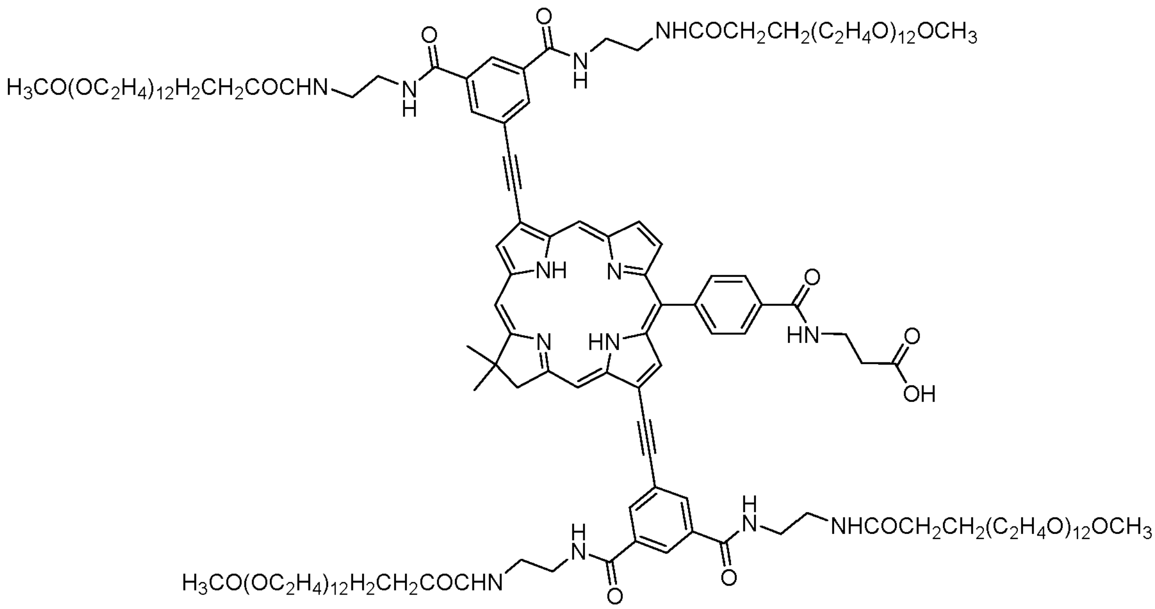

or a pharmaceutical composition comprising the same, or conjugate thereof. In some embodiments, the compound is conjugated to one or more of the group consisting of a small molecule, an antigen, a microparticle, a nanoparticle, a polymer, a peptide, a protein, an antibody or an antibody fragment, a nucleic acid, a hormone, and a growth factor.

Accordingly, it is an object of the presently disclosed subject matter to provide chlorin derivatives, in some embodiments water-soluble chlorin derivatives, conjugates thereof, pharmaceutical compositions comprising the derivatives and/or conjugates, and methods of using and making the same.

These and other objects are achieved in whole or in part by the presently disclosed subject matter. Further, objects of the presently disclosed subject matter having been stated above, other objects and advantages of the presently disclosed subject matter will become apparent to those skilled in the art after a study of the following description, Figures, and EXAMPLES.

BRIEF DESCRIPTION OF THE FIGURES

Figure 1 is Scheme 1, an exemplary scheme for synthesizing representative **WH** building blocks for di-bromo-chlorin derivatives of the presently disclosed subject matter.
Figure 2 is Scheme 2, an exemplary scheme for synthesizing representative **EH** building blocks for di-bromo-chlorin derivatives of the presently disclosed subject matter.
Figure 3 is Scheme 3, an exemplary scheme for synthesizing Compound **CP-1.**
Figure 4 is Scheme 4, an exemplary scheme for synthesizing the Western half of Compound **C-1.**
Figure 5 is Scheme 5, an exemplary scheme for synthesizing the Eastern half of Compound **C-1** and cyclized intermediate Compound **C-1I**.
Figure 6 is Scheme 6, an exemplary scheme for the final steps for synthesizing Compound **C-1.**
Figure 7 is Scheme 7, an exemplary scheme for the final steps for synthesizing Compound **4.**

DETAILED DESCRIPTION

The presently disclosed subject matter now will be described more fully hereinafter, in which some, but not all embodiments of the presently disclosed subject matter are described. Indeed, the presently disclosed subject matter can be embodied in many different forms and should not be construed as limited to the embodiments set forth herein; rather, these embodiments are provided so that this disclosure will satisfy applicable legal requirements.

I. Definitions

The terminology used herein is for the purpose of describing particular embodiments only and is not intended to be limiting of the presently disclosed subject matter.

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent techniques that would be apparent to one of skill in the art. While the following terms are believed to be well understood by one of ordinary skill in the art, the following definitions are set forth to facilitate explanation of the presently disclosed subject matter.

In describing the presently disclosed subject matter, it will be understood that a number of techniques and steps are disclosed. Each of these has individual benefit and each can also be used in conjunction with one or more, or in some cases all, of the other disclosed techniques.

Accordingly, for the sake of clarity, this description will refrain from repeating every possible combination of the individual steps in an unnecessary fashion. Nevertheless, the specification and claims should be read with the understanding that such combinations are entirely within the scope of the invention and the claims.

Following long-standing patent law convention, the terms "a", "an", and "the" refer to "one or more" when used in this application, including the claims. For example, the phrase "a fluorescent microparticle and/or nanoparticle" refers to one or more fluorescent microparticles and/or nanoparticles, including a plurality of the same fluorescent microparticle and/or nanoparticle. Similarly, the phrase "at least one", when employed herein to refer to an entity, refers to, for example, 1, 2, 3, 4, 5, 6, 7, 8, 9, 10, 15, 20, 25, 30, 35, 40, 45, 50, 75, 100, or more of that entity, including but not limited to whole number values between 1 and 100 and greater than 100.

Unless otherwise indicated, all numbers expressing quantities of ingredients, reaction conditions, and so forth used in the specification and claims are to be understood as being modified in all instances by the term "about". The term "about", as used herein when referring to a measurable value such as an amount of mass, weight, time, volume, concentration or percentage is meant to encompass variations of in some embodiments $\pm$20%, in some embodiments $\pm$10%, in some embodiments $\pm$5%, in some embodiments $\pm$1%, in some embodiments $\pm$0.5%, and in some embodiments $\pm$0.1 % from the specified amount, as such variations are appropriate to perform the disclosed methods. Accordingly, unless indicated to the contrary, the numerical parameters set forth in this specification and attached claims are approximations that can vary depending upon the desired properties sought to be obtained by the presently disclosed subject matter.

As used herein, the term "and/or" when used in the context of a list of entities, refers to the entities being present singly or in combination. Thus, for example, the phrase "A, B, C, and/or D" includes A, B, C, and D individually, but also includes any and all combinations and subcombinations of A, B, C, and D.

The term "comprising", which is synonymous with "including" "containing", or "characterized by", is inclusive or open-ended and does not exclude additional, unrecited elements and/or method steps. "Comprising" is a term of art that means that the named elements and/or steps are present, but that other elements and/or steps can be added and still fall within the scope of the relevant subject matter.

As used herein, the phrase "consisting of" excludes any element, step, or ingredient not specifically recited. It is noted that, when the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of the related disclosure or claim to the specified materials and/or steps, plus those that do not materially affect the basic and novel characteristic(s) of the disclosed and/or claimed subject matter. For example, a fluorescent microparticle and/or nanoparticle can "consist essentially of" a polymeric matrix and at least one chlorin associated therewith, which means that the recited polymeric matrix is the only polymeric matrix present in the fluorescent microparticle and/or nanoparticle.

With respect to the terms "comprising", "consisting of', and "consisting essentially of", where one of these three terms is used herein, the presently disclosed and claimed subject matter can include the use of either of the other two terms. For example, in some embodiments, the presently disclosed subject matter relates to fluorescent microparticles and/or nanoparticles. It would be understood by one of ordinary skill in the art after review of the instant disclosure that the presently disclosed subject matter thus encompasses fluorescent microparticles and/or nanoparticles that consist essentially of the polymeric matrices and at least one chlorin associated therewith of the presently disclosed subject matter, as well as fluorescent microparticles and/or nanoparticles that consist of the polymeric matrices and at least one chlorin associated therewith of the presently disclosed subject matter.

"Halo" as used herein refers to any suitable halogen, including -F, -Cl, -Br, and -I.

"Mercapto" as used herein refers to an -SH group.

"Azido" as used herein refers to an $-N_3$ group.

"Cyano" as used herein refers to a -CN group.

"Hydroxyl" as used herein refers to an -OH group.

"Nitro" as used herein refers to an $-NO_2$ group.

"Alkyl" as used herein alone or as part of another group, refers to a straight or branched chain hydrocarbon containing from 1 or 2 to 10, 20 or 50 carbon atoms (*e.g.*, $C_1$ to $C_4$ alkyl; $C_4$ to $C_{10}$ alkyl; $C_{11}$ to $C_{50}$ alkyl). Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, and the like. "Loweralkyl" as used herein, is a subset of alkyl, in some embodiments preferred, and refers to a straight or branched chain hydrocarbon group containing from 1 to 4 carbon atoms. Representative examples of loweralkyl include, but are not limited to, methyl, ethyl, n-propyl, iso-propyl, n-butyl, iso-butyl, tert-butyl, and the like. The term "akyl" or "loweralkyl" is intended to include both substituted and unsubstituted alkyl and substituted and unsubstituted loweralkyl unless otherwise indicated, and these groups can be substituted with groups selected from halo, alkyl, haloalkyl, alkenyl, alkynyl, cycloalkyl, cycloalkylalkyl, aryl, arylalkyl, heterocyclo, heterocycloalkyl, hydroxyl, alkoxy, alkenyloxy, alkynyloxy, haloalkoxy, cycloalkoxy, cycloalkylalkyloxy, aryloxy, arylalkyloxy, heterocyclooxy, heterocycloalkyloxy, mercapto, alkyl-$S(O)_m$, haloalkyl-$S(O)_m$, alkenyl-$S(O)_m$, alkynyl-$S(O)_m$, cycloalkyl-$S(O)_m$, cycloalkylalkyl-$S(O)_m$, aryl-$S(O)_m$, arylalkyl-$S(O)_m$, heterocyclo-$S(O)_m$, heterocycloalkyl-$S(O)_m$, amino, carboxy, alkylamino, alkenylamino, alkynylamino, halo alkylamino, cycloalkylamino, cycloalkylalkylamino, arylamino, arylalkylamino, heterocycloamino, heterocycloalkylamino, disubstituted-amino, acylamino, acyloxy, ester, amide, sulfonamide, urea, alkoxyacylamino, aminoacyloxy, nitro or cyano, where m = 0, 1, 2 or 3.

"Alkylene" as used herein refers to a difunctional linear, branched or cyclic alkyl group, which can be substituted or unsubstituted, and where "alkyl" is as defined above.

"Alkenyl" as used herein alone or as part of another group, refers to a straight or branched chain hydrocarbon containing from 1 or 2 to 10, 20, or 50 carbon atoms (*e.g.*, $C_1$ to $C_4$ alkenyl; $C_4$ to $C_{10}$ alkenyl; $C_{11}$ to $C_{50}$ alkenyl, or in loweralkenyl 1 to 4 carbon atoms) which include 1 to 4 double bonds in the normal chain. Representative examples of alkenyl include, but are not limited to, vinyl, 2-propenyl, 3-butenyl, 2-butenyl, 4-pentenyl, 3-pentenyl, 2-hexenyl, 3-hexenyl, 2,4-heptadienyl, and the like. The term "alkenyl" or "loweralkenyl" is intended to include both substituted and unsubstituted alkenyl or loweralkenyl unless otherwise indicated and these groups can be substituted with groups as described in connection with alkyl and loweralkyl above.

"Alkenylene" as used herein refers to a difunctional linear, branched, or cyclic alkenyl group, which can be substituted or unsubstituted, and where "alkenyl" is as defined above.

"Alkynyl" as used herein alone or as part of another group, refers to a straight or branched chain hydrocarbon containing from 1 or 20 to 10, 20, or 50 carbon atoms (*e.g.*, $C_1$ to $C_4$ alkynyl; $C_4$ to $C_{10}$ alkynyl; $C_{11}$ to $C_{50}$ alkynyl, or in loweralkynyl 1 to 4 carbon atoms) which include 1 triple bond in the normal chain. Representative examples of alkynyl include, but are not limited to, 2-propynyl, 3-butynyl, 2-butynyl, 4-pentynyl, 3-pentynyl, and the like. The term "alkynyl" or "loweralkynyl" is intended to include both substituted and unsubstituted alkynyl or substituted and unsubstituted

loweralknynyl unless otherwise indicated, and these groups can be substituted with the same groups as set forth in connection with alkyl and loweralkyl above.

"Alkynylene" as used herein refers to a difunctional linear, branched, or cyclic alkynyl group, which can be substituted or unsubstituted, and where "alkynyl" is as defined above.

"Alkylidene chain" as used herein refers to a difunctional linear, branched, and/or cyclic organic group, which can be substituted or unsubstituted, which can be saturated or unsaturated, and which can optionally contain one, two, or three heteroatoms selected from the group consisting of N, O, and S. Examples include but are not limited to alkylene, alkenylene, alkynylene, arylene, alkarylene, and aralkylene. *See e.g.*, U.S. Patent No. 6,946,533. The alkylidene chain can contain any suitable number of carbon atoms (*e.g.*, a $C_1$ to $C_4$; $C_4$ to $C_{10}$; $C_{10}$ to $C_{20}$; $C_{20}$ to $C_{50}$).

"Alkoxy" as used herein alone or as part of another group, refers to an alkyl or loweralkyl group, as defined herein, appended to the parent molecular moiety through an oxy group, -O-. Representative examples of alkoxy include, but are not limited to, methoxy, ethoxy, propoxy, 2-propoxy, butoxy, tert-butoxy, pentyloxy, hexyloxy, and the like.

"Acyl" as used herein alone or as part of another group refers to a -C(O)R radical, where R is any suitable substituent such as aryl, alkyl, alkenyl, alkynyl, cycloalkyl, or other suitable substituent as described herein.

"Haloalkyl" as used herein alone or as part of another group, refers to at least one halogen, as defined herein, appended to the parent molecular moiety through an alkyl group, as defined herein. Representative examples of haloalkyl include, but are not limited to, chloromethyl, 2-fluoroethyl, trifluoromethyl, pentafluoroethyl, 2-chloro-3-fluoropentyl, and the like.

"Perhaloalkyl" as used herein alone or as part of another group, refers to an alkyl group wherein each hydrogen atom of the alkyl group is replaced by halo. In some embodiments, the perhaloalkyl is a perfluoroalkyl group, wherein each hydrogen atom of an alkyl group is replaced by fluoro. A representative perhaloalkyl group is trifluoromethyl (i.e., $-CF_3$).

"Alkylthio" as used herein alone or as part of another group, refers to an alkyl group, as defined herein, appended to the parent molecular moiety through a thio moiety, as defined herein. Representative examples of alkylthio include, but are not limited to, methylthio, ethylthio, tert-butylthio, hexylthio, and the like.

"Aryl" as used herein alone or as part of another group, refers to a monocyclic carbocyclic ring system or a bicyclic carbocyclic fused ring system having one or more aromatic rings. Representative examples of aryl include, azulenyl, indanyl, indenyl, naphthyl, phenyl, tetrahydronaphthyl, and the like. The term "aryl" is intended to include both substituted and unsubstituted aryl unless otherwise indicated and these groups can be substituted with the same groups as set forth in connection with alkyl and loweralkyl above.

"Arylene" as used herein refers to a difunctional aryl group, which can be substituted or unsubstituted, and where "aryl" is as defined above.

"Arylalkyl" as used herein alone or as part of another group, refers to an aryl group, as defined herein, appended to the parent molecular moiety through an alkyl group, as defined herein. Representative examples of arylalkyl include, but are not limited to, benzyl, 2-phenylethyl, 3-phenylpropyl, 2-naphth-2-ylethyl, and the like.

"Alkarylene" and "aralkylene" as used herein alone or as part of another group, refers to a difunctional group comprising at least one arylene group and at least one alkyl, alkenyl, or alkynyl group, as defined herein.

"Amino" as used herein refers to the radical $-NH_2$.

"Alkylamino" as used herein alone or as part of another group refers to the radical - NHR, where R is an alkyl group.

"Arylalkylamino" as used herein alone or as part of another group refers to the radical -NHR, where R is an arylalkyl group.

"Disubstituted-amino" as used herein alone or as part of another group refers to the radical -NRaRb, where Ra and Rb are independently selected from the groups alkyl, haloalkyl, alkenyl, alkynyl, cycloalkyl, cycloalkylalkyl, aryl, arylalkyl, heterocyclo, and heterocycloalkyl.

"Acylamino" as used herein alone or as part of another group refers to the radical - NRaRb, where Ra is an acyl group as defined herein and Rb is selected from the groups hydrogen, alkyl, haloalkyl, alkenyl, alkynyl, cycloalkyl, cycloalkylalkyl, aryl, arylalkyl, heterocyclo, and heterocycloalkyl.

"Acyloxy" as used herein alone or as part of another group refers to the radical -OR, where R is an acyl group as defined herein.

"Ester" as used herein alone or as part of another group refers to a -C(O)OR radical, where R is any suitable substituent such as alkyl, cycloalkyl, alkenyl, alkynyl, or aryl.

"Formyl" as used herein refers to a -C(O)H group.

"Carboxylic acid" as used herein refers to a -C(O)OH group.

"Sulfoxyl" as used herein refers to a compound of the formula -S(O)R, where R is any suitable substituent such as alkyl, cycloalkyl, alkenyl, alkynyl, or aryl.

"Sulfonyl" as used herein refers to a compound of the formula -S(O)(O)R, where R is any suitable substituent such as alkyl, cycloalkyl, alkenyl, alkynyl, or aryl.

"Sulfonate" as used herein refers to a compound of the formula -S(O)(O)OR, where R is any suitable substituent such as alkyl, cycloalkyl, alkenyl, alkynyl, or aryl.

"Sulfonic acid" as used herein refers to a compound of the formula -S(O)(O)OH.

"Amide" as used herein alone or as part of another group refers to a $-C(O)NR_aR_b$ radical, where $R_a$ and $R_b$ are any suitable substituent such as H, alkyl, cycloalkyl, alkenyl, alkynyl, or aryl.

"Sulfonamide", alone or as part of another group, refers to a $-S(O)_2NR_aR_b$ radical, where $R_a$ and $R_b$ are any suitable substituent such as H, alkyl, cycloalkyl, alkenyl, alkynyl, or aryl.

"Urea", alone or as part of another group, refers to an $-N(R_c)C(O)NR_aR_b$ radical, where $R_a$, $R_b$, and $R_c$ are any suitable substituent such as H, alkyl, cycloalkyl, alkenyl, alkynyl, or aryl.

"Alkoxyacylamino", alone or as part of another group, refers to an $-N(R_a)C(O)OR_b$ radical, where $R_a$, $R_b$ are any suitable substituent such as H, alkyl, cycloalkyl, alkenyl, alkynyl, or aryl.

"Aminoacyloxy", alone or as part of another group, refers to an $-OC(O)NR_aR_b$ radical, where $R_a$ and $R_b$ are any suitable substituent such as H, alkyl, cycloalkyl, alkenyl, alkynyl, or aryl.

"Cycloalkyl" as used herein alone or as part of another group, refers to a saturated or partially unsaturated cyclic hydrocarbon group containing from 3, 4, 5, 6, 7, or 8 carbons (which carbons can be replaced in a heterocyclic group as discussed below). Representative examples of cycloalkyl include, cyclopropyl, cyclobutyl, cyclopentyl, cyclohexyl, cycloheptyl, and cyclooctyl. These rings can be optionally substituted with additional substituents as described herein such as halo or loweralkyl. The term "cycloalkyl" is generic and intended to include heterocyclic groups as discussed below unless specified otherwise.

The term "polyoxyethylene chain" as used herein refers to a moiety comprising or consisting of a poly(ethylene glycol) (PEG) group, such as but not limited to a group having the formula $-(C_2H_4O)_n-$, wherein n is an integer of 2 or more (e.g., 3, 4, 5, 6, 7, 8, 9, or 10, or more). In some embodiments, n is an integer between 4 and 5000, between 4 and 1000, between 4 and 100, between 4 and 50, between 4 and 28, or between 4 and 25. The term "polyoxyethylene chain" as used herein can refer to monodisperse or polydisperse PEG chains and to straight or branched PEG chains. "Monodisperse" refers to a PEG with a polydispersity index (PDI) of 1, while polydisperse" refers to PEG with a PDI greater than 1, wherein the PEG comprises a Gaussian distribution of chain lengths and molecular weights.

The term "bioconjugatable group" as used herein refers to a reactive chemical functional group that can form a bond (e.g., a covalent bond) with a group on another entity, e.g., a protein; a peptide; a targeting agent, such as an antibody or antibody fragment; a polymer; a particle, such as a nanoparticle, an organic, polymeric, or inorganic bead; another solid support surface, etc., to form a conjugate of one of the presently disclosed chlorin or bacteriochlorin compounds and the other entity. For example, the bioconjugatable group can be an aldehyde, which can form a covalent bond with an amino group on an amino-substituted biomolecule via reductive amination, or a carboxylic acid, which can be coupled to an amino-substituted biomolecule via carbodiimide activation) Bioconjugatable groups include amines (including amine derivatives) such as isocyanates, isothiocyanates, iodoacetamides, azides, diazonium salts, etc.; carboxylic acids or acid derivatives such as N-hydroxysuccinimide (NHS) esters (more generally, active esters derived from carboxylic acids; e.g., p-nitrophenyl ester), acid hydrazides, etc.; and other groups such as, but not limited to, aldehydes, sulfonyl chlorides, sulfonyl hydrazides, epoxides, hydroxyl groups, thiol groups, maleimides, aziridines, acryloyls, halo groups, biotin, 2-iminobiotin, etc.

The term "microparticle" refers to a structure having at least one region with a dimension (*e.g.*, length, width, diameter, etc.) of less than about 1,000 μm but greater than about 1000 nm. The dimension can be in some embodiments less than about 500 μm, in some embodiments less than about 250 μm, in some embodiments less than about 200 μm, in some embodiments less than about 150 μm, in some embodiments less than about 125 μm, in some embodiments less than about 100 μm, in some embodiments less than about 80 μm, in some embodiments less than about 70 μm, in some embodiments less than about 60 μm, in some embodiments less than about 50 μm, in some embodiments less than about 40 μm, in some embodiments less than about 30 μm, in some embodiments less than about 20 μm, in some embodiments less than about 10 μm, and in some embodiments less than about 5 μm. In some embodiments, the dimension is between about 1 μm and about 250 μm (*e.g.*, about 5, 10, 15, 20, 30, 40, 50, 60, 70, 80, 90, 100, 110, 120, 130, 140, 150, 160, 170, 180, 190, 200, 210, 220, 230, 240, or 250 μm).

Similarly, the term "nanoparticle" refers to a structure having at least one region with a dimension (e.g., length, width, diameter, etc.) of less than about 1,000 nm. In some embodiments, the dimension is smaller (e.g., less than about 500 nm, less than about 250 nm, less than about 200 nm, less than about 150 nm, less than about 125 nm, less than about 100 nm, less than about 80 nm, less than about 70 nm, less than about 60 nm, less than about 50 nm, less than about 40 nm, less than about 30 nm or even less than about 20 nm). In some embodiments, the dimension is between about 5 nm and about 250 nm (e.g., about 1, 5, 10, 15, 20, 30, 40, 50, 60, 70, 80, 90, 100, 110, 120, 130, 140, 150, 160, 170, 180, 190, 200, 210, 220, 230, 240, or 250 nm).

In some embodiments, the microparticle or nanoparticle is approximately spherical. When the microparticle or nanoparticle is approximately spherical, the characteristic dimension can correspond to the diameter of the sphere. In addition to spherical shapes, the microparticle or nanoparticle can be disc-shaped, plate-shaped (*e.g.*, hexagonally

platelike), oblong, polyhedral, rod-shaped, cubic, or irregularly-shaped.

The microparticle or nanoparticle can comprise a core region (*i.e.*, the space between the outer dimensions of the particle) and an outer surface (*i.e.*, the surface that defines the outer dimensions of the particle). In some embodiments, the microparticle or nanoparticle can have one or more coating layers surrounding or partially surrounding the microparticle or nanoparticle core. Thus, for example, a spherical microparticle or nanoparticle can have one or more concentric coating layers, each successive layer being dispersed over the outer surface of a smaller layer closer to the center of the particle.

The terms "polymer" and "polymeric" refer to chemical structures that have repeating units (*i.e.*, multiple copies of a given chemical substructure). Polymers can be formed from polymerizable monomers. A polymerizable monomer is a molecule that comprises one or more moieties that can react to form bonds (*e.g.*, covalent or coordination bonds) with moieties on other molecules of polymerizable monomer. In some embodiments, each polymerizable monomer molecule can bond to two or more other molecules/moieties. In some cases, a polymerizable monomer will bond to only one other molecule, forming a terminus of the polymeric material.

Polymers can be organic, or inorganic, or any combination thereof. As used herein, the term "inorganic" refers to a compound or composition that contains at least some atoms other than carbon, hydrogen, nitrogen, oxygen, sulfur, phosphorous, or one of the halides. Thus, for example, an inorganic compound or composition can contain one or more silicon atoms and/or one or more metal atoms. In some embodiments, the polymer is polystyrene, and the microparticle and/or nanoparticle is made up of polystyrene. In some embodiments, the microparticle and/or nanoparticle is a polystyrene bead.

As used herein, the term "porphyrin" refers to a cyclic structure typically composed of four pyrrole rings together with four nitrogen atoms and two replaceable hydrogens for which various metal atoms can readily be substituted. A typical porphyrin is hemin.

As used herein, a "chlorin" differs from a porphyrin in having one partially saturated pyrrole ring. The basic chromophore of chlorophyll, the green pigment of plant photosynthesis, is a chlorin. The terms "chlorin" and "chlorin derivative" are used interchangeably herein.

The phrase "associated with" refers to any interaction between two entities, e.g., a polymeric matrix and a chlorin. In some embodiments, a polymeric matrix and a chlorin are associated with each other by a non-covalent bond such as but not limited to one or more of hydrophobic, electrostatic, and van der Walls interactions. In some embodiments, a polymeric matrix and a chlorin are associated with each other as a result of the polymeric matrix (e.g., a nanoparticle, a microparticle, a bead, etc.) encompassing the chlorin such that the chlorin is present within the polymeric matrix. In such an embodiment, the polymeric matrix is also referred to as being "doped by" or "doped with" the chlorin, and the chlorin can be considered "embedded" within the polymeric matrix. In some embodiments, a polymeric matrix and a chlorin are associated with each other by a covalent bond that attaches the chlorin to a surface of the polymeric matrix.

"Treatment" as used herein means any manner in which one or more of the symptoms of a disease or disorder are ameliorated or otherwise beneficially altered. Treatment also encompasses any pharmaceutical use of the compositions herein, such as use for treating hyperproliferating tissue or neovascularization mediated diseases or disorders, or diseases or disorders in which hyperproliferating tissue or neovascularization is implicated. As used herein, amelioration of the symptoms of a particular disorder by administration of a particular compound or pharmaceutical composition refers to any lessening, whether permanent or temporary, lasting or transient that can be attributed to or associated with administration of the composition.

"Prodrug" as used herein is a compound that, upon in vivo administration, is metabolized by one or more steps or processes or otherwise converted to the biologically, pharmaceutically or therapeutically active form of the compound.

"Antibody" as used herein refers generally to immunoglobulins or fragments thereof that specifically bind to antigens to form immune complexes. The antibody can be whole immunoglobulin of any class, e.g., IgG, IgM, IgA, IgD, IgE, chimeric or hybrid antibodies with dual or multiple antigen or epitope specificities. It can be a polyclonal antibody, preferably an affinity-purified antibody from a human or an appropriate animal, e.g., a primate, goat, rabbit, mouse or the like. Monoclonal antibodies are also suitable for use in the presently disclosed subject matter and can be preferred because of their high specificities. They are readily prepared by what are now considered conventional procedures of immunization of mammals with immunogenic antigen preparation, fusion of immune lymph or spleen cells with an immortal myeloma cell line, and isolation of specific hybridoma clones. More unconventional methods of preparing monoclonal antibodies are not excluded, such as interspecies fusions and genetic engineering manipulations of hypervariable regions, since it is primarily the antigen specificity of the antibodies that affects their utility. Newer techniques for production of monoclonals can also be used, e.g., human monoclonals, interspecies monoclonals, chimeric (e.g., human/mouse) monoclonals, genetically engineered antibodies and the like.

Accordingly, the terms "antibody" and "antibodies" refer to proteins comprising one or more polypeptides substantially encoded by immunoglobulin genes or fragments of immunoglobulin genes. Immunoglobulin genes typically include the kappa ($\kappa$), lambda ($\lambda$), alpha ($\alpha$), gamma ($\gamma$), delta ($\delta$), epsilon ($\varepsilon$), and mu ($\mu$) constant region genes, as well as myriad immunoglobulin variable region genes. Light chains are classified as either $\kappa$ or $\lambda$. In mammals, heavy chains

are classified as γ, μ, α, δ, or ε, which in turn define the immunoglobulin classes, IgG, IgM, IgA, IgD, and IgE, respectively. Other species have other light and heavy chain genes (e.g., certain avians produce what is referred to as IgY, which is an immunoglobulin type that hens deposit in the yolks of their eggs), which are similarly encompassed by the presently disclosed subject matter.

A typical immunoglobulin (antibody) structural unit is known to comprise a tetramer. Each tetramer is composed of two identical pairs of polypeptide chains, each pair having one "light" chain (average molecular weight of about 25 kiloDalton (kDa)) and one "heavy" chain (average molecular weight of about 50-70 kDa). The two identical pairs of polypeptide chains are held together in dimeric form by disulfide bonds that are present within the heavy chain region. The N-terminus of each chain defines a variable region of about 100 to 110 or more amino acids primarily responsible for antigen recognition. The terms variable light chain ($V_L$) and variable heavy chain ($V_H$) refer to these light and heavy chains, respectively.

Antibodies typically exist as intact immunoglobulins or as a number of well-characterized fragments that can be produced by digestion with various peptidases. For example, digestion of an antibody molecule with papain cleaves the antibody at a position N-terminal to the disulfide bonds. This produces three fragments: two identical "Fab" fragments, which have a light chain and the N-terminus of the heavy chain, and an "Fc" fragment that includes the C-terminus of the heavy chains held together by the disulfide bonds. Pepsin, on the other hand, digests an antibody C-terminal to the disulfide bond in the hinge region to produce a fragment known as the "$F(ab)'_2$" fragment, which is a dimer of the Fab fragments joined by the disulfide bond. The $F(ab)'_2$ fragment can be reduced under mild conditions to break the disulfide linkage in the hinge region, thereby converting the $F(ab')_2$ dimer into two "Fab'" monomers. The Fab' monomer is essentially an Fab fragment with part of the hinge region. With respect to these various fragments, Fab, $F(ab')_2$, and Fab' fragments include at least one intact antigen binding domain (referred to as a "paratope"), and thus are capable of binding to antigens.

While various antibody fragments are defined in terms of the digestion of an intact antibody, one of skill will appreciate that various of these fragments (including, but not limited to Fab' fragments) can be synthesized de novo either chemically or by utilizing recombinant DNA methodology. Thus, the term "antibody" as used herein also includes antibody fragments either produced by the modification of whole antibodies or synthesized de novo using recombinant DNA methodologies. In some embodiments, the term "antibody" comprises a fragment that has at least one antigen binding domain.

The antibodies, fragments, and derivatives of the presently disclosed subject matter can also include chimeric antibodies. As used herein in the context of antibodies, the term "chimeric", and grammatical variants thereof, refers to antibody derivatives that have constant regions derived substantially or exclusively from antibody constant regions from one species and variable regions derived substantially or exclusively from the sequence of the variable region from another species. A particular kind of chimeric antibody is a "humanized" antibody, in which the antibodies are produced by substituting the complementarity determining regions (CDRs) of, for example, a mouse antibody, for the CDRs of a human antibody (see e.g., PCT International Patent Application Publication No. WO 1992/22653). Thus in some embodiments, a humanized antibody has constant regions and variable regions other than the CDRs that are derived substantially or exclusively from the corresponding human antibody regions, and CDRs that are derived substantially or exclusively from a mammal other than a human.

The antibodies, fragments, and derivatives of the presently disclosed subject matter can also be single chain antibodies and single chain antibody fragments. Single-chain antibody fragments contain amino acid sequences having at least one of the variable regions and/or CDRs of the whole antibodies described herein but are lacking some or all of the constant domains of those antibodies. These constant domains are not necessary for antigen binding but constitute a major portion of the structure of whole antibodies.

Single-chain antibody fragments can overcome some of the problems associated with the use of antibodies containing a part or all of a constant domain. For example, single-chain antibody fragments tend to be free of undesired interactions between biological molecules and the heavy-chain constant region, or other unwanted biological activity. Additionally, single-chain antibody fragments are considerably smaller than whole antibodies and can therefore have greater capillary permeability than whole antibodies, allowing single-chain antibody fragments to localize and bind to target antigen-binding sites more efficiently. Also, antibody fragments can be produced on a relatively large scale in prokaryotic cells, thus facilitating their production. Furthermore, the relatively small size of single-chain antibody fragments makes them less likely to provoke an immune response in a recipient than whole antibodies. The single-chain antibody fragments of the presently disclosed subject matter include, but are not limited to single chain fragment variable (scFv) antibodies and derivatives thereof such as, but not limited to tandem di-scFv, tandem tri-scFv, diabodies, including bispecific diabodies, triabodies, tetrabodies, miniantibodies, minibodies, tetravalent bispecific molecules, bi-specific $F(ab')_2$ fragments, etc.

"Infecting agent" as used herein denotes invading microbes or parasites. As used herein, "microbe" denotes virus, bacteria, rickettsia, mycoplasma, protozoa, fungi and like microorganisms, and "parasite" denotes infectious, generally microscopic or very small multicellular invertebrates, or ova or juvenile forms thereof, which are susceptible

to antibody-induced clearance or lytic or phagocytic destruction, e.g., malarial parasites, spirochetes and the like.

"Tumor" as used herein denotes a neoplasm and includes both benign and malignant tumors. This term particularly includes malignant tumors which can be either solid (such as a breast, liver, or prostate carcinoma) or non-solid (such as a leukemia). Tumors can also be further divided into subtypes, such as adenocarcinomas (e.g. of the breast, prostate or lung).

"Target" as used herein denotes the object that is intended to be detected, diagnosed, impaired or destroyed by the methods provided herein, and includes target cells, target tissues, and target compositions.

"Target tissues" and "target cells" as used herein are those tissues that are intended to be impaired or destroyed by this treatment method. Photosensitizing compounds bind to or collect in these target tissues or target cells; then when sufficient radiation is applied, these tissues or cells are impaired or destroyed. Target cells are cells in target tissue, and the target tissue includes, but is not limited to, vascular endothelial tissue, abnormal vascular walls of tumors, solid tumors such as (but not limited to) tumors of the head and neck, tumors of the eye, tumors of the gastrointestinal tract, tumors of the liver, tumors of the breast, tumors of the prostate, tumors of the lung, nonsolid tumors and malignant cells of the hematopoietic and lymphoid tissue, neovascular tissue, other lesions in the vascular system, bone marrow, and tissue or cells related to autoimmune disease. Also included among target cells are cells undergoing substantially more rapid division as compared to non-target cells.

"Non-target tissues" as used herein are all the tissues of the subject which are not intended to be impaired or destroyed by the treatment method. These non-target tissues include but are not limited to healthy blood cells, and other normal tissue, not otherwise identified to be targeted.

"Target compositions" as used herein are those compositions that are intended to be impaired or destroyed by this treatment method, and can include one or more pathogenic agents, including but not limited to bacteria, viruses, fungi, protozoa, and toxins as well as cells and tissues, infected or infiltrated therewith. The term "target compositions" also includes, but is not limited to, infectious organic particles such as prions, toxins, peptides, polymers, and other compounds that can be selectively and specifically identified as an organic target that is intended to be impaired or destroyed by this treatment method.

"Hyperproliferative tissue" as used herein means tissue that grows out of control and includes neoplastic tissue, tumors and unbridled vessel growth such as blood vessel growth found in age-related macular degeneration and often occurring after glaucoma surgeries.

"Hyperproliferative disorders" as used herein denotes those conditions disorders sharing as an underlying pathology excessive cell proliferation caused by unregulated or abnormal cell growth and include uncontrolled angiogenesis. Examples of such hyperproliferative disorders include, but are not limited to, cancers or carcinomas, acute and membrano-proliferative glomerulonephritis, myelomas, psoriasis, atherosclerosis, psoriatic arthritis, rheumatoid arthritis, diabetic retinopathies, macular degeneration, corneal neovascularization, choroidal hemangioma, recurrence of pterygii, and scarring from excimer laser surgery and glaucoma filtering surgery.

"Therapeutically effective dose" as used herein is a dose sufficient to prevent advancement, or to cause regression of the disease, or which is capable of relieving symptoms caused by the disease.

"Biological materials" as used herein refers to both tissues (such as biopsy tissues) and cells, as well as biological fluids such as blood, urine, plasma, cerebrospinal fluid, mucus, sputum, etc.

"Irradiating" and "irradiation" as used herein includes exposing a subject to all wavelengths of light. Preferably, the irradiating wavelength is selected to match the wavelength(s) which excite the photosensitive compound. Preferably, the radiation wavelength matches the excitation wavelength of the photosensitive compound and has low absorption by the non-target tissues of the subject, including blood proteins.

Irradiation is further defined herein by its coherence (laser) or non-coherence (non-laser), as well as intensity, duration, and timing with respect to dosing using the photosensitizing compound. The intensity or fluence rate must be sufficient for the light to reach the target tissue. The duration or total fluence dose must be sufficient to photoactivate enough photosensitizing compound to act on the target tissue. Timing with respect to dosing with the photosensitizing compound is important, because 1) the administered photosensitizing compound requires some time to home in on target tissue and 2) the blood level of many photosensitizing compounds decreases with time. The radiation energy is provided by an energy source, such as a laser or cold cathode light source, that is external to the subject, or that is implanted in the subject, or that is introduced into a subject, such as by a catheter, optical fiber or by ingesting the light source in capsule or pill form (e.g., as disclosed in U.S. Patent No. 6,273,904).

While some embodiments of the presently disclosed subject matter are drawn to the use of light energy for administering photodynamic therapy (PDT) to destroy tumors, other forms of energy are within the scope of the presently disclosed subject matter, as will be understood by those of ordinary skill in the art. Such forms of energy include, but are not limited to: thermal, sonic, ultrasonic, chemical, light, microwave, ionizing (such as x-ray and gamma ray), mechanical, and electrical. For example, sonodynamically induced or activated agents include, but are not limited to: gallium-porphyrin complex (see Yumita et al. (1997) Cancer Letters 112:79-86), other porphyrin complexes, such as protoporphyrin and hematoporphyrin (see Umemura et al. (1996) Ultrasonics Sonochemistry 3:S187-S191); other cancer drugs,

such as daunorubicin and adriamycin, used in the presence of ultrasound therapy (see Yumita et al. (1987) Japanese Journal of Hyperthermic Oncology 3(2):175-182).

"Coupling agent" as used herein, refers to a reagent capable of coupling a photosensitizer to a targeting agent.

"Targeting agent" refers to a compound that homes in on or preferentially associates or binds to a particular tissue, receptor, infecting agent or other area of the body of the subject to be treated, such as a target tissue or target composition. Examples of a targeting agent include but are not limited to an antibody, a ligand, one member of a ligand-receptor binding pair, nucleic acids, peptide-nucleic acids (PNA), aptamers, proteins and peptides, and liposomal suspensions, including tissue-targeted liposomes.

"Specific binding pair" and "ligand-receptor binding pair" as used herein refers to two different molecules, where one of the molecules has an area on the surface or in a cavity which specifically attracts or binds to a particular spatial or polar organization of the other molecule, causing both molecules to have an affinity for each other. The members of the specific binding pair are referred to as ligand and receptor (anti-ligand). The terms ligand and receptor are intended to encompass the entire ligand or receptor or portions thereof sufficient for binding to occur between the ligand and the receptor. Examples of ligand-receptor binding pairs include, but are not limited to, hormones and hormone receptors, for example epidermal growth factor and epidermal growth factor receptor, tumor necrosis factor-$\alpha$ and tumor necrosis factor-receptor, and interferon and interferon receptor; avidin and biotin or antibiotin; antibody and antigen pairs; enzymes and substrates, drug and drug receptor; cell-surface antigen and lectin; two complementary nucleic acid strands; nucleic acid strands and complementary oligonucleotides; interleukin and interleukin receptor; and stimulating factors and their receptors, such as granulocyte-macrophage colony stimulating factor (GMCSF) and GMCSF receptor and macrophage colony stimulating factor (MCSF) and MCSF receptor.

"Linkers" are aromatic or aliphatic groups (which can be substituted or unsubstituted and can optionally contain heteroatoms such as N, O, or S) that are utilized to couple a bioconjugatable group, cross-coupling group, surface attachment group, hydrophilic group or the like to the parent molecule. Examples include but are not limited to aryl, alkyl, heteroaryl, heteroalkyl (e.g., oligoethylene glycol), peptide, and polysaccharide linkers, etc.

Subjects to be treated by the methods of the presently disclosed subject matter for diagnostic or therapeutic purposes include both human subjects and other animal subjects (particularly mammalian subjects such as dogs, cats, horses, monkeys, chimpanzees, etc.) for veterinary purposes.

More particularly, the terms "subject", "patient", and "recipient" as used herein can be used interchangeably and can refer to a member of any invertebrate or vertebrate species. Accordingly, the term "subject" is intended to encompass any member of the Kingdom Animalia including, but not limited to the phylum Chordata (e.g., members of Classes Osteichythyes (bony fish), Amphibia (amphibians), Reptilia (reptiles), Aves (birds), and Mammalia (mammals)), and all Orders and Families encompassed therein.

The compositions and methods of the presently disclosed subject matter are particularly useful for warm-blooded vertebrates. Thus, the presently disclosed subject matter concerns mammals and birds. More particularly provided are compositions and methods derived from and/or for use in mammals such as humans and other primates, as well as those mammals of importance due to being endangered (such as Siberian tigers), of economic importance (animals raised on farms for consumption by humans) and/or social importance (animals kept as pets or in zoos) to humans, for instance, carnivores other than humans (such as cats and dogs), swine (pigs, hogs, and wild boars), ruminants (such as cattle, oxen, sheep, giraffes, deer, goats, bison, and camels), rodents (such as mice, rats, hamsters, guinea pigs, and rabbits), marsupials, and horses. Also provided is the use of the disclosed methods and compositions on birds, including those kinds of birds that are endangered, kept in zoos or as pets (e.g., parrots, cockatiels and the like), as well as fowl, and more particularly domesticated fowl, e.g., poultry, such as turkeys, chickens, ducks, geese, guinea fowl, and the like, as they are also of economic importance to humans. Thus, also provided is the use of the disclosed methods and compositions on livestock, including but not limited to domesticated swine (pigs and hogs), ruminants, horses, poultry, and the like.

## II. Chlorin Compounds

The presently disclosed subject matter provides, in some embodiments, chlorin derivatives. In some embodiments, a chlorin derivative of the presently disclosed subject matter is a water-soluble chlorin derivative, wherein the chlorin derivative has a solubility of about 1 milligram per milliliter (mg/mL) or more in an aqueous solution (e.g., water, saline, PBS, etc.). Water solubility is provided, for example, by adding solubilizing groups comprising PEG chains at $\beta$-pyrrole positions. In some embodiments, the PEG chains are attached to the chlorin via different groups and/or are longer than PEG chains that have been attached to previously described Pegylated chlorin compounds. In some embodiments, the presently disclosed chlorin has a solubility in an aqueous solution of about 2.5 mg/mL or more. In some embodiments, the chlorin has a solubility in an aqueous solution of about 5.0 mg/mL or more. In some embodiments, the chlorin has a solubility in an aqueous solution of about 10 mg/mL or more. In some embodiments, the chlorin has a solubility of about 700, 800 or 900 $\mu$M or more in aqueous solution. In some embodiments, the chlorin has a solubility

of about 1, 1.5, 2, 2.5, or 3.0 mM or more in aqueous solution.

In some embodiments, the chlorin derivative (including but not limited to water-soluble chlorin derivatives) comprises a linker moiety comprising a bioconjugatable group that can be used to conjugate the chlorin derivative to another substance, for example, that can act as a targeting agent or as a substance to be detected. In some embodiments, the substance to which the chlorin derivative can be conjugated can be a small molecule (e.g., a non-polymeric synthetic molecule having a molecular weight of about 900 dalton (Da) or less), an antigen, a microparticle, a nanoparticle, a polymer, a peptide, a protein, an antibody or an antibody fragment, a nucleic acid, a hormone, or a growth factor. The bioconjugatable group can include, for example, a carboxylic acid or active ester, a hydroxyl, an amine, a thiol, or an aldehyde. In some embodiments, the linker moiety further includes both arylene and alkylene groups. In some embodiments, the linker moiety includes an arylene and/or alkynylene group proximal to the main chlorin structure, while an alkylene group is proximal to the bioconjugatable group.

The presently disclosed chlorin derivatives can comprise at least one solubilizing group. In some embodiments, the solubilizing group includes one or more polyoxyethylene chains (e.g., PEG chains). In some embodiments, the solubilizing group comprises at least two PEG chains. In some embodiments, the PEG chains are monodisperse and comprise at least 4 $-CH_2CH_2O-$ repeating units. In some embodiments, the PEG chains comprise at least 6, 8, 10, or 12 $-CH_2CH_2O-$ repeating units. Thus, the solubilizing group can comprise two PEG6, PEG8, PEG10, or PEG12 groups. In some embodiments, the PEG chains comprise 12 or more $-CH_2CH_2O-$ repeating units (e.g., between about 12 and about 24 or about 28 $-CH_2CH_2O-$ repeating units). In some embodiments, the compound comprises two solubilizing groups attached to two different pyrrolic carbon atoms. In some embodiments, each of the two solubilizing groups comprises two PEG chains.

In some embodiments, the solubilizing groups are β-pyrrole substituents, that comprise an arylene or alkynyl-arylene group directly attached to a β-pyrrole carbon atom, but where the group is free of an oxo linker directly between a PEG chain and the aryl group. In some embodiments, the solubilizing groups comprise one or more amide linkages between the aryl group and the PEG chains. In some embodiments, the amide linkages further include one or more alkylene spacers (e.g., ethylene, propylene, etc.).

The chlorin derivative is a compound of Formula (I):

(I)

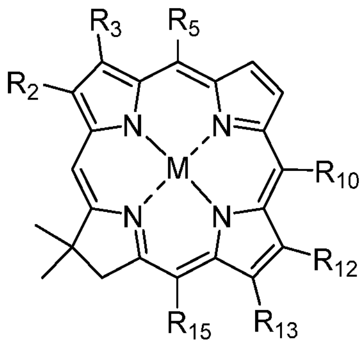

,

wherein:

M is a metal or is two hydrogens (i.e., -H, -H);

$R_5$, and $R_{15}$ are independently selected from H, alkoxy, and a linker group having the formula: $-L_1-(X_1-L_2)_p-G$, wherein p is 0 or 1, $L_1$ is alkylidene, $X_1$ is - C(=O)NH- or -NHC(=O)-, $L_2$ is $-(CH_2CH_2O)_q$-alkylene-, wherein q is an integer between 1 and 24, alkylene, or substituted alkylene (e.g., alkylene substituted by a one or more groups comprising a polyoxyethylene chain and/or an amide group);

and G is a bioconjugatable group; $R_{10}$ is

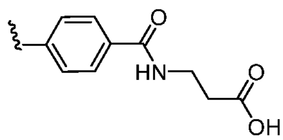

;

and $R_2$, $R_3$, $R_{12}$, and $R_{13}$ are independently selected from H, halo, cyano, perhaloalkyl (e.g., perfluoroalkyl, including, but not limited to, trifluoromethyl), sulfonate, sulfonamide, ester, carboxylic acid, formyl, acetyl, a linker group having

the formula $-L_1-(X_1-L_2)_p-G$, and a solubilizing group, wherein the solubilizing group is selected from $-aryl-(R_s)_w$ and $-alkynyl-aryl-(R_s)_w$, wherein w is an integer between 0 and 5 inclusive, wherein when w is 0, the chlorin derivative is water-insoluble (i.e., hydrophobic), and wherein when w is 1, 2, 3, 4, or 5, the chlorin derivative is water-soluble (i.e., hydrophilic), and $R_S$ is a group having the formula:

$$-X_2-(L_3)_z-R_{17},$$

wherein: z is 0 or 1; $X_2$ is $-CH_2NHC(=O)-$, -C(=O)NH-alkylene-NH-, or triazolyl; $L_3$ is - C(=O)-alkylene-C(=O)-NH-, and $R_{17}$ is selected from $-(C_2H_4O)_m-R_{18}$, $-C(=O)C_2H_4-(OC_2H_4)_mOR_{18}$, and $-(C_2H_4O)_n-C_2H_4-C(=O)NH-C(R_{19})_3$, wherein m is an integer of 4 or more (e.g., at least 8 , at least 10 or more, or 12 or more); n is an integer between 1 and 5; $R_{18}$ is loweralkyl (e.g., methyl); and $R_{19}$ is $-CH_2O-C_2H_4-C(=O)NH-(C_2H_4O)_mR_{18}$; subject to the proviso that at least one of $R_2$, $R_3$, $R_{12}$, and $R_{13}$ is $-aryl-(R_s)_w$ or $-alkynyl-aryl-(R_s)_w$.

M can be any suitable metal ion (e.g., Pd, Pt, Mg, Al, Ga, In, Sn, Au, Ni, Cu, Co, Fe, or Zn) or be absent (e.g., in which case it is replaced by two hydrogens (-H, -H), i.e., such that two of the pyrrole nitrogen atoms of the chlorin ring are protonated. In some embodiments, M is Zn or is replaced by -H, H. Thus, the compounds of Formula (I) include metallochlorin derivatives and free base chlorin derivatives.

In some embodiments, $R_5$, and $R_{15}$ are selected from H, methoxy, and a linker group having the formula: $-L_1-(X_1-L_2)_p-G$. In some embodiments, at least one of $R_5$, and $R_{15}$ is a linker group having the formula: $-L_1-(X_1-L_2)_p-G$.

In some embodiments, $L_1$ is arylene or alkarylene (e.g., comprising an alkenyl or alkynyl group attached to an aryl group). In some embodiments, $L_1$ is phenylene or -C=C-phenyl-. In some embodiments, p is 1 and the linker group includes a polyoxyethylene chain to improve solubility and/or spacer (e.g., an alkylene spacer) to improve the reactivity of G as compared to the reactivity of G in a comparable chlorin where G is directly attached to $L_1$.

In some embodiments, the linker group (e.g., at $R_5$, or $R_{15}$) having a formula $-L_1-(X_1-L_2)_p-G$ wherein $L_1$ is phenylene, p is 1; $X_1$ is -C(=O)NH-, $L_2$ is alkylene, and G is selected from a carboxylic acid or an active ester (e.g., a NHS-ester). In some embodiments, $L_2$ is ethylene. Thus, in some embodiments, the linker group includes a β-alanine spacer to improve the reactivity of G.

In some embodiments, the linker (e.g. $R_5$, or $R_{15}$) is:

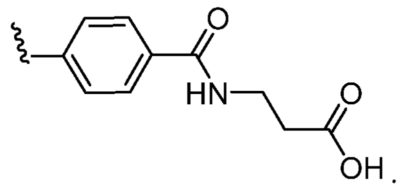

In some embodiments, at least one of $R_2$ and $R_3$ and one of $R_{12}$ and $R_{13}$ is $-aryl-(R_s)_w$ or $-alkynyl-aryl-(R_s)_w$. Thus, in some embodiments, the compound of Formula (I) includes at least two solubilizing groups. For example, in some embodiments, both $R_2$ and $R_{12}$ are each $-aryl-(R_s)_w$ or $-alkynyl-aryl-(R_s)_w$. In some embodiments, $R_3$ and $R_{13}$ are each $-aryl-(R_s)_w$ or $-alkynyl-aryl-(R_s)_w$.

In some embodiments, $R_3$ and $R_{13}$ are each $-aryl-(R_s)_w$. In some embodiments, $R_3$ and $R_{13}$ are each -phenyl-$(R_s)_2$.

In some embodiments, each $R_s$ has a formula $-X_2-(L_3)_z-R_{17}$, wherein: z is 0, $X_2$ is - $CH_2NHC(=O)-$, and $R_{17}$ is $-(C_2H_4O)_m-R_{18}$, wherein m is an integer between 12 and 24 (i.e., 12, 13, 14, 15, 16, 17, 18, 19, 20, 21, 22, 23, or 24).

In some embodiments, m is 12 and $R_{18}$ is methyl. In some embodiments, $R_3$ and $R_{13}$ are each:

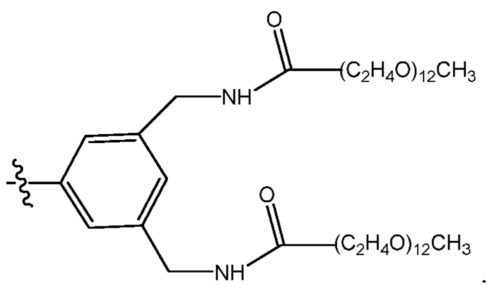

In some embodiments, the compound is C-1, the compound having the structure:

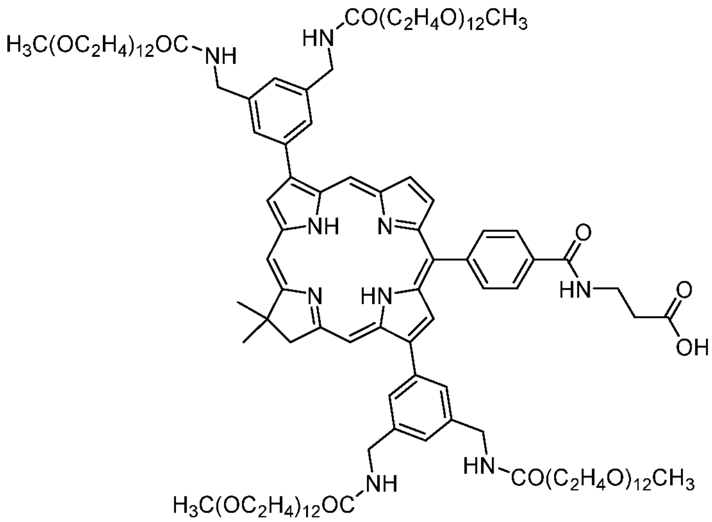

.

In some embodiments, the compound is Compound 4, the compound having the structure:

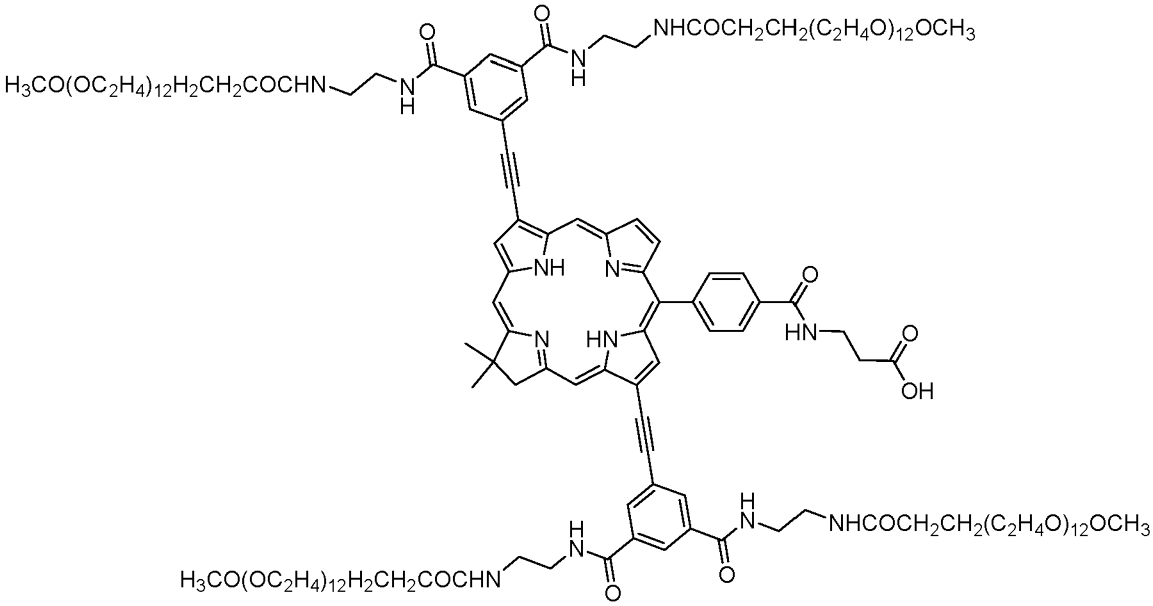

In some embodiments, the presently disclosed subject matter provides a covalent conjugate formed between (a) a compound of Formula (I) as defined in above and subject to the proviso that at least one of $R_2$, $R_3$, $R_5$, $R_{10}$, $R_{12}$, $R_{13}$, and $R_{15}$ is a linker group; and (b) one or more of the group comprising a small molecule, an antigen, a microparticle, a nanoparticle, a polymer, a peptide, a protein, an antibody or an antibody fragment, a nucleic acid, a hormone, and a growth factor. In some embodiments, for example, the conjugate can be formed by reacting a compound of Formula (I) comprising a linking group comprising a carboxylic acid or active ester (i.e., as the bioconjugatable group G) with an amino group of a small molecule, peptide, protein, antibody, or polymer.

III. Methods of Synthesis

Methods of synthesizing chlorins that can be adapted for use in preparing the presently disclosed chlorins are described, for example, in U.S. Patent Nos. 8,212,055 and 8,980,565. In some embodiments, the presently disclosed compounds of Formula (I) can be prepared by preparing a suitable trans beta-substituted chlorin, such as a chlorin wherein two beta-chlorin substituents are halo (e.g., Br) substituents and then further reacting the beta-substituents to replace them with suitable water solubilizing groups. Methods of synthesizing trans beta-substituted chlorins have been previously described. See e.g., U.S. Patent No. 6,559,374.

For example, in some embodiments, the chlorins of Formula (I) can be prepared by condensing a dihydrodipyrrin

building block representing the "Western half' (WH) of the chlorin with a building block representing the "Eastern half' (EH) of the chlorin. In some embodiments, the WH building block can have a structure as follows:

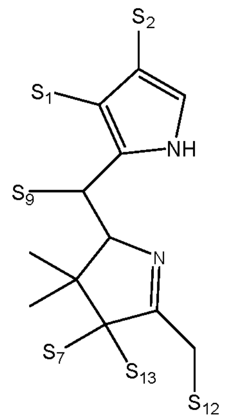

wherein $S_1$, $S_2$, $S_7$, $S_9$, $S_{12}$, and $S_{13}$ are independent selected from H, aryl, substituted aryl, phenyl, cycloalkyl, alkyl, substituted alkyl, alkenyl, alkynyl, halo, alkoxy, alkylthio, perfluoroalkyl, perfluoroaryl, pyridyl, cyano, thiocyanto, nitro, amino, alkylamino, acyl, sulfoxyl, sulfonyl, imido, amido, and carbamoyl; or wherein $S_7$ and $S_{13}$ together form =O; or where $S_1$ and $S_2$ together form a substituted or unsubstituted alkylene or arylene group. In some embodiments, $S_{12}$ is H.

The EH building block compound can have the structure:

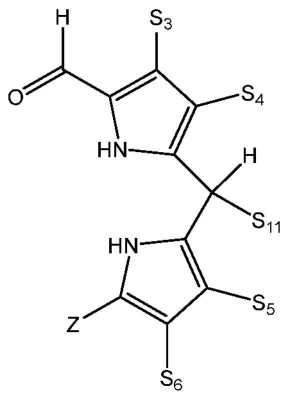

wherein $S_3$, $S_4$, $S_5$, $S_6$, and $S_{11}$ are independent selected from H, aryl, substituted aryl, phenyl, cycloalkyl, alkyl, substituted alkyl, alkenyl, alkynyl, halo, alkoxy, alkylthio, perfluoroalkyl, perfluoroaryl, pyridyl, cyano, thiocyanto, nitro, amino, alkylamino, acyl, sulfoxyl, sulfonyl, imido, amido, and carbamoyl; or wherein $S_6$ and $S_5$ together form a substituted or unsubstituted alkylene or arylene group; and where Z is halo (e.g., Br, Cl, or I).

More particularly, methods of preparing di-bromo substituted chlorins and their corresponding bromo-substituted dihydropyrrin WH and EH building blocks have been previously described. See e.g., Krayer et al. (2009) Journal of Porphyrins and Phthalocyanines 13(10):1098-1110; Jiang et al. (2014) Organic & Biomolecular Chemistry 12:86-103. For instance, a chlorin derivative comprising two bromo substituents at the 3 and 13 positions of the chlorin can be prepared from a WH building block prepared, for example, from 2-formyl pyrrole as set forth in Scheme 1 shown in Figure 1.

As set forth in Scheme 1 in Figure 1, 2-formyl pyrrole (pyrrole-2-carboxaldehyde) is first brominated using N-bromosuccinimde (NBS) to provide bromo-substituted pyrrole **a.** The nitrogen atom can then be protected by a suitable protecting group Pg to provide protected bromo-substituted pyrrole **b.** In some embodiments, the protecting group can be a tosyl group installed by deprotonating the nitrogen atom of bromo pyrrole a and then contacting the deprotonated compound with p-toluenesulfonyl chloride to provide a N-tosyl pyrrole. Protected pyrrole **b** is then treated with nitromethane (e.g., in the presence of potassium acetate and a slight excess of methylamine-hydrochloride) to provide aldol condensation product c. The reduction of the carbon-carbon double bond in c with a suitable reducing agent (e.g., $LiBH_4$) provides compound **d,** which is treated with mesityl oxide in the presence of a non-nucleophilic base (e.g., 1,8-diazabicyclo[5.4.0]undec-7-ene (DBU)) to undergo a Michael addition reaction. Michael addition product e is then treated with formamide and zinc powder to undergo reductive cyclization to provide protected dihydropyrrin **f,** which is deprotected to provide chlorin **WH.**

A suitable EH chlorin building block can be prepared as set forth in Scheme 2 shown in Figure 2. See also, Laha et al. (2003) Organic Process Research & Development 7(6):799-812; Yu et al. (2013) Journal of Organic Chemistry 78(21):10678-10691; and Liu et al. (2016) New Journal of Chemistry 40(9):7721-7740. The EH chlorin building block in Scheme 2 comprises an aryl substituent that can be used as a linker group (e.g., after hydrolysis of the methyl ester) or be further elaborated (e.g., via reaction with an amino-containing alkyl compound further comprising a second functional group that can act as a bioconjugatable group or be reacted to form a bioconjugatable group) to provide a longer linker group in the corresponding chlorin.

As set forth in Scheme 2 shown in Figure 2, pyrrole can be reacted with methyl-4-formylbenzoate to provide dipyrromethane **g.** If desired, the use of an aldehyde other than methyl-4-formyl benzoate can provide a dipyrromethane substituted by a group other than the methyl benzoate. Aldehyde **h** was obtained by Vilsmeier formylation of **g.** Aldehyde **h** can be di-brominated using NBS to provide dibromo-dipyrromethane **EH.**

The **WH** and **EH** building blocks can be condensed in the presence of an acid (e.g., a Bronsted or Lewis acid, such as trifluoroacetic acid or tosylic acid (TsOH)) to form a condensation product and then oxidatively cyclized in an organic solvent in the presence of a base (e.g., 2,2,6,6-tetramethylpiperidine), an oxidant (silver trifluoromethanesulfonate), and a metal salt (zinc acetate) to provide a metallochlorin. Optionally, the metal (e.g., Zn) can be displaced with an acid (e.g., trifluoroacetic acid) to provide the chlorin free base. In some embodiments, the chlorin free base can have the structure:

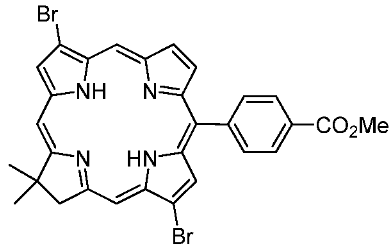

The halo substituents of the di-halo chlorins, such as the di-bromo chlorin free base above, can be further elaborated to provide solubilizing groups using coupling chemistry known in the art, including, but not limited to Stille coupling, Hiyama coupling, Suzuki coupling, Negishi coupling, Sonogashira coupling, and Kumada coupling reactions. For example, the dihalo chlorin can be reacted with a boronic acid in the presence of a Pd(0) catalyst; an organotin compound in the presence of a Pd catalyst; a pseudo halide or an organosilane in the presence of a Pd catalyst; an organozinc compound in the presence of a Ni or Pd catalysts, or a Grignard reagent in the presence of a Ni or Pd catalysts. In some embodiments, the di-halo chlorin (e.g., the di-bromo chlorin free base shown above) can be reacted with an aryl boronic acid Suzuki coupling reaction partner. See Jiang et al. (2015) New Journal of Chemistry 39(7):5694-5714; and Zhang et al. (2016) New Journal of Chemistry 40(9):7750-7767. In some embodiments, the aryl boronic acid can include additional chemical functional groups or protected chemical functional groups that can be further elaborated after the Suzuki coupling reaction. For example, in some embodiments, the Suzuki coupling reaction can include one or more protected amino groups that, after deprotection, can be reacted with a suitable PEG reagent (e.g., an activated PEG ester). Suitable catalysts for Suzuki coupling reactions include, but are not limited to, $Pd(PPh_3)_4$, and $PdCl_2(PPh_3)_4$. Suitable solvents include water, toluene, THF, dioxane, DMF and combinations thereof. Suitable bases for use in Suzuki coupling reactions include, but are not limited to $Cs_2CO_3$, $K_3PO_4$, NaOH, NaOEt, NaOtBu, $Na_2CO_3$, $K_2CO_3$, alkyl lithium compounds, and trialkylamines (e.g., triethylamine).

In some embodiments, the presently disclosed compounds can employ tert-butyloxycarbonyl (BOC) protecting groups for amino groups present on the Suzuki or other type of coupling agent, optionally in combination with the use of t-butyl ester protection of carboxylic acids (e.g., in linker groups). Surprisingly, deprotection of BOC groups during the preparation of the presently disclosed compounds using trifluoroacetic acid (TFA), a common method of BOC deprotection, was found to lead to significant decomposition, particularly, for example, in compounds including alkyne bonds. Thus, in some embodiments, BOC deprotection is performed using other conditions, e.g., 4 M HCl in dioxane, which avoids the decomposition seen when using TFA.

IV. Pharmaceutical Compositions

Compounds of the presently disclosed subject matter can be provided as pharmaceutically acceptable salts. Such salts include, but are not limited to, amine salts, such as but not limited to N,N'-dibenzylethylenediamine, chloroprocaine, choline, ammonia, diethanolamine, and other hydroxyalkylamines, ethylenediamine, N-methylglucamine, procaine, N-benzylphenethylamine, 1-para-chlorobenzyl-2-pyrrolidin-1'-ylmethyl-benzimidazole, diethylamine, and other alkylamines, piperazine and tris(hydroxymethyl)aminomethane; alkali metal salts, such as but not limited to lithium,

potassium and sodium; alkali earth metal salts, such as but not limited to barium, calcium and magnesium; transition metal salts, such as but not limited to zinc; and other metal salts, such as but not limited to sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates, and fumarates. Pharmaceutically acceptable esters include, but are not limited to, alkyl, alkenyl, alkynyl, aryl, heteroaryl, aralkyl, heteroaralkyl, cycloalkyl and heterocyclyl esters of acidic groups, including, but not limited to, carboxylic acids, phosphoric acids, phosphinic acids, sulfonic acids, sulfinic acids, and boronic acids.

Compounds of the presently disclosed subject matter can also include prodrugs of the compounds described herein. As noted above, a "prodrug" is a compound that, upon in vivo administration, is metabolized by one or more steps or processes or otherwise converted to the biologically, pharmaceutically or therapeutically active form of the compound. To produce a prodrug, the pharmaceutically active compound is modified such that the active compound will be regenerated by metabolic processes. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound (see e.g., Nogrady (1985) Medicinal Chemistry A Biochemical Approach, Oxford University Press, New York, New York, United States of America, pages 388-392).

Utility. The methods and intermediates described herein are useful for the synthesis of compounds of Formula (I) as described herein. Such compounds are useful per se or in further modified form (e.g., as a salt, metalated compound, conjugate or prodrug) for diagnostic and therapeutic purposes in like manner as other compounds described for photodynamic therapy, such as described in U.S. Patent Application Publication No. 2004/0044197 to Pandey et al. and as set forth in further detail below.

Stability. An advantage of some embodiments of chlorin compounds of the presently disclosed subject matter is their stability and absorption characteristics. Thus, the presently disclosed subject matter provides a "neat" composition consisting of an active compound of the presently disclosed subject matter (e.g., compounds of Formula (I), or the pharmaceutically acceptable salts, prodrugs, or conjugates thereof (e.g, with a targeting agent such as a protein, peptide, or antibody)), wherein the composition has or is characterized by a peak Molar absorption coefficient in solution of at least 10,000, up to 300,000 $M^{-1}$ $cm^{-1}$ or more, at a wavelength between about 600 and about 800 nanometers (it being understood that (a) the active compound must be placed into solution to determine its peak Molar absorption coefficient at the indicated wavelength; and (b) the compound can exhibit additional peaks outside of this range, or multiple peaks within this range).

In addition, the presently disclosed subject matter provides compositions comprising or consisting essentially of a compound of Formula (I), or a pharmaceutically acceptable salt, prodrug, or conjugate thereof (e.g., with a targeting agent such as a protein, peptide or antibody)) in a solvent. The amount of solvent is not critical and can comprise from 0.01 or 1 to 99 or 99.99 percent by weight of the composition. The composition has or is characterized by a peak Molar absorption coefficient in solution of at least 10,000, up to 300,000 $M^{-1}$ $cm^{-1}$ or more, at a wavelength between about 600 and about 800 nanometers. It will be appreciated that agitation can be used as needed to break agglomerated particles back into solution prior to determining molar absorption, but that some level of agglomeration can be desired for practical use of the composition. Suitable solvents depend upon the particular compound and intended use for that compound, but include both organic solvents, aqueous solvents and combinations thereof.

The compositions, be they the chlorin compound or compounds in "neat" form or the chlorin compound or compounds mixed with a solvent, have or exhibit a loss of not more than about 10, 15, or 20 percent by weight of the chlorin compound of the presently disclosed subject matter (due to degradation thereof) when stored in a sealed vessel (e.g., a flask ampoule or vial), at room temperature in the absence of ambient light for at least 3 or 4 months. Degradation can be determined by spectroscopy, thin-layer chromatography, NMR spectroscopy, and/or mass spectrometry, in accordance with known techniques.

Solubility. An advantage of some embodiments of compounds of the presently disclosed subject matter is their aqueous solubility. Thus the presently disclosed subject matter provides compositions, including but not limited to pharmaceutical formulations, comprising, consisting of, or consisting essentially of: (a) an aqueous solvent (for example, distilled water, saline solution, buffer solution); and (b) from about 1, 2, 5 or 10 $\mu$M up to 200, 300, or 500 mM of an active compound as described herein solubilized in the aqueous solvent.

Formulation of Pharmaceutical Compositions. The pharmaceutical compositions provided herein contain therapeutically effective amounts of one or more of the compounds provided herein that are useful in the prevention, treatment, or amelioration of one or more of the symptoms of diseases or disorders associated with hyperproliferating tissue or neovascularization, or in which hyperproliferating tissue or neovascularization is implicated, in a pharmaceutically acceptable carrier. Diseases or disorders associated with hyperproliferating tissue or neovascularization include, but are not limited to, cancer, psoriasis, atherosclerosis, heart disease, and age-related macular degeneration. Pharmaceutical

carriers suitable for administration of the compounds provided herein include any such carriers known to those skilled in the art to be suitable for the particular mode of administration.

Pharmaceutical compositions preferably exhibit the absorption characteristics and storage or stability characteristics described above.

In addition, the compounds can be formulated as the sole pharmaceutically active ingredient in the composition or can be combined with other active ingredients.

The compositions contain one or more compounds (e.g., compounds of Formula (I)) provided herein. The compounds are, in some embodiments, formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. In some embodiments, the compounds described above are formulated into pharmaceutical compositions using techniques and procedures well known in the art (see e.g., Ansel (1985) Introduction to Pharmaceutical Dosage Forms, Fourth Edition, Lea & Febiger, Philadelphia, Pennsylvania, United States of America, page 126).

In the compositions, effective concentrations of one or more compounds or pharmaceutically acceptable derivatives thereof is (are) mixed with a suitable pharmaceutical carrier. The compounds can be derivatized as the corresponding salts, esters, enol ethers or esters, acetals, ketals, orthoesters, hemiacetals, hemiketals, acids, bases, solvates, hydrates, or prodrugs prior to formulation, as described above. The concentrations of the compounds in the compositions are effective for delivery of an amount, upon administration, that treats, prevents, or ameliorates one or more of the symptoms of diseases or disorders associated with hyperproliferating tissue or neovascularization or in which hyperproliferating tissue or neovascularization is implicated.

In some embodiments, the compositions are formulated for single dosage administration. To formulate a composition, the weight fraction of compound is dissolved, suspended, dispersed or otherwise mixed in a selected carrier at an effective concentration such that the treated condition is relieved, prevented, or one or more symptoms are ameliorated.

The active compound (i.e., the compound of Formula (I), or a pharmaceutically acceptable salt, prodrug or conjugate thereof) is included in the pharmaceutically acceptable carrier in an amount sufficient to exert a therapeutically useful effect in the absence of undesirable side effects on the patient treated. The therapeutically effective concentration can be determined empirically by testing the compounds in in vitro and in vivo systems described herein and in U.S. Patent No. 5,952,366 to Pandey et al. and then extrapolated therefrom for dosages for humans.

The concentration of active compound in the pharmaceutical composition can depend on absorption, inactivation and excretion rates of the active compound, the physicochemical characteristics of the compound, the dosage schedule, and amount administered as well as other factors known to those of skill in the art. For example, the amount that is delivered is sufficient to ameliorate one or more of the symptoms of diseases or disorders associated with hyperproliferating tissue or neovascularization or in which hyperproliferating tissue or neovascularization is implicated, as described herein.

In some embodiments, a therapeutically effective dosage should produce a serum concentration of active ingredient of from about 0.1 ng/ml to about 50-100 μg/ml. In one embodiment, a therapeutically effective dosage is from 0.001, 0.01 or 0.1 to 10, 100 or 1000 mg of active compound per kilogram of body weight per day. Pharmaceutical dosage unit forms are prepared to provide from about 0.01 mg, 0.1 mg or 1 mg to about 500 mg, 1000 mg or 2000 mg, and in some embodiments from about 10 mg to about 500 mg of the active ingredient or a combination of essential ingredients per dosage unit form.

The active ingredient can be administered at once, or can be divided into a number of smaller doses to be administered at intervals of time. It is understood that the precise dosage and duration of treatment is a function of the disease being treated and can be determined empirically using known testing protocols or by extrapolation from in vivo or in vitro test data. It is to be noted that concentrations and dosage values can also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

In instances in which the compounds exhibit insufficient solubility, methods for solubilizing compounds can be used. Such methods are known to those of skill in this art, and include, but are not limited to, using cosolvents, such as dimethylsulfoxide (DMSO), using surfactants, such as polyoxyethylene sorbitol esters (e.g., sold under the tradename TWEEN®), or dissolution in aqueous sodium bicarbonate. Derivatives of the compounds, such as prodrugs of the compounds can also be used in formulating effective pharmaceutical compositions.

Upon mixing or addition of the compound(s), the resulting mixture can be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and can be empirically determined.

The pharmaceutical compositions are provided for administration to humans and animals in unit dosage forms, such as tablets, capsules, pills, powders, granules, sterile parenteral solutions or suspensions, and oral solutions or suspensions, and oil-water emulsions containing suitable quantities of the compounds or pharmaceutically acceptable derivatives thereof. The pharmaceutically therapeutically active compounds and derivatives thereof are, in some embodiments, formulated and administered in unit-dosage forms or multiple-dosage forms. Unit-dose forms as used herein refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampoules and syringes and individually packaged tablets or capsules. Unit-dose forms can be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

Liquid pharmaceutically administrable compositions can, for example, be prepared by dissolving, dispersing, or otherwise mixing an active compound as defined above (e.g., a compounds of Formula (I), or a pharmaceutically acceptable salt, prodrug or conjugate thereof) and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered can also contain minor amounts of nontoxic auxiliary substances such as wetting agents, emulsifying agents, solubilizing agents, pH buffering agents and the like, for example, acetate, sodium citrate, cyclodextrin derivatives, sorbitan monolaurate, triethanolamine sodium acetate, triethanolamine oleate, and other such agents.

Actual methods of preparing such dosage forms are known, or will be apparent, to those skilled in this art; for example, see Remington's Pharmaceutical Sciences, 15th Edition, 1975, Mack Publishing Company, Easton, Pennsylvania, United States of America.

Dosage forms or compositions containing active ingredient in the range of 0.005% to 100% with the balance made up from non-toxic carrier can be prepared. Methods for preparation of these compositions are known to those skilled in the art. The contemplated compositions can contain 0.001%-100% active ingredient, in one embodiment 0.1-95%, in another embodiment 75-85%.

Compositions for Oral Administration. Oral pharmaceutical dosage forms are either solid, gel or liquid. The solid dosage forms are tablets, capsules, granules, and bulk powders. Types of oral tablets include compressed, chewable lozenges and tablets which can be enteric-coated, sugar-coated or film-coated. Capsules can be hard or soft gelatin capsules, while granules and powders can be provided in non-effervescent or effervescent form with the combination of other ingredients known to those skilled in the art.

Solid Compositions for Oral Administration. In some embodiments, the formulations are solid dosage forms, in some embodiments, capsules or tablets. The tablets, pills, capsules, troches and the like can contain one or more of the following ingredients, or compounds of a similar nature: a binder; a lubricant; a diluent; a glidant; a disintegrating agent; a coloring agent; a sweetening agent; a flavoring agent; a wetting agent; an emetic coating; and a film coating. Examples of binders include microcrystalline cellulose, gum tragacanth, glucose solution, acacia mucilage, gelatin solution, molasses, polyinylpyrrolidine, povidone, crospovidones, sucrose, and starch paste. Lubricants include talc, starch, magnesium or calcium stearate, lycopodium, and stearic acid. Diluents include, for example, lactose, sucrose, starch, kaolin, salt, mannitol, and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar, and carboxymethylcellulose. Coloring agents include, for example, any of the approved certified water-soluble FD and C dyes, mixtures thereof; and water insoluble FD and C dyes suspended on alumina hydrate. Sweetening agents include sucrose, lactose, mannitol, and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate, and polyoxyethylene laural ether. Emetic-coatings include fatty acids, fats, waxes, shellac, ammoniated shellac and cellulose acetate phthalates. Film coatings include hydroxyethylcellulose, gellan gum, sodium carboxymethylcellulose, polyethylene glycol 4000 (PEG4000), and cellulose acetate phthalate.

The compound, or pharmaceutically acceptable derivative thereof, could be provided in a composition that protects it from the acidic environment of the stomach. For example, the composition can be formulated in an enteric coating that maintains its integrity in the stomach and releases the active compound in the intestine. The composition can also be formulated in combination with an antacid or other such ingredient. When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup,

wafer, sprinkle, chewing gum, or the like. A syrup can contain, in addition to the active compounds, sucrose as a sweetening agent and certain preservatives, dyes, colorings, and/or flavors.

The active materials can also be mixed with other active materials which do not impair the desired action, or with materials that supplement the desired action, such as antacids, $H_2$ blockers, and diuretics. The active ingredient is a compound or pharmaceutically acceptable derivative thereof as described herein. Higher concentrations, up to about 98% by weight of the active ingredient can be included.

In some embodiments, tablets and capsules formulations can be coated as known by those of skill in the art in order to modify or sustain dissolution of the active ingredient. Thus, for example, they can be coated with a conventional enterically digestible coating, such as phenylsalicylate, waxes and cellulose acetate phthalate.

Liquid Compositions for Oral Administration. Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil-in-water or water-in-oil.

Elixirs are clear, sweetened, hydroalcoholic preparations. Pharmaceutically acceptable carriers used in elixirs include solvents. Syrups are concentrated aqueous solutions of a sugar, for example, sucrose, and can contain a preservative. An emulsion is a two-phase system in which one liquid is dispersed in the form of small globules throughout another liquid. Pharmaceutically acceptable carriers used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms. Solvents include glycerin, sorbitol, ethyl alcohol, and syrup. Examples of preservatives include glycerin, methyl and propylparaben, benzoic acid, sodium benzoate, and alcohol. Examples of non-aqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, xanthan gum, Veegum, and acacia. Sweetening agents include sucrose, syrups, glycerin, and artificial sweetening agents such as saccharin. Wetting agents include propylene glycol monostearate, sorbitan monooleate, diethylene glycol monolaurate, and polyoxyethylene lauryl ether. Organic acids include citric and tartaric acid. Sources of carbon dioxide include sodium bicarbonate and sodium carbonate. Coloring agents include any of the approved certified water-soluble FD and C dyes, and mixtures thereof. Flavoring agents include natural flavors extracted from plants such fruits, and synthetic blends of compounds which produce a pleasant taste sensation. For a solid dosage form, the solution or suspension, in for example propylene carbonate, vegetable oils, and/or triglycerides, is in some embodiments encapsulated in a gelatin capsule. Such solutions, and the preparation and encapsulation thereof, are disclosed in U.S. Patent Nos. 4,328,245; 4,409,239; and 4,410,545. For a liquid dosage form, the solution, e.g., for example, in a polyethylene glycol, can be diluted with a sufficient quantity of a pharmaceutically acceptable liquid carrier, e.g., water, to be easily measured for administration.

Alternatively, liquid or semi-solid oral formulations can be prepared by dissolving and/or dispersing the active compound or salt in vegetable oils, glycols, triglycerides, propylene glycol esters (e.g., propylene carbonate), and/or other such carriers, and/or encapsulating these solutions or suspensions in hard or soft gelatin capsule shells. Other useful formulations include those set forth in U.S. Patent No. RE28,819 and U.S. Patent No. 4,358,603. Briefly, such formulations include, but are not limited to, those containing a compound provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(loweralkyl)acetals of loweralkyl aldehydes such as acetaldehyde diethyl acetal.

Injectables, Solutions and Emulsions. Parenteral administration, in some embodiments characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. The injectables, solutions and emulsions also contain one or more excipients. Suitable excipients are, for example, water, saline, dextrose, glycerol, or ethanol. In addition, if desired, the pharmaceutical compositions to be administered can also contain minor amounts of non-toxic auxiliary substances such as wetting or emulsifying agents, pH buffering agents, stabilizers, solubility enhancers, and other such agents, such as for example,

sodium acetate, sorbitan monolaurate, triethanolamine oleate, and cyclodextrins.

Implantation of a slow-release or sustained-release system, such that a constant level of dosage is maintained (see e.g., U.S. Patent No. 3,710,795) is also contemplated herein. Briefly, a compound provided herein is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol, and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers, with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the compound and the needs of the subject.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions can be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents, and other pharmaceutically acceptable substances.

Examples of aqueous vehicles include Sodium Chloride Injection, Ringers Injection, Isotonic Dextrose Injection, Sterile Water Injection, Dextrose, and Lactated Ringers Injection. Nonaqueous parenteral vehicles include fixed oils of vegetable origin, cottonseed oil, corn oil, sesame oil, and peanut oil. Antimicrobial agents in bacteriostatic or fungistatic concentrations can be added to parenteral preparations packaged in multiple-dose containers which include phenols or cresols, mercurials, benzyl alcohol, chlorobutanol, methyl and propyl p-hydroxybenzoic acid esters, thimerosal, benzalkonium chloride, and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, xanthan gum, hydroxypropyl methylcellulose, and polyvinylpyrrolidone. Emulsifying agents include Polysorbate 80 (sold under the tradename TWEEN® 80). A sequestering or chelating agent of metal ions includes EDTA. Pharmaceutical carriers also include ethyl alcohol, polyethylene glycol and propylene glycol for water miscible vehicles; and sodium hydroxide, hydrochloric acid, citric acid, or lactic acid for pH adjustment.

The concentration of the pharmaceutically active compound can be adjusted so that an injection provides an effective amount to produce the desired pharmacological effect. The exact dose depends on the age, weight and condition of the patient or animal as is known in the art.

The unit-dose parenteral preparations are packaged in an ampoule, a vial, or a syringe with a needle. All preparations for parenteral administration should be sterile, as is known and practiced in the art.

Illustratively, intravenous or intraarterial infusion of a sterile aqueous solution containing an active compound is an effective mode of administration. Another embodiment is a sterile aqueous or oily solution or suspension containing an active material injected as necessary to produce the desired pharmacological effect.

Injectables are designed for local and systemic administration. In one embodiment, a therapeutically effective dosage is formulated to contain a concentration of at least about 0.1% w/w up to about 90% w/w or more, in certain embodiments more than 1% w/w of the active compound to the treated tissue(s).

The compound can be suspended in micronized or other suitable form or can be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the condition and can be empirically determined.

Lyophilized Powders. Lyophilized powders, which can be reconstituted for administration as solutions, emulsions and other mixtures, can also be used to carry out the presently disclosed subject matter. They can also be reconstituted and formulated as solids or gels.

The sterile, lyophilized powder is prepared by dissolving a compound provided herein, or a pharmaceutically

acceptable derivative thereof, in a suitable solvent. The solvent can contain an excipient which improves the stability or other pharmacological component of the powder or reconstituted solution, prepared from the powder. Excipients that can be used include, but are not limited to, dextrose, sorbital, fructose, corn syrup, xylitol, glycerin, glucose, sucrose, or other suitable agent. The solvent can also contain a buffer, such as citrate, sodium, or potassium phosphate or other such buffer known to those of skill in the art at, in some embodiments, about neutral pH. Subsequent sterile filtration of the solution followed by lyophilization under standard conditions known to those of skill in the art provides the desired formulation. In some embodiments, the resulting solution will be apportioned into vials for lyophilization. Each vial can contain a single dosage or multiple dosages of the compound. The lyophilized powder can be stored under appropriate conditions, such as at about 4°C to room temperature.

Reconstitution of this lyophilized powder with water for injection provides a formulation for use in parenteral administration. For reconstitution, the lyophilized powder is added to sterile water or another suitable carrier. The precise amount depends upon the selected compound. Such amount can be empirically determined.

Topical Administration. Topical mixtures are prepared as described for the local and systemic administration. The resulting mixture can be a solution, suspension, emulsions or the like, and are formulated as creams, gels, ointments, emulsions, solutions, elixirs, lotions, suspensions, tinctures, pastes, foams, aerosols, irrigations, sprays, suppositories, bandages, dermal patches, or any other formulations suitable for topical administration.

The compounds or pharmaceutically acceptable derivatives thereof can be formulated as aerosols for topical application, such as by inhalation (see e.g., U.S. Patent Nos. 4,044,126; 4,414,209; and 4,364,923, which describe aerosols for delivery of a steroid useful for treatment of inflammatory diseases, particularly asthma). These formulations for administration to the respiratory tract can be in the form of an aerosol or solution for a nebulizer, or as a microfine powder for insufflation, alone or in combination with an inert carrier such as lactose. In such a case, the particles of the formulation will, in some embodiments, have diameters of less than 50 microns, in some embodiment less than 10 microns.

The compounds can be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the active compound alone or in combination with other pharmaceutically acceptable excipients can also be administered. These solutions, particularly those intended for ophthalmic use, can be formulated as 0.01%-10% isotonic solutions, pH about 5-7, with appropriate salts.

Compositions for other Routes of Administration. Other routes of administration, such as transdermal patches, including iontophoretic and electrophoretic devices, and rectal administration, are also contemplated herein.

Transdermal patches, including iotophoretic and electrophoretic devices, are well known to those of skill in the art. For example, such patches are disclosed in U.S. Patent Nos. 6,267,983; 6,261,595; 6,256,533; 6,167,301; 6,024,975; 6,010715; 5,985,317; 5,983,134; 5,948,433 and 5,860,957.

For example, pharmaceutical dosage forms for rectal administration are rectal suppositories, capsules, and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax (polyoxyethylene glycol) and appropriate mixtures of mono-, di-, and triglycerides of fatty acids. Combinations of the various bases can be used. Agents to raise the melting point of suppositories include spermaceti and wax. Rectal suppositories can be prepared either by the compressed method or by molding. The weight of a rectal suppository is in some embodiments about 2 to 3 grams.

Tablets and capsules for rectal administration are manufactured using the same pharmaceutically acceptable substance and by the same methods as for formulations for oral administration.

Targeted Formulations. The compounds provided herein, or pharmaceutically acceptable derivatives thereof, can also be formulated to be targeted to a particular tissue, receptor, infecting agent, or other area of the body of the subject to be treated. Many such targeting methods are well known to those of skill in the art. All such targeting methods are contemplated herein for use in the instant compositions. For non-limiting examples of targeting methods, see e.g., U.S. Patent Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

Liposomes. In some embodiments, liposomal suspensions, including tissue-targeted liposomes, such as tumor-targeted liposomes, can also be suitable as pharmaceutically acceptable carriers. These can be prepared according to methods known to those skilled in the art. For example, liposome formulations can be prepared as described in U.S. Patent No. 4,522,811. Briefly, liposomes such as multilamellar vesicles (MLV's) can be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation,

and then resuspended in PBS.

Ligands. In some embodiments, the disclosed compounds can be targeted to specific target tissues or target compositions using ligands specific for the target tissue or target composition, for example, using ligands or ligand-receptor pairs such as antibodies and antigens. Antibodies against tumor antigens and against pathogens are known. For example, antibodies and antibody fragments which specifically bind markers produced by or associated with tumors or infectious lesions, including viral, bacterial, fungal, and parasitic infections, and antigens and products associated with such microorganisms have been disclosed, inter alia, in U.S. Patent Nos. 3,927,193; 4,331,647; 4,348,376; 4,361,544; 4,468,457; 4,444,744; 4,818,709; and 4,624,846. Antibodies against an antigen, e.g., a gastrointestinal, lung, breast, prostate, ovarian, testicular, brain or lymphatic tumor, a sarcoma or a melanoma, can be used.

A wide variety of monoclonal antibodies against infectious disease agents have been developed and are summarized in a review by Polin (1984) European Journal of Clinical Microbiology 3(5):387-398, showing ready availability. These include monoclonal antibodies (MAbs) against pathogens and their antigens such as the following: Antibacterial Mabs such as those against Streptococcus agalactiae, Legionella pneumophilia, Streptococcus pyogenes, Esherichia coli, Neisseria gonorrhosae, Neisseria meningitidis, Pneumococcus, Hemophilis influenzae B, Treponema pallidum, Lyme disease, spirochetes, Pseudomonas aeruginosa, Mycobacterium leprae, Brucella abortus, Mycobacterium tuberculosis, Tetanus toxin, Anti-protozoan Mabs such as those against Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Mesocestoides corti, Emeria tenella, Onchocerca volvulus, Leishmania tropica, Trichinella spiralis, Theileria parva, Taenia hydatigena, Taenia ovis, Taenia saginata, Antiviral MAbs such as those against HIV-1, -2, and -3, Hepatitis A, B, C, D, Rabies virus, Influenza virus, Cytomegalovirus, Herpes simplex I and II, Human serum parvo-like virus, Respiratory syncytial virus, Varicella-Zoster virus, Hepatitis B virus, Measles virus, Adenovirus, Human T-cell leukemia viruses, Epstein-Barr virus, Mumps virus, Sindbis virus, Mouse mammary tumor virus, Feline leukemia virus, Lymphocytic choriomeningitis virus, Wart virus, Blue tongue virus, Sendai virus, Reo virus, Polio virus, Dengue virus, Rubella virus, Murine leukemia virus, Antimycoplasmal MAbs such as those against Acholeplasma laidlawii, Mycoplasma arthritidis, M. hyorhinis, M. orale, M. arginini, M. pneumonia; etc.

Suitable MAbs have been developed against most of the micro-organisms (bacteria, viruses, protozoa, other parasites) responsible for the majority of infections in humans, and many have been used previously for in vitro diagnostic purposes. These antibodies, and newer MAbs that can be generated by conventional methods, are appropriate for use as target agents with the compounds provided herein.

MAbs against malaria parasites can be directed against the sporozoite, merozoite, schizont and gametocyte stages. Monoclonal antibodies have been generated against sporozoites (circumsporozoite antigen) and have been shown to neutralize sporozoites in vitro and in rodents. See Yoshida et al. (1980) Science 207:71-73. Monoclonal antibodies to T. gondii, the protozoan parasite involved in toxoplasmosis have been developed. See Kasper et al. (1982) Journal of Immunology 129:1694-1699. MAbs have been developed against schistosomular surface antigens and have been found to act against schistosomulae in vivo or in vitro. See Simpson et al. (1981) Parasitology 83:163-177; Smith et al. (1982) Parasitology 84:83-91; Gryzch et al. (1982) Journal of Immunology 129:2739-2743; Zodda et al. (1982) Journal of Immunology 129:2326-2328; and Dissous et al. (1982) Journal of Immunology 129:2232-2234.

Mixtures of antibodies and immunoglobulin classes can be used, as can hybrid antibodies. Multispecific, including bispecific and hybrid, antibodies and antibody fragments are especially preferred in the methods of the presently disclosed subject matter for detecting and treating target tissue and are comprised of at least two different substantially monospecific antibodies or antibody fragments, wherein at least two of said antibodies or antibody fragments specifically bind to at least two different antigens produced or associated with the targeted lesion or at least two different epitopes or molecules of a marker substance produced or associated with the target tissue. Multispecific antibodies and antibody fragments with dual specificities can be prepared analogously to the anti-tumor marker hybrids disclosed in U.S. Patent No. 4,361,544. Other techniques for preparing hybrid antibodies are disclosed in, e.g., U.S. Patent Nos. 4,474,893 and 4,479,895, and in Milstein et al. (1984) Immunology Today 5:299.

Antibody fragments useful in the presently disclosed subject matter include $F(ab')_2$, $F(ab)_2$, Fab', Fab, Fv and the like including hybrid fragments. Preferred fragments are Fab', $F(ab')_2$, Fab, and $F(ab)_2$. Also useful are any subfragments retaining the hypervariable, antigen-binding region of an immunoglobulin and having a size similar to or smaller than a Fab' fragment. This can include genetically engineered and/or recombinant proteins, whether single-chain or multiple-chain, which incorporate an antigen-binding site and otherwise function in vivo as targeting vehicles in substantially the same way as natural immunoglobulin fragments. Such single-chain binding molecules are disclosed in U.S. Patent No. 4,946,778. Fab' antibody fragments can be conveniently made by reductive cleavage of $F(ab')_2$ fragments, which themselves can be made by pepsin digestion of intact immunoglobulin. Fab antibody fragments can be made by papain digestion of intact immunoglobulin, under reducing conditions, or by cleavage of $F(ab)_2$ fragments which result from careful papain digestion of whole immunoglobulin.

A ligand or one member of a ligand-receptor binding pair can be conjugated to the compounds provided herein for targeting the compounds to specific target tissues or target compositions. Examples of ligand-receptor binding pairs

are set out in U.S. Patent Nos. 4,374,925 and 3,817,837.

Conjugation to ligands. Many compounds that can serve as targets for ligand-receptor binding pairs, and more specifically, antibodies, have been identified, and the techniques to construct conjugates of such ligands with compounds of Formula (I) are well known to those of ordinary skill in this art. For example, Rakestraw et al. teaches conjugating Sn(IV) chlorin via covalent bonds to monoclonal antibodies using a modified dextran carrier. See Rakestraw et al. (1990) Proceedings of the National Academy of Science of the United States of America 87:4217-4221. The compounds disclosed herein can also be conjugated to a ligand, such as an antibody, by using a coupling agent. Any bond which is capable of linking the components such that they are stable under physiological conditions for the time needed for administration and treatment is suitable, but covalent linkages are preferred. The link between two components can be direct, e.g., where a compound of Formula (I) is linked directly to a targeting agent, or indirect, e.g., where a compound of Formula (I) is linked to an intermediate and that intermediate being linked to the targeting agent.

A coupling agent should function under conditions of temperature, pH, salt, solvent system, and other reactants that substantially retain the chemical stability of the photosensitizer, the backbone (if present), and the targeting agent. Coupling agents should link component moieties stably, but such that there is only minimal or no denaturation or deactivation of the compound of Formula (I) or the targeting agent. Many coupling agents react with an amine and a carboxylate, to form an amide, or an alcohol and a carboxylate to form an ester. Coupling agents are known in the art. See e.g., Bodansky (1993) Principles of Peptide Synthesis, 2nd ed., Springer & Hermanson (1996) Bioconjugate Techniques, 1st Ed., Academic Press, New York, New York, United States of America.

The conjugates of the compounds provided herein with ligands such as antibodies can be prepared by coupling the compound to targeting moieties by coupling a carboxylic acid or ester moiety on the compound via peptide linkages to the antibody through an N terminus, or by other methods known in the art. A variety of coupling agents, including cross-linking agents, can be used for covalent conjugation. Examples of cross-linking agents include N,N'-dicyclohexylcarbodiimide (DCC), N-succinimidyl-5-acetyl-thioacetate (SATA), N-succinimidyl-3-(2-pyridyldi-thio)propionate (SPDP), ortho-phenylene-dimaleimide (o-PDM), and sulfosuccinimidyl 4-(N-maleimido-methyl)-cyclohexane-1-carboxylate (sulfo-SMCC). See e.g., Karpovsky et al. (1984) Journal of Experimental Medicine 160(6):1686-1701; and Liu et al. (1985) Proceedings of the National Academy of Science of the United States of America 82(24):8648-8652. Other methods include those described by Brennan et al. (1985) Science 229:81-83 and Glennie et al. (1987) Journal of Immunology 139:2367-2375.

For example, DCC is a useful coupling agent that can be used to promote coupling of the alcohol NHS to a chlorin carboxylic acid group in DMSO forming an activated ester which can be cross-linked to polylysine. DCC is a carboxy-reactive cross-linker commonly used as a coupling agent in peptide synthesis and has a molecular weight of 206.32. Another useful cross-linking agent is SPDP, a heterobifunctional cross-linker for use with primary amines and sulfhydryl groups. SPDP has a molecular weight of 312.4, a spacer arm length of 6.8 angstroms, is reactive to NHS-esters and pyridyldithio groups, and produces cleavable cross-linking such that, upon further reaction, the agent is eliminated so the photosensitizer can be linked directly to a backbone or targeting agent. Other useful conjugating agents are SATA for introduction of blocked SH groups for two-step cross-linking, which is deblocked with hydroxylamine-HCl, and sulfo-SMCC, reactive towards amines and sulfhydryls. Other cross-linking and coupling agents are also available from Pierce Chemical Co. Additional compounds and processes, particularly those involving a Schiff base as an intermediate, for conjugation of proteins to other proteins or to other compositions, for example to reporter groups or to chelators for metal ion labeling of a protein, are disclosed in European Patent EP 0 243 929 B1.

Photosensitizers which contain carboxyl groups can be joined to lysine ε-amino groups in the target polypeptides either by preformed reactive esters (such as N-hydroxy succinimide (NHS) ester) or esters conjugated in situ by a carbodiimide-mediated reaction. The same applies to photosensitizers which contain sulfonic acid groups, which can be transformed to sulfonyl chlorides which react with amino groups. Chlorins which have carboxyl groups can be joined to amino groups on the polypeptide by an in situ carbodiimide method. Chlorins can also be attached to hydroxyl groups, of serine or threonine residues or to sulfhydryl groups of cysteine residues.

Methods of joining components of a conjugate, e.g., coupling polyamino acid chains bearing photosensitizers to antibacterial polypeptides, can use heterobifunctional cross-linking reagents. These agents bind a functional group in one chain and to a different functional group in the second chain. These functional groups typically are amino, carboxyl, sulfhydryl, and aldehyde. There are many permutations of appropriate moieties which will react with these groups and with differently formulated structures, to conjugate them together. See Hermanson (1996) Bioconjugate Techniques, 1st Ed., Academic Press, New York, New York, United States of America; and Merrifield et al. (1994) Ciba Foundation Symposium 186:5-20.

The compounds or pharmaceutically acceptable derivatives thereof can be packaged as articles of manufacture containing packaging material, a compound or pharmaceutically acceptable derivative thereof provided herein, which is effective for modulating the activity of hyperproliferating tissue or neovascularization, or for treatment, prevention or amelioration of one or more symptoms of hyperproliferating tissue or neovascularization mediated diseases or disorders, or diseases or disorders in which hyperproliferating tissue or neovascularization activity, is implicated, within the packaging material, and a label that indicates that the compound or composition, or pharmaceutically acceptable derivative thereof, is used for modulating the activity of hyperproliferating tissue or neovascularization, or for treatment, prevention or amelioration of one or more symptoms of hyperproliferating tissue or neovascularization mediated diseases or disorders, or diseases or disorders in which hyperproliferating tissue or neovascularization is implicated.

The articles of manufacture provided herein contain packaging materials. Packaging materials for use in packaging pharmaceutical products are well known to those of skill in the art. See e.g., U.S. Patent Nos. 5,323,907; 5,052,558; and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions provided herein are contemplated as are a variety of treatments for any disease or disorder in which hyperproliferating tissue or neovascularization is implicated as a mediator or contributor to the symptoms or cause.

## V. Photodynamic Therapy, Diagnostic and Therapeutic Applications

In some embodiments, the presently disclosed compounds of Formula (I) (or their pharmaceutically acceptable salts or conjugates) can act as a photosensitizer in a method of treating a disease (e.g., a hyperproliferative disease, such as cancer) involving photodynamic therapy (PDT). Briefly, the photosensitizing compound, conjugate or pharmaceutical composition thereof is generally administered to the subject before a target tissue, target composition or subject is subjected to illumination with light. The photosensitizing compound is administered as described elsewhere herein.

The dose of photosensitizing compound can be determined clinically. Depending on the photosensitizing compound used, an equivalent optimal therapeutic level will have to be established. A certain length of time is allowed to pass for the circulating or locally delivered photosensitizer to be taken up by the target tissue. The unbound photosensitizer is cleared from the circulation during this waiting period, or additional time can optionally be provided for clearing of the unbound compound from non-target tissue. The waiting period can be determined clinically and can vary from compound to compound.

At the conclusion of this waiting period, a laser light source or a non-laser light source (including but not limited to artificial light sources such as fluorescent or incandescent light, or natural light sources such as ambient sunlight) is used to activate the bound drug. The area of illumination is determined by the location and dimension of the pathologic region to be detected, diagnosed, or treated. The duration of illumination period can depend on whether detection or treatment is being performed and can be determined empirically. A total or cumulative period of time anywhere from between about 4 minutes and about 72 hours can be used. In some embodiments, the illumination period is between about 60 minutes and 148 hours. In some embodiments, the illumination period is between about 2 hours and 24 hours.

The total fluence or energy of the light used for irradiating, as measured in Joules, is in some embodiments between about 10 Joules and about 25,000 Joules; in some embodiments between about 100 Joules and about 20,000 Joules; and in some embodiments between about 500 Joules and about 10,000 Joules. Light of a wavelength and fluence sufficient to produce the desired effect is selected, whether for detection by fluorescence or for therapeutic treatment to destroy or impair a target tissue or target composition. Light having a wavelength corresponding at least in part with the characteristic light absorption wavelength of the photosensitizing agent is preferably used for irradiating the target issue.

The intensity or power of the light used is measured in watts, with each Joule equal to one watt-sec. Therefore, the intensity of the light used for irradiating in the presently disclosed methods can be substantially less than 500 $mW/cm^2$. Since the total fluence or amount of energy of the light in Joules is divided by the duration of total exposure time in seconds, the longer the amount of time the target is exposed to the irradiation, the greater the amount of total energy or fluence can be used without increasing the amount of the intensity of the light used. The presently disclosed subject matter employs an amount of total fluence of irradiation that is sufficiently high to activate the photosensitizing agent.

In some embodiments of using compounds disclosed herein for photodynamic therapy, the compounds are injected into the mammal, e.g. human, to be diagnosed or treated. The level of injection is usually between about 0.1 and about 0.5 $\mu$mol/kg of body weight. In the case of treatment, the area to be treated is exposed to light at the desired wavelength and energy, e.g. from about 10 to 200 $J/cm^2$. In the case of detection, fluorescence is determined upon exposure to light at a wavelength sufficient to cause the compound to fluoresce at a wavelength different than that used to illuminate the compound. The energy used in detection is sufficient to cause fluorescence and is usually significantly lower than is required for treatment.

Any one of the photosensitizing compounds disclosed herein or a pharmaceutically acceptable derivative thereof can be supplied in a kit along with instructions on conducting any of the methods disclosed herein. Instructions can be in any tangible form, such as printed paper, a computer disk that instructs a person how to conduct the method, a video cassette containing instructions on how to conduct the method, or computer memory that receives data from a remote location and illustrates or otherwise provides the instructions to a person (such as over the Internet). A person can be instructed in how to use the kit using any of the instructions above or by receiving instructions in a classroom or in the

course of treating a patient using any of the methods disclosed herein, for example.

Additional examples and specific examples of methods of using compounds and compositions of the presently disclosed subject matter include but are not limited to the following:

(i) Treatment of opportunistic infections. Compounds, compositions, and methods of the presently disclosed subject matter are useful for PDT of opportunistic infections, particularly of soft tissue. For antimicrobial treatment (via PDT) of infections, particularly wound infections, the infecting organism can include (as nonlimiting examples) Staphylococcus aureus, Pseudomonas aeruginosa, Escherichia coli. In nosocomial infections, P. aeruginosa is responsible for 8% of surgical-wound infections and 10% of bloodstream infections. In some embodiments the subjects are immunocompromised subjects, such as those afflicted with AIDS or undergoing treatment with immunosuppressive agents.

(ii) Treatment of burns. Infections by S. aureus and gram-positive bacteria in general are particularly pronounced in burns. The multidrug resistance of S. aureus presents significant medical challenges. In this regard, compounds, compositions, and methods of the presently disclosed subject matter are useful for the treatment of opportunistic infections of burns.

(iii) Sepsis. Compounds, compositions, and methods of the presently disclosed subject matter are useful for the PDT treatment of subjects afflicted with opportunistic infections of Vibrio vulnificus. V. vulnificus, a gram-negative bacterium, causes primary sepsis, wound infections, and gastrointestinal illness in humans.

(iv) Ulcers. Compounds, compositions, and methods of the presently disclosed subject matter are useful for PDT treatment of the bacterium that causes ulcers (Helicobacter pylori). In the clinic, treatment can be effected in any suitable manner, such as by insertion of a fiber optic cable (akin to an endoscope but with provisions for delivery of red or near-IR light) into the stomach or afflicted region.

(v) Periodontal disease. Compounds, compositions, and methods of the presently disclosed subject matter are useful in PDT for the treatment of periodontal disease, including gingivitis. Periodontal disease is caused by the overgrowth of bacteria, such as the gram-negative anaerobe Porphyromonas gingivalis. As with many PDT treatments, targeting or solubilizing entities in conjunction with the photoactive species are essential for appropriate delivery of the photoactive species to the desired cells. The oral pathogens of interest for targeting include Porphyromonas gingivalis, Actinobacillus actinonzycetemcomitans, Bacteroides forsythus, Campylobacter rectus, Eikenella corrodens, Fusobacterium nucleatum subsp. Polymorphum, Actinomyces viscosus, and the streptococci. For such applications the compounds or compositions of the presently disclosed subject matter can be topically applied (e.g., as a mouthwash or rinse) and then light administered with an external device, in-the-mouth instrument, or combination thereof.

(vi) Atherosclerosis. Compounds, compositions, and methods of the presently disclosed subject matter are useful in PDT to treat vulnerable atherosclerotic plaque. Without wishing to be bound to any particular theory, invading inflammatory macrophages are believed to secrete metalloproteinases that degrade a thin layer of collagen in the coronary arteries, resulting in thrombosis, which often is lethal. Active compounds targeted to such inflammatory macrophages are useful for PDT of vulnerable plaque.

(vii) Cosmetic and dermatologic applications. Compounds, compositions, and methods of the presently disclosed subject matter are useful in PDT to treat a wide range of cosmetic dermatological problems, such as hair removal, treatment of psoriasis, or removal of skin discoloration. Ruby lasers are currently used for hair removal; in many laser treatments melanin is the photosensitized chromophore. Such treatments work reasonably well for fair-skinned individuals with dark hair. Compounds, compositions and methods of the presently disclosed subject matter can be used as near-IR sensitizers for hair removal, which enables targeting a chromophore with a more specific and sharp absorption band.

(viii) Acne. Compounds, compositions, and methods of the presently disclosed subject matter are useful in PDT to treat acne. Acne vulgaris is caused by Propionibacterium acnes, which infects the sebaceous gland; some 80% of young people are affected. Here again, the growing resistance of bacteria to antibiotic treatment is leading to an upsurge of acne that is difficult to treat. Current PDT treatments of acne typically rely on the addition of aminolevulinic acid, which in the hair follicle or sebaceous gland is converted to free base porphyrins. Compounds and compositions of the presently disclosed subject matter can be administered to subjects topically or parenterally (e.g., by subcutaneous injection) depending upon the particular condition.

(ix) Infectious diseases. Compounds, compositions, and methods of the presently disclosed subject matter are useful in PDT to treat infectious diseases. For example, Cutaneous leishmaniasis and sub-cutaneous leishmaniasis, which occurs extensively in the Mediterranean and Mideast regions, is currently treated with arsenic-containing compounds. PDT has been used to reasonable effect recently, at least in one case, on a human patient. The use of compounds and compositions of the presently disclosed subject matter are likewise useful, and potentially offer advantages such as ease of synthesis and better spectral absorption properties.

(x) Tissue sealants. Compounds, compositions, and methods of the presently disclosed subject matter are useful

in PDT as tissue sealants in subjects in need thereof. Light-activated tissue sealants are attractive for sealing wounds, bonding tissue, and closing defects in tissue There are many applications where sutures or staples are undesirable and use of such mechanical methods of sealing often lead to infection and scarring.

(xi) Neoplastic disease. Compounds, compositions, and methods of the presently disclosed subject matter are useful in PDT for treating neoplastic diseases or cancers, including skin cancer, lung cancer, colon cancer, breast cancer, prostate cancer, cervical cancer, ovarian cancer, basal cell carcinoma, leukemia, lymphoma, squamous cell carcinoma, melanoma, plaque-stage cutaneous T-cell lymphoma, and Kaposi sarcoma.

In addition to PDT, the compositions provided herein can be used as imaging enhancing agents in diagnostic imaging techniques, or for the labeling of target tissues or target compositions for diagnostic radiology. In the modern medical field, there are a variety of treatments including magnetic resonance imaging (MRI) for the diagnosis of diseases. Detection of cancer in its early stages should improve the ability to cure eliminate the cancerous tissue. Early diagnosis of precancerous regions and minute cancer are important subject matters in modern cancer treatments. MRI has emerged as a powerful tool in clinical settings because it is noninvasive and yields an accurate volume rendering of the subject. The image is created by imposing one or more orthogonal magnetic field gradients upon the subject or specimen while exciting nuclear spins with radio frequency pulses as in a typical nuclear magnetic resonance (NMR) experiment. After collection of data with a variety of gradient fields, deconvolution yields a one, two, or three-dimensional image of the specimen/subject. Typically, the image is based on the NMR signal from the protons of water where the signal intensity in a given volume element is a function of the water concentration and relaxation times. Local variation in these parameters provide the vivid contrast observed in MR images.

MRI contrast agents act by increasing the rate of relaxation, thereby increasing the contrast between water molecules in the region where the imaging agent accretes and water molecules elsewhere in the body. However, the effect of the agent is to decrease both $T_1$ and $T_2$, the former resulting in greater contrast while the latter results in lesser contrast. Accordingly, the phenomenon is concentration-dependent, and there is normally an optimum concentration of a paramagnetic species for maximum efficacy. This optimal concentration can vary with the particular agent used, the locus of imaging, the mode of imaging, i.e., spin-echo, saturation-recovery, inversion-recovery and/or various other strongly Ti-dependent or $T_2$-dependent imaging techniques, and the composition of the medium in which the agent is dissolved or suspended. These factors, and their relative importance are known in the art. See e.g., Pykett (1982) Scientific American 246:78; and Runge et al. (1983) American Journal of Radiology 141:1209. When MRI contrast agents are used diagnostically, they are vascularly perfused, enhancing the contrast of blood vessels and reporting on organ lesions and infiltration. However, the labeling of specific tissues for diagnostic radiology remains a difficult challenge for MRI. Efforts to develop cell and tissue-specific MRI image enhancing agents by modifying existing immunological techniques has been the focus of much research in diagnostic radiology. For example, antibodies labeled with paramagnetic ions, generally the gadolinium chelate Gd-DTPA, have been generated and tested for their effects on MRI contrast of tumors and other tissues. See U.S. Patent No. 5,059,415. Unfortunately, the relaxivity of Gd bound to antibodies has been found to be only slightly better than that of unbound Gd-DTPA. See Paajanen et al. (1990) Magnetic Resononance in Medicine 13:38-43.

MRI is generally used to detect $^{1}$H nuclei in the living body. However, MRI is capable of detecting NMR spectrums of other nuclear species, including $^{13}$C, $^{15}$N, $^{31}$P, and $^{19}$F. The $^{19}$F is not abundant in the living body. By incorporating isotopes useful in MRI, such as $^{13}$C, $^{15}$N, $^{31}$P, or $^{19}$F, and particularly $^{19}$F in the compositions provided herein and administering to a subject, the compounds provided herein would accumulate in target tissue, and subsequent MR imaging would produce NMR data with enhanced signal from the targeted tissue or target compositions due to the presence of the accumulated compound with the MRI recognizable isotope, such as $^{19}$F. Thus, the disclosed compounds can be used as image enhancing agents and provide labeling of specific target tissues or target compositions for diagnostic radiology, including MRI.

In addition to PDT, the compositions provided herein can be used to detect target cells, target tissue, or target compositions in a subject. When the compounds provided herein are to be used for detection of target tissue or target composition, the compounds are introduced into the subject and sufficient time is allowed for the compounds to accumulate in the target tissue or to become associated with the target composition. The area of treatment is then irradiated, generally using light of an energy sufficient to cause fluorescence of the compound, and the energy used is usually significantly lower than is required for photodynamic therapy treatment. Fluorescence is determined upon exposure to light at the desired wavelength, and the amount of fluorescence can be correlated to the presence of the compound, qualitatively or quantitatively, by methods known in the art.

The compositions provided herein can also be used to diagnose the presence of an infecting agent, or the identity of an infecting agent in a subject. The compounds provided herein can be conjugated to one or more ligands specific for an infecting agent, such as an antibody or antibody fragment, that selectively associates with the infecting agent, and after allowing sufficient time for the targeted compound to associate with the infecting agent and to clear from non-target tissue, the compound can be visualized, such as by exposing to light of an energy sufficient to cause

fluorescence of the compound, or by imaging using diagnostic radiology, including MRI. By way of example, any one of the compounds provided herein can be conjugated to an antibody that is targeted against a suitable Helicobacter pylori antigen, and formulated into a pharmaceutical preparation that, when introduced into a subject, releases the conjugated compound to a gastric mucus/epithelial layer where the bacterium is found. After sufficient time for the compound to selectively associate with the target infecting agent, and for any unbound compound to clear from non-target tissue, the subject can be examined to determine whether any Helicobacter pylori is present. This can be done by MRI to detect accumulated compound because of the presence of $^{19}F$ substituents, for example, or by irradiating the suspect target area with light of an energy sufficient to cause fluorescence of the compound, such as by using fiberoptics, and detecting any fluorescence of the targeted compound.

In some embodiments, the presently disclosed compounds or their conjugates can be useful in flow cytometry. Flow cytometry is known and described in, for example, U.S. Patent Nos. 5,167,926; 5,915,925; 6,248,590; 6,589,792; and 6,890,487. In some embodiments, the particle being detected, such as a cell, is labelled with a luminescent compound such as a phosphor or fluorophore for detection. Labelling can be carried out by any suitable technique such as coupling the luminescent compound to another compound such as an antibody which in turn specifically binds to the particle or cell, by uptake or internalization of the luminescent compound into the cell or particle, by non-specific adsorption of the luminescent compound to the cell or particle, etc. The active compounds described herein are useful in flow cytometry as such luminescent compounds, which flow cytometry techniques (including fluorescent activated cell sorting or FACS) can be carried out in accordance with known techniques or variations thereof which will be apparent to those skilled in the art based upon the instant disclosure.

## EXAMPLES

The following Examples provide illustrative embodiments. In light of the present disclosure and the general level of skill in the art, those of skill will appreciate that the following Examples are intended to be exemplary only and that numerous changes, modifications, and alterations can be employed without departing from the scope of the presently disclosed subject matter.

## EXAMPLE 1

### Synthesis of Compound CP-1

Di-BOC-protected Suzuki coupling partner 1 (CP-1) was prepared as shown in Scheme 3, Figure 3.

**1-Bromo-3,5-bis(bromomethyl)benzene** (CP-1a). N-Bromosuccinimide (NBS, 35.60 g, 200.0 mmol) was added to a flame-dried 3-necked 1 L round bottom flask (RBF) with stir bar and the flask was fitted with a glass stopper, septum topped condenser, and a rubber septum. The NBS was dried under high vacuum for 30 min, then the flask was flushed with argon and acetonitrile (ACN, 400 mL) was added via cannula to approx. half volume (-450 mL). 1-Bromo-3,5-dimethylbenzene (15.26 g, 80.0 mmol) was added via syringe, followed by brief opening of the system under argon flow and bulk addition of solid azobisisobutyronitrile (AIBN, 0.670 g, 4.00 mmol). The flask was heated to gentle reflux (oil bath set at 90°C) under argon.

After 16 h, the reaction mixture was transferred to a single neck 1 L RBF and concentrated to remove ACN. The solid residue was dried further under high vac, suspended in dichloromethane (DCM, 75 mL), and heated to a gentle boil. The mixture was allowed to equilibrate to room temperature and was filtered with DCM wash. The filtrate was concentrated, dried under high vacuum, and recrystallized in ethanol (EtOH, 55 mL total) with heating in water bath set to 65°C. Solid was filtered and washed with ice-chilled EtOH, then dried under high vac. Isolated compound CP-1a as a white crystalline solid (17.40 g, 51%).

$^1H$ NMR (400 MHz, $CDCl_3$) $\delta$ 4.41 (s, 4H), 7.34 (s, 1H), 7.47 (d, J = 2.0 Hz, 2H).

2,2'-((5-Bromo-1,3-phenylene)bis(methylene))bis(isoindoline-1,3-dione) (CP-1b). Compound CP-1a (18.43 g, 53.75 mmol) was dried in a 500 mL RBF with stir bar. The flask was flushed with argon and dimethylformamide (DMF, 215 mL, 0.25 M) was added. The clear, colorless solution was stirred and potassium phthalimide (23.37 g, 123.63 mmol) was added in portions. The flask was fitted with a condenser topped with a drying tube and heated in an oil bath set to 90°C.

After 16 h, the mixture was cooled to room temp, diluted with water (1 L total), and extracted with chloroform (400, 300, and 200 mL, 1 × each). The organic layers were combined and washed with 0.2 N aq. NaOH (500 mL) and water (500 mL). The organic layer was separated, dried with sodium sulfate, filtered, and concentrated. The solid was dried further under high vac then transferred to a filter and washed with room temp diethyl ether ($Et_2O$) (3x). Isolated compound **CP-1b** as a white powdery solid (17.98 g, 70%).

$^1H$ NMR (400 MHz, $CDCl_3$) $\delta$ 4.78 (s, 4H), 7.42 - 7.47 (m, 3H), 7.78 - 7.70 (m, 4H), 7.87 - 7.82 (m, 4H).

**(5-Bromo-1,3-phenylene)dimethanamine (CP-1c)** Compound **CP-1b** (7.63 g, 16.06 mmol) was suspended in EtOH (70.0 mL) and heated in 85°C oil bath. Hydrazine hydrate (4.88 mL, 80.29 mmol) was added in bulk and the

flask was topped with a condenser. The mixture was heated further at reflux temp for 15 min, then the reaction mixture was allowed to cool gradually to room temp.

6 N aq. HCl was added until the solution was acidic by litmus test (20 mL total). The resulting mixture was heated to reflux temp again. The flask was flushed with argon, stirred for 1 h, and then allowed to cool and chill in an ice bath. The mixture was filtered to give a clear, pale amber solution. The filtrate was cooled in an ice bath and basified with 2 N aq. NaOH (30 mL total). The aqueous layer was extracted with chloroform (3 × 75 mL). The organic layers were combined, washed with brine, dried over sodium sulfate, filtered, and concentrated to approx. 10 mL volume. A white residue was noted on walls of the flask. The remaining solution was filtered, and the filtrate was concentrated to give 2.3 g yellowish oil. Stored in refrigerator.

The sample solidified after overnight storage at 4°C and dried further under high vacuum to give 2.047 g (59%) of compound **CP-1c** as an amber semi-solid.

**Di-tert-butyl ((5-bromo-1,3-phenylene)bis(methylene))dicarbamate (CP-1d).** Compound **CP-1c** (2.00 g, 9.11 mmol) was added to a flame-dried 250 mL RBF with stir bar. The flask was evacuated and argon flushed. Tetrahydrofuran (THF, 45 mL) was added and the flask was lowered into a water bath. Diisopropylethylamine (3.83 mL, 21.86 mmol) was added and the heterogeneous mixture was chilled in an ice bath. Boc anhydride (4.86 g, 21.86 mmol) was prepared as a solution in THF (10 mL) and added dropwise in 1 mL portions. The solution was stirred at 0°C for 1 h, then allowed to equilibrate to room temp.

Reaction was allowed to stir at room temp overnight. The mixture was concentrated to give a white solid, which was re-dissolved in ethyl acetate (EtOAc, 70 mL). The organic layer was washed with sat. aq. $NH_4Cl$, water, sat. aq. $NaHCO_3$, and brine (1 × 50 mL each). Then, the organic layer was dried with sodium sulfate, filtered, and concentrated to a pale amber oil that crystallized on standing. The solid was washed in frit filter with chilled 1:1 $Et_2O$/hexanes. Solid dried under high vacuum to give 3.50 g (93%) compound **CP-1d** as a white powdery solid.

**Coupling Partner 1 (CP-1).** Dimethyl sulfoxide (DMSO, reagent grade, 20.0 mL) was added to a 100 mL RBF and argon was bubbled with stirring for a total of 45 min. Compound **CP-1d** (1.25 g, 3.01 mmol), bis(binacolato)diboron (0.917 g, 3.61 mmol), potassium acetate (0.886 g, 9.03 mmol), and Pd(dppf)$Cl_2$ (0.066 g, 0.090 mmol) were added together in a dry 250 mL RBF and the flask was evacuated for 30 min. The flask was flushed with argon and de-gassed DMSO was added. The solution was frozen in a dry ice/acetone bath and placed under vacuum, then allowed to thaw under argon. The reaction mixture was then heated in oil bath at 85°C.

After 16 h, the reaction mixture was cooled to room temperature, diluted in EtOAc (100 mL), and washed with brine (3 × 100 mL). The organic layer was dried over sodium sulfate, filtered, and concentrated.

The concentrate was added neat with minimal DCM rinse onto a 40 g silica column and eluted with 0 - 2% MeOH in DCM. Main product fractions were combined and concentrated to give a clear oil. Product solidified upon further drying under high vacuum with a stir bar present. Isolated compound **CP-1** as a white, waxy solid (1.224 g, 88%).

EXAMPLE 2

Synthesis of Compound **C-1**

**4-Bromopyrrole-2-carboxaldehyde (C-1a).** As shown in Scheme 4, Figure 4, a stirred solution pyrrole-2-carboxaldehyde (70.0 g, 737 mmol) in THF (737 mL) was cooled to 0°C under argon atmosphere in a 2 L round bottom flask. NBS (133 g, 737 mmol, reagent grade, un-recrystallized) was added all at once. The reaction mixture was stirred for 15 min at 0°C under argon before the solvent was removed on a rotary evaporator.

The resulting solid was dried under high vacuum for 2 h. Water (370 mL) was added to the flask and the suspension was filtered with a Büchner funnel. The filter cake was washed with an additional 370 mL of water. The solid filtered material was transferred to a 2 L Erlenmeyer flask and dissolved in 180 mL of hot ethanol (78°C) by refluxing in a hot water bath. Hot water (1400 mL, 100°C) was added all at once. Upon cooling to room temperature, the product crystallized from solution. The mixture was further cooled to - 10.0°C for 2 - 4 h to promote more crystallization. The mixture was filtered by vacuum filtration and was dried under high vacuum for 24-48 h to give light brown crystals of 4-bromopyrrole-2-carboxaldehyde **(C-1a,** 104 g, 81%).

$^1H$ NMR (400 MHz, $CDCl_3$) δ 6.91-7.03 (m, 1H), 7.05-7.17 (m, 1H), 9.48 (s, 1H), 9.68 (br s, 1H).

**4-Bromo-2-formyl-N-tosylpyrrole (C-1b).** A suspension of 90% NaH (10.3 g, 429 mmol) in anhydrous THF (352 mL) was stirred in a 1 L oven dried round bottom flask, vacuum evacuated, and cooled to 0°C under argon. The mixture was treated portion wise over ~15 min with compound **C-1a** (62.0 g, 356 mmol). The mixture was stirred for 30 min at 0°C before treating with p-toluenesulfonyl chloride (67.9 g, 356 mmol). The reaction was stirred at room temperature for 3 h, whereupon water (200 mL) was slowly added to quench the reaction.

Ethyl acetate (200 mL) was added, and the organic layer was separated and was washed with brine (100 mL), dried (~50 g $Na_2SO_4$), filtered, and concentrated to an oily liquid on a rotary evaporator. The oily liquid was dried under high vacuum overnight in a 1 L round bottom flask. The resulted crude solid was dissolved in hexanes/ethyl acetate

(600 mL, 5:1) by refluxing in a hot water bath. Upon allowing to cool to room temperature, the product crystallized from the solution. The mixture was further cooled for 3 h to -10 °C to promote additional crystallization. The mixture was filtered by vacuum filtration and the filtered brown crystal were dried under high vacuum to give compound **C-1b** (94 g, 81%).

$^1H$ NMR (400 MHz, $CDCl_3$) δ 2.44 (s, 3H), 6.77 (s, 1H), 7.31 (d, J = 13.40 Hz, 1H), 7.36 (d, J = 8.25 Hz, 2H), 7.60 (s, 1H), 7.76 (d, J = 8.25 Hz, 2H), 8.44 (d, J = 13.40 Hz, 1H).

**4-Bromo-2-(2-nitrovinyl)-N-tosylpyrrole (C-1c).** A stirred mixture of 4-bromo-2-formyl-N-tosylpyrrole **(C-1b,** 84.2 g, 257 mmol), potassium acetate (20.1 g, 205 mmol), methylamine hydrochloride (13.8 g, 205 mmol), and acetic acid (1.00 mL) in -99.5% ethanol, pure (90.0 mL) in a 500 mL round bottom flask was treated with nitromethane (34.6 mL, 641 mmol). The mixture was stirred for 2 h, whereupon water was added (200 mL) and the resulting yellow precipitate was filtered by vacuum filtration. The solid filtered material was washed with water (500 mL) followed by cold ethanol (1.2 L, 0°C) until eluent was clear. The yellow filtered solid was dried overnight under high vacuum to give 4-bromo-2-(2-nitrovinyl)-N-tosylpyrrole **(C-1c,** 79 g, 83%).

$^1H$ NMR (400 MHz, $CDCl_3$) δ 2.44 (s, 3H), 6.77 (s, 1H), 7.31 (d, J = 13.40 Hz, 1H), 7.36 (d, J = 8.25 Hz, 2H), 7.60 (s, 1H), 7.76 (d, J = 8.25 Hz, 2H), 8.44 (d, J = 13.40 Hz, 1H).

**4-Bromo-2-(2-nitroethyl)-N-tosylpyrrole (C-1d).** A solution of compound **C-1c** (74.0 g, 199 mmol) in anhydrous THF (1 L) was cooled to -10°C (ice-acetone (1:1)) under argon in a 2 L round bottom flask. The solution was treated with 95% $LiBH_4$ (4.34 g, 199 mmol) all at once under vigorous stirring. The reaction mixture was stirred for 30 min at - 10°C, until all starting material had disappeared. Upon completion, the reaction mixture was quenched by slowly adding a cold saturated aqueous $NH_4Cl$ solution (340 mL, 0°C). The mixture was stirred for 5 min and extracted with ethyl acetate (340 mL), dried (43 g of anhydrous $Na_2SO_4$), concentrated to dark brown solid on a rotary evaporator, and dried under high vacuum for 2 h in 2 L round bottom flask. The crude solid material was dissolved in isopropyl alcohol (IPA, 1.2 L) by refluxing in a hot water bath. Upon cooling to room temperature, the product crystallized from solution. The mixture was further cooled for 4 h at -10°C to promote more crystallization. The mixture was filtered by vacuum filtration, and the filtered light brown crystals were dried under high vacuum overnight to give compound **C-1d** (47 g, 62%).

$^1H$ NMR (400 MHz, $CDCl_3$) δ 2.44 (s, 3H), 3.39 (t, J = 7.01 Hz, 2H), 4.60 (t, J = 7.01 Hz, 2H), 6.10 (d, J = 1.93 Hz, 1H), 7.32 (d, J = 1.93 Hz, 1H), 7.35 (d, J = 7.98 Hz, 2H), 7.69 (d, J = 7.98 Hz, 2H).

**6-(4-Bromo-1-tosyl-1H-pyrrol-2-yl)-4,4-dimethyl-5-nitrohexan-2-one (C-1e).** A mixture of compound **C-1d** (13.3 g, 35.7 mmol) and 1,1-dimethoxy-4-methyl-3-penten-2-one (10.5 g, 107.0 mmol, 3.0 equiv.) in a RBF was treated with 1,8-diazabicyclo[5.4.0]undec-7-ene (DBU, 16 mL, 107.0 mmol, 3.0 equiv.) The resulting mixture was stirred at rt for 1 h, diluted with EtOAc. The organic layer was washed with (3X water, brine), dried ($Na_2SO_4$) and concentrated. Any excess 1,1-dimethoxy-4-methyl-3-penten-2-one was removed under high vacuum for 16 h. The resulting crude product was dissolved in a minimum amount of $CH_2Cl_2$ (8 mL) and prepared as a silica cake. The cake was eluted on a 120 g $SiO_2$ column with 25-40% EtOAc in hexanes gradient over for 58 min. The major fractions were combined, concentrated, and dried under high vacuum rotavapor to give 11.4 g (68%) **C-1e** as a brown solid.

$^1H$ NMR (400 MHz, $CDCl_3$) δ 1.11 (s, 3H), 1.24 (s, 3H), 2.13 (s, 3H), 2.40 (AB, J = 17.8 Hz, 1H), 2.43 (s, 3H), 2.55 (AB, J = 17.8 Hz, 1H), 3.18 (AB, J = 16.2 Hz, 1H), 3.36 (ABX, 3J = 16.2 Hz, 2J = 11.8 Hz, 1H), 5.14 (AB, J = 11.8 Hz, 1H), 6.00-6.02 (m, 1H), 7.22-7.24 (m, 1H), 7.34 (AB, J = 8.2 Hz, 2H), 7.64 (AB, J = 8.2 Hz, 2H).

**4-Bromo-1-tosyl-2-((3,3,5-trimethyl-3,4-dihydro-2H-pyrrol-2-yl)methyl)-1H-pyrrole (C-1f).** A solution of compound **C-1e** (10.5 g, 22.3 mmol) in THF (105 mL) in a RBF was treated with $HCO_2NH_4$ (28.1 g, 446 mmol) and zinc powder (28.3 g, 446 mmol). The resulting suspension was stirred vigorously at room temperature for 2 h. The reaction mixture was filtered through a pad of silica (50 g) glass frit. The filter cake was eluted with EtOAc (500 mL). The filtrate was concentrated to fluffy, light brown solid. The residue was dissolved in $CH_2Cl_2$ and prepared as a silica cake. The cake was eluted on a 120 g $SiO_2$ column with 75-80% hexanes in EtOAc for 60 min. The single major product was combined, concentrated and dried under high vacuum to give 6.29 g (67%) **C-1f** as a light brown solid.

$^1H$ NMR (400 MHz, $CDCl_3$) δ 0.88 (s, 3H), 1.07 (s, 3H), 1.97 (s, 3H), 2.28 (AB, J = 16.8 Hz, 1H), 2.36 (AB, J = 16.8 Hz, 1H), 2.41 (s, 3H), 2.63 (ABX, 2J = 16.1 Hz, 3J = 10.2 Hz, 1H), 2.92 (ABX, 2J = 16.1 Hz, 3J = 3.8 Hz, 1H), 3.67-3.70 (m, 1H), 6.25-6.28 (m, 1H), 7.28-7.30 (m, 3H), 7.68 (AB, J = 8.2 Hz, 2H).

**4-Bromo-2-((3,3,5-trimethyl-3,4-dihydro-2H-pyrrol-2-yl)methyl)-1H-pyrrole (C-1g).** Compound **C-1f** (2.79 g, 6.59 mmol) in RBF was treated with tetra-n-butylammonium fluoride (TBAF, 19.8 mL, 1.0 M in THF, 19.8 mmol, 3 equiv.) and the reaction was stirred at reflux (64-67°C) for 1 h. A saturated solution of aqueous $NaHCO_3$ (68 mL) was added followed by the ethyl acetate (68 mL). The mixture was extracted with ethyl acetate (134 mL). The organic layer was dried (anhyd. $Na_2SO_4$), concentrated to a dark brown oil on rotary evaporator, and dried under high vacuum for 2 h. The residue was dissolved in DCM and prepared as a silica cake. The cake was eluted on 40 g $SiO_2$ column with 25-33% EtOAc in hexanes gradient over 55 min. Main product fractions were combined, concentrated, and dried under high vacuum to give 1.56 g (88%) compound C-1g as a light brown solid.

$^1H$ NMR (400 MHz, $CDCl_3$) δ 0.92 (s, 3H), 1.11 (s, 3H), 2.03 (s, 3H), 2.28 (AB, J = 16.8 Hz, 1H), 2.38 (AB, J = 16.8 Hz, 1H), 2.54 (ABX, 2J = 14.9 Hz, 3J = 11.8 Hz, 1H), 2.69 (ABX, 2J = 11.8 Hz, 3J = 2.5 Hz, 1H), 3.56-3.62 (m,

1H), 5.85-5.94 (m, 1H), 6.63-6.69 (m, 1H), 9.72-10.01 (br s, 1H).

The synthesis of the Eastern half of the chlorin and its cyclization with the Western half are shown in Scheme 5, Figure 5.

**5-(4-(Methoxycarbonyl)phenyl)dipyrromethane (C-1h).** A mixture of pyrrole (183.9 g, 2741 mmol, 190 mL, 45 equiv.) and methyl-4-formylbenzoate (10.0 g, 60.9 mmol) in a 500 mL RBF was degassed under argon for 30 min. Trifluoacetic acid (TFA, 1.18 mL, 0.25 equiv.) was added and stirred under argon atmosphere for another 30 min. Thin layer chromatography (TLC) on an aliquot of the reaction mixture using $CH_2Cl_2$/hexanes (2:1) showed complete conversion of methyl-4-formyl benzoate to **C-1h.** The reaction mixture was diluted with 400 mL $CH_2Cl_2$ and washed with 200 mL of 0.1 N NaOH. Then, the organic layer was washed with brine (200 mL), dried over (20 g of anhyd. $Na_2SO_4$), filtered and concentrated to dryness under rotary evaporator. The crude solid was recrystallized using 200 mL of methanol/water (10:1) and filtered by vacuum filtration to afford a light brown solid. Multiple recrystallizations using methanol/water (10:1), followed by filtration in a 150 mL glass frit resulted in 13.5 g (79%) of 5-(4-(methoxycarbonyl)phenyl)dipyrromethane **(C-1h).** Alternatively, the residue was dissolved in $CH_2Cl_2$ and prepared as a silica cake. The cake was eluted on 120 g $SiO_2$ column with 75-100% $CH_2Cl_2$ in a hexanes gradient over 70 min. Main product fractions were combined, concentrated, and dried under high vacuum to give a light brown solid.

**1-Formyl-5-(4-(methoxycarbonyl)phenyl)dipyrromethane (C-1i).** Vilsmeier reagent was prepared following a reported procedure. See Laha et al., J. Org. Chem. 2006, 71, 4092-4102. A sample of anhydrous DMF (8.0 mL) was treated with $POCl_3$ (1.90 mL, 1.2 equiv., 20.3 mmol) under argon and stirred for 20 min at 0°C in an oven dried 50 mL round bottomed flask. The resulting mixture was added (via canula) to a second oven dried, vacuum evacuated and argon flushed round bottomed flask (250 mL) containing a solution of 5-(4-(methoxycarbonyl)phenyl)dipyrromethene **(C-1h)** (5.43, 19.4 mmol) in DMF (35 mL) at 0°C . After 1.5 h, saturated $NaHCO_3$ solution (87 mL) was gently added. The resulting mixture was stirred overnight and extracted with ethyl acetate. Organic layers were combined, washed (brine), dried in anhydrous $Na_2SO_4$, filtered, and concentrated. The residue was dissolved in $CH_2Cl_2$ and prepared as a silica cake (10 g). The cake was eluted on an 80 g $SiO_2$ column with 0-40% EtOAc in hexanes gradient over 51 min. Main product fractions were combined, concentrated, and dried under high vacuum to give 3.37 g (56%) **C-1i** as a light brown solid.

**8,9-Dibromo-1-formyl-5-(4-(methoxycarbonyl)phenyl) dipyrromethane (C-1j).** In an oven dried round bottom flask (250 mL), **C-1i** (1.40 g, 4.54 mmol) in THF (45 mL) was treated with recrystallized NBS (1.62g, 9.08 mmol) at -78°C. After 1 h, the cooling bath was removed, the reaction mixture was allowed to warm to -20°C, and a mixture of hexane and water (1:1, 13 mL) was added. The resulting mixture was diluted with ethyl acetate, washed with brine, dried ($Na_2SO_4$), and concentrated. The residue was dissolved in $CH_2Cl_2$ and prepared as a silica cake or slurry (~ 6.0 g). The cake was eluted on 80 g $SiO_2$ column with 0-20% EtOAc in hexane gradient for 86 min. The main product fractions were combined, concentrated, and dried under high vacuum to give 2.00 g (94%) **C-1j** as a yellow solid.

**3,13-Dibromo-10-(4-(methoxycarbonyl)-18,18-dimethylchlorin Zn(II) (C-1k).** A suspension of 3,4,5,6-tetrahydro-1,3,3-trimethyldipyrrin **(C-1g,** 0.63 g, 2.35 mmol) and 8,9-dibromo-1-formyl-5-(4-(methoxycarbonyl)phenyl)-dipyrromethene **(C-1j,** 1.09 g, 2.35 mmol) in $CH_2Cl_2$ (35 mL) was treated with p-toluenesulfonic acid monohydrate (2.23g, 11.7 mmol) in methanol (12 mL) and stirred at room temperature for 40 min. A sample was checked with Uv-vis for a strong broad band at ~480 nm. The resulting mixture was treated with 2,2,6,6-tetramethylpiperidine for 5-10 min (7.92 mL, 46.9 mmol). The reaction mixture was concentrated on a rotary evaporator, and the resulting brown solid was suspended in acetonitrile (235 mL) and treated with zinc acetate (6.47 g, 35.2 mmol), 2,2,6,6-tetramethylpiperidine (15.9 mL, 93.9 mmol), and silver trifluoromethanesulfonate (1.82 g, 7.04 mmol). The resulting suspension was refluxed for 18-24 h. The reaction mixture was cooled and concentrated, and the residue was dissolved in $CH_2Cl_2$ and filtered through a silica bed on a glass frit until the eluant was clear. The residue was prepared as a silica cake and eluted on a 120 g $SiO_2$ column with 50% $CH_2Cl_2$ in hexanes for 3 min and held at that gradient for another 15 min. The gradient was changed to 70% $CH_2Cl_2$ in hexanes for 3 min and held at that gradient for additional 5 min. The gradient was subsequently changed to 80% $CH_2Cl_2$ in hexanes for 5 min and held at that gradient for 20 min. All green fractions were combined after TLC to give **C-1k** as a green solid (318 mg; 20 % yield).

**3,13-Dibromo-10-(4-(methoxycarbonyl)-18,18-dimethylchlorin (C-11). C-1k** (49 mg, 0.070 mmol) was placed in a round bottom flask. To this flask was added a mixture of 5.0 mL $CH_2Cl_2$/TFA (4.90/0.10 ml) and the resulting mixture was stirred for 2 h. The mixture was quenched with saturated aqueous $NaHCO_3$ (20 mL), washed with brine, and dried ($Na_2SO_4$), filtered, and concentrated. The concentrate was recrystallized in hexanes/ $CH_2Cl_2$ (20 mL) to give compound **C-11** as dark powder in (34 mg, 75%).

Compound **C-11** was then used to prepare Compound **C1** through intermediates Compounds **C-1m** and **C-1n** as shown in Scheme 6, Figure 6.

**Suzuki coupled precursor (C-1m).** A mixture of compound **C-11** (63.2 mg, 100 μmol), coupling partner **CP-1** (102 mg, 220 μmol), $Pd(PPh_3)_4$ (69.4 mg, 60.0 μmol) and $Cs_2CO_3$ (196 mg, 60.0 μmol) was placed in a 25 mL RBF. The flask was placed under high vacuum for 1 h and then further de-aerated via three evacuation-refill cycles. Toluene/DMF (2:1, 10 mL total) was added via syringe and the solution was heated at 90°C for 18 h.

After cooling to room temperature, the reaction mixture was diluted with ethyl acetate, washed with aq. $NaHCO_3$, and dried over $Na_2SO_4$. The resulting mixture was concentrated and chromatographed [silica 40 g, hexanes/ethyl acetate (0-75%)] to afford compound **C-1m** (94.9 mg, 83%) as a green solid.

**Suzuki coupled precursor with t-butyl protected β-alanine linker (C-1n).** Compound **C-1m** (38.1 mg, 33.3 μmol) in THF (13.3 mL) and MeOH (6.7 mL) was treated with 1.0 M aqueous NaOH (6.7 mL). The reaction mixture was stirred at room temperature in the dark under argon. After 3.5 h, the reaction mixture was diluted with EtOAc, washed with 1.0% aq. HCl, dried over $Na_2SO_4$, and concentrated.

To the crude product residue, O-(N-succinimidyl)-N,N,N',N'-tetramethyluronium tetrafluoroborate (TSTU, 40.1 mg, 133 μmol), $CH_2Cl_2$ (8.3 mL) and TEA (18.5 μL, 133 μmol) were added. The reaction mixture was stirred at room temperature in the dark under argon. After 1 h, the reaction mixture was diluted with EtOAc, washed with brine, dried over $Na_2SO_4$, and concentrated.

To the resulting residue, β-alanine tert-butyl ester hydrochloride (45.3 mg, 333 μmol) and $Cs_2CO_3$ (109 mg, 333 μmol) were added. The system was evacuated, argon flushed, and $CH_2Cl_2$ (8.3 mL) was added. The reaction mixture was stirred at room temperature in the dark under argon. After 3 h, the reaction mixture was diluted with EtOAc, washed with brine, dried over $Na_2SO_4$, and concentrated. Chromatography [silica 12 g, hexanes/ethyl acetate (0-100%)] afforded compound **C-1n** (40.2 mg, 97%) as a green solid. MS: obsd 1257.04, calcd 1256.68 $[(M + H)^+$, M = $C_{72}H_{89}N_9O_{11}]$.
**C-1.** Compound **C-1n** (12.6 mg, 10.0 μmol) in a 10 mL RBF was treated with 4.0 M HCl in dioxane (2.5 mL). The reaction mixture was stirred at room temperature in the dark under argon. After 1.5 h, the reaction mixture was placed under high vacuum for 16 h.

To the resulting residue, $CH_3(OC_2H_4)_{12}CONHS$ ($mPEG_{12}$-NHS, 54.9 mg, 80.0 μmol) and $Cs_2CO_3$ (52.2 mg, 160 μmol) were added and the flask was evacuated and inert gas flushed. DMF (2.5 mL) was added and the reaction mixture was stirred at room temperature. After 1 h, the reaction mixture was chromatographed [C18 gold, 15.5 g, $H_2O/CH_3CN$ (0-40%)] to afford **C-1** as a green solid (4.0 mg, 13%). The synthesis of C-1 was later scaled up such that starting from compound **C-1n** (155.3 mg, 124 μmol), 252 mg of compound **C-1** was obtained (66% yield).

MS: obsd 1541.94, calcd 1541.86 $[M + 2H]^{2+}$; obsd 1553.24, calcd 1552.85 $[(M + H + Na]^{2+}$; obsd 1563.89, calcd 1563.84 $[(M + 2Na]^{2+}$; $\lambda_{abs}$ 417, 651 nm (PBS); $\lambda_{em}$ 656 nm (PBS). Quantum yield: 26% (PBS); FWHM: 20 nm (PBS).

Chlorin Solubility can be assessed by measuring absorption values in a series of dilutions of a chlorin stock solution. See Jiang et al. (2014) Organic & Biomolecular Chemistry 12:86-103. Solubility of **C-1** was determined to be > 10 mg/mL (PBS).

EXAMPLE 3

Synthesis of Compound **4**

**Sonogashira coupled NIRvana 680 precursor (2).** Compound **4,** also referred to herein as NIRvana 680, was synthesized as set forth in Scheme 7 shown in Figure 7. Compound **C237** was prepared from a mixture of 5-ethynyl-1,3-benzenedicarboxylic acid (500 mg, 2.63 mmol), tert-butyl N-(2-aminoethyl)carbamate (2.08 mL, 13.2 mmol), 1-Ethyl-3-(3-dimethylaminopropyl)carbodiimide (EDCI, 2.00 g, 10.4 mmol) and 4-Dimethylaminopyridine (DMAP, 1.48 g, 13.2 mmol) was dissolved in DMF (3.3 mL). The flask was stirred at room temperature for 16 h. The reaction mixture was directly loaded on silica gel and chromatographed [silica, $CH_2Cl_2$/MeOH (0-10%)] to afford a white solid. The resulting solid was found to contain DMAP by $^1H$ NMR and therefore was re-dissolved in ethyl acetate and washed with aqueous 1.0% HCl, dried over $Na_2SO_4$, and concentrated to afford **C237** as a white solid (938 mg, 75%). A mixture of Compound **1** (60.0 mg, 0.095 mmol), Coupling Partner **C237** (225 mg, 0.474 mmol, 5.0 equiv.), and $PdCl_2(PPh_3)_2$ (17 mg, 0.024 mmol, 0.25 equiv.) was placed in an oven dried 100 mL Schlenk flask. The flask was placed under high vacuum for 0.5 h and then further de-aerated via three evacuation-refill cycles. Toluene/$Et_3N$ (2:1, 9 mL total) was added via syringe and the solution was heated at 85-90 °C for 24 h. After allowing to cool to room temperature, the reaction mixture was diluted with ethyl acetate, washed with aq. $NaHCO_3$, brine, dried over $Na_2SO_4$, and concentrated. The residue was dissolved in a minimum amount of $CH_2Cl_2$ and prepared as a silica cake. The cake was eluted on a 40 g silica column with $CH_2Cl_2$/MeOH (0-1%)] to afford Compound **2** (132 mg, 98%) as a dark solid. MS: $[M + H]^+$ calc. 1419.68; obs. 1419.86.

**Sonogashira coupled NIRvana 680 precursor, t-butyl protected β-alanine linker (3).** Compound **2** (45.8 mg, 32.3 μmol) in THF (6.0 mL) and MeOH (3.0 mL) was treated with 1.0 M aqueous NaOH (3.0 mL). The reaction mixture was stirred at room temperature in the dark. After 4.5 h, the reaction mixture was quenched with 1.0 M aq. HCl (4.5 mL), diluted with EtOAc (25 mL), and washed with brine (25 mL), dried over $Na_2SO_4$ (4.0 g), filtered, concentrated and dried under high vacuum. To the crude product residue, TSTU (19.4 mg, 64.4 μmol, 2.0 equiv.), $CH_2Cl_2$ (10.0 mL, 3.2 mM) and triethylamine (9.0 μL, 194 μmol, 6.0 equiv.) were added. The reaction mixture was stirred at room temperature in the dark under argon for approximately 1 h. To the resulting residue was added β-alanine t-butyl ester hydrochloride (17.6 mg, 96.8 μmol, 3.0 equiv.), triethylamine (27.0 μL, 64.4 μmol, 2.0 equiv.) and the reaction was stirred at room temperature overnight. Additional TEA (54 μL, 12 equiv.), and β-alanine tert-butyl ester hydrochloride (52.8 mg,

9.0 equiv.) were added and the reaction was stirred overnight. The reaction was then heated to reflux for 10 min. The reaction mixture was diluted with $CH_2Cl_2$ (15 mL), washed with brine, and the organic layer was separated and dried with anhyd. $Na_2SO_4$, filtered, and concentrated. Chromatography [silica 12g, was wet-loaded and eluted with $CH_2Cl_2$/MeOH (0-1%)] to afford Compound **3** (38.2 mg, 77%) as a dark solid. MS: $[M + H]^+$ calc. 1532.76; obs. 1533.11.

**NIRvana 680 (Compound 4).** Compound **3** (38.2 mg, 24.9 μmol) in a scintillation vial with a sealed screw-cap was vacuum evacuated and argon flushed. HCl solution (4.0 M in dioxane, 2.5 mL) was added under argon. The reaction mixture was stirred at room temperature in the dark under argon. After 1.5 h, the reaction mixture was placed under a high stream of argon flow with an outlet needle to remove solvent. Upon completion, the vial was dried under vacuum overnight. To the resulting residue, $CH_3O(OC_2H_4)_{12}CH_2CH_2COONHS$ ester (109.1 mg, 149.5 μmol, 6.0 equiv.) and $Cs_2CO_3$ (81.2 mg, 249.2 μmol, 10 equiv.) were added and the flask was evacuated and inert gas flushed. DMF (3.0 mL) was added and the reaction mixture was shielded from light and stirred at room temperature under argon. After 1.5 h, $CH_3O(OC_2H_4)_{12}CH_2CH_2COONHS$ ester (109.1 mg, 149.5 μmol, 6.0 equiv.) and $Cs_2CO_3$ (81.2 mg, 249.2 μmol, 10 equiv.) were added and the vial was vacuum evacuated and argon flushed for 10 min and continue to stir under argon atmosphere. After 3.5 h the reaction was concentrated. The sample was dissolved in 30% ACN in water (2.0 mL) and submitted to reverse phase preparative LC with a 30-80% ACN in water gradient for 45 min. Main product peaks combined, concentrated, transferred to storage vial in ACN, concentrated, and dried under high vacuum to give 17.5 mg (20%) Compound **4** as a green residue. MS: $[M + 2H]^{2+}$ calc. 1767.45; obs. 1767.54.

EXAMPLE 4

Flow Cytometry

Instrumentation: Samples were analyzed on either a nineteen parameter LSR-II SORP flow cytometer (BD Biosciences, San Jose, California, United States of America) equipped with 7 lasers (355, 405, 488, 532, 561, 594, and 633 nm) or a LSRFortessa (BD Biosciences, San Jose, California, United States of America) equipped with 5 lasers (355, 405, 488, 561, and 640 nm) and using FACSDiva 8.0 acquisition software. **C-1** data was collected in channel A of the 100 mW 405 nm laser with a 630 nm longpass (LP) filter and a 660/20 nm bandpass (BP) filter in place. Data for chlorin $H_2C12$-$PEG_6$-NHS used a 635 nm LP and 655/40 nm BP filters.

Antibody Bioconjugation: The NHS ester of **C-1** was prepared using TSTU and TEA in $CH_2Cl_2$ as described above for the preparation of the intermediate NHS ester in the synthesis of **C-1n** and displayed a single peak in HPLC-MS (MS: obsd. 1591 $[M+2H]^{2+}$, 1602 $[M+H+Na]^{2+}$. Solutions were prepared in microcentrifuge tubes from 106 μL of 9.4 mg/mL (1.0 mg) of anti-human CD8 mouse monoclonal antibody (clone UCHT-4, Leinco Technologies, Inc., St. Louis, Missouri, United States of America), 15 μL 1M bicarbonate (pH 8.4), and 44 μL of PEGylated dye NHS ester in PBS (between 5 and 20 molar equivalents). The tubes were shielded from light and gently rotated for 1-2 hours at room temperature. The reaction was quenched by addition of 15 μL of 200 μM Tris at room temperature for a further 1 hour. The bioconjugates were purified using either Sephadex G50M, G75M, or G100M size exclusion chromatography columns eluting with PBS. Antibody bioconjugates prepared from dyes with longer PEG chains (12 units or more) typically were purified with the G75M or G100M media. Column fractions were characterized by absorptions at 280 nm (protein) and the red or NIR dye absorption maxima. Fluorophore to protein (F/P) labeling ratios of pooled fractions were determined from these two maxima with corrections for dye absorption at 280 nm.

Cell Staining: Cryopreserved human peripheral blood mononuclear cells (PBMCs) were obtained from ZenBio, Inc. (Research Triangle Park, North Carolina, United States of America; Product SER-PBMC-F), thawed and prepared for staining according to the vendor's guidelines. Cells were divided into six 1.5 mL microcentrifuge tubes and centrifuged at 400 x g (2000 rpm) for 5 mins. Cells were washed three times with wash buffer (PBS with 0.5% BSA) and resuspended in 0.5 mL of wash buffer. An aliquot was diluted 1:2 with trypan blue and cell number and viability determined by counting the 4 nL square on a hemocytometer. Viability typically was > 96%. The cells were diluted to 1 x $10^6$/mL with wash buffer and 50 μL (500,000 cells) was aliquoted to microcentrifuge tubes. Typically, a maximum labeled antibody concentration was 4.74 μg/5×$10^5$ cells (designated 3.16X) and half-log dilutions prepared. For all except control antibodies, these dilutions were made such that 15 μL was added to cell aliquots. Cells and antibodies were incubated with mixing for 30 mins. at RT. Each tube was washed twice with 1 mL of wash buffer, then re-suspended cells in 0.5 mL wash buffer containing 1% formaldehyde. Samples were filtered through nylon filtration cloth into flow cytometry tubes before characterization by flow cytometry.

As needed, positive control bioconjugates were selected from CD8 (UCHT-4)-fluorescein isothiocyanate (FITC) (Leinco Technologies, Inc., St. Louis, Missouri, United States of America; Catalogue No. C119). CD4 (RPA-T4)-BUV737 antibody (BD Biosciences, San Jose, California, United States of America, Catalogue No. 564306), and/or CD8 (UCHT-4)-DY650 antibody (Leinco Technologies, Inc., St. Louis, Missouri, United States of America, Catalogue No. C2064) and titrated with PBMCs by the same general procedures. The Stain Index (SI) was calculated from mean fluorescence intensity (MFI) values according to Maecker et al. (Cytometry A. 2004, 62, 169-173) as follows:

$$SI = (Mean:positive - Mean:background)/(2 \times S.D.background)$$

Table 1, below, shows the stain index data for titrations of anti-CD8 bioconjugates of **C-1,** as well as for an anti-CD8 bioconjugate of $H_2C12$-$PEG_6$-NHS, a previously described chlorin with three PEG6 chains attached through a 10-phenyl group. See Liu et al., Molecules, 2018, 23, 130. For comparison the anti-CD8 bioconjugate prepared from FITC (Leinco Technologies, Inc., St. Louis, Missouri, United States of America, cat# C119) is also provided.

Table 1

| Stain Index Data for Titrations of Anti-CD8 dye conjugates | | |
|---|---|---|
| **Dye** | **F/P Ratio** | **Maximum Stain Index** |
| $H_2C12$-$PEG_6$-NHS pegylated chlorin | 2.1 | 0 |
| **C-1** | 3.8 | 11.5 |
| FITC | Not determined | 63 |

These results showed a significant enhancement in performance for the PEGylation design of **C-1.**

**Claims**

**1.** A compound of Formula (I):

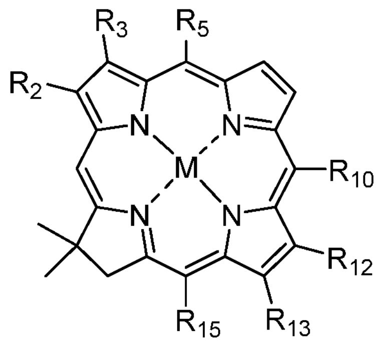

(I)

,

wherein:

M is a metal or is two hydrogens (-H, -H);
$R_5$, and $R_{15}$ are independently selected from H, alkoxy, and a linker group having the formula:

$$-L_1-(X_1-L_2)_p-G;$$

wherein p is 0 or 1; $L_1$ is alkylidene; $X_1$ is -C(=O)NH- or -NHC(=O)-; $L_2$ is - $(CH_2CH_2O)_q$-alkylene-, wherein q is an integer between 1 and 24, alkylene, or substituted alkylene, optionally wherein substituted alkylene is alkylene substituted by a one or more groups comprising a polyoxyethylene chain and/or an amide group; and G is a bioconjugatable group;
$R_{10}$ is:

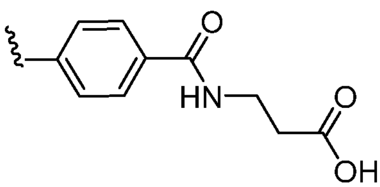

;

and
$R_2$, $R_3$, $R_{12}$, and $R_{13}$ are independently selected from H, cyano, halo, perhaloalkyl, sulfonate, sulfonamide, ester, carboxylic acid, formyl, acetyl, a linker group having the formula $-L_1-(X_1-L_2)_p-G$, and a solubilizing group, wherein the solubilizing group is selected from -aryl-$(R_s)_w$ and -alkynyl-aryl-$(R_s)_w$, wherein w is an integer between 0 and 5 inclusive, and Rs is a group having the formula:

$$-X_2-(L_3)_z-R_{17},$$

wherein: z is 0 or 1; $X_2$ is $-CH_2NHC(=O)-$, -C(=O)NH-alkylene-NH-, or triazolyl; $L_3$ is -C(=O)-alkylene-C(=O)-NH-, and $R_{17}$ is selected from $-(C_2H_4O)_m-R_{18}$, - $C(=O)C_2H_4-(OC_2H_4)_mOR_{18}$, and $-(C_2H_4O)_n-C_2H_4-C(=O)NH-C(R_{19})_3$, wherein m is an integer of 4 or more, optionally wherein m is an integer of 8 or more; n is an integer between 1 and 5; $R_{18}$ is loweralkyl, optionally methyl; and $R_{19}$ is $-CH_2O-C_2H_4-C(=O)NH-(C_2H_4O)_mR_{18}$;
subject to the proviso that at least one of $R_2$, $R_3$, $R_{12}$, and $R_{13}$ is -aryl-$(R_s)_w$ or -alkynyl-aryl-$(R_s)_w$.

2. The compound of claim 1, wherein $R_5$, and $R_{15}$ are selected from H, methoxy, and a linker group having the formula: $-L_1-(X_1-L_2)_p-G$.

3. The compound of claim 1 or claim 2, wherein $R_3$ and $R_{13}$ are each -aryl-$(R_s)_w$, optionally wherein $R_3$ and $R_{13}$ are each -phenyl-$(R_s)_2$.

4. The compound of claim 3, wherein each $R_s$ has a formula $-X_2-(L_3)_z-R_{17}$, wherein: z is 0, $X_2$ is $-CH_2NHC(=O)-$, and $R_{17}$ is $-(C_2H_4O)_m-R_{18}$, wherein m is an integer between 12 and 24

5. The compound of claim 4, wherein $R_3$ and $R_{13}$ are each:

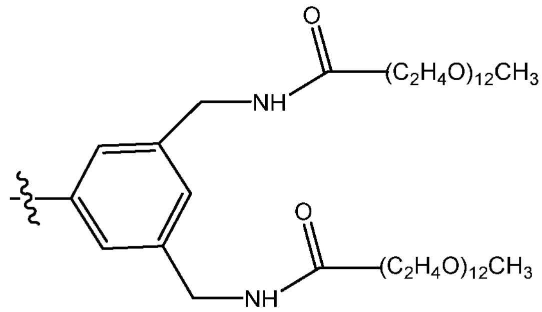

.

6. The compound of any one of claims 1-4, wherein the compound is:

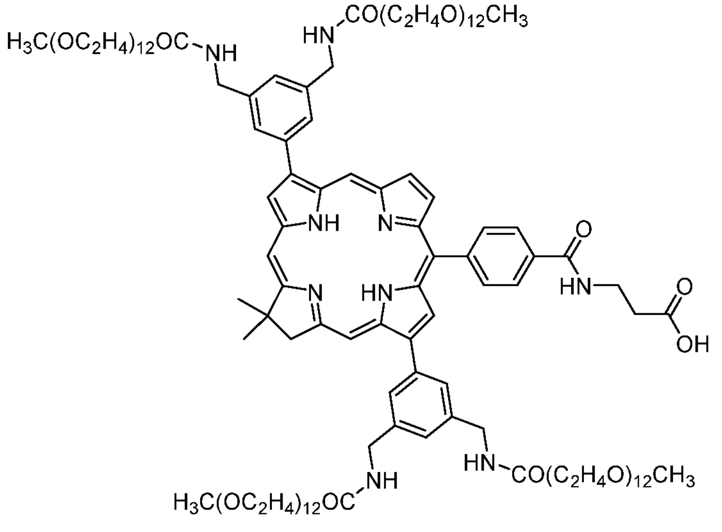

or

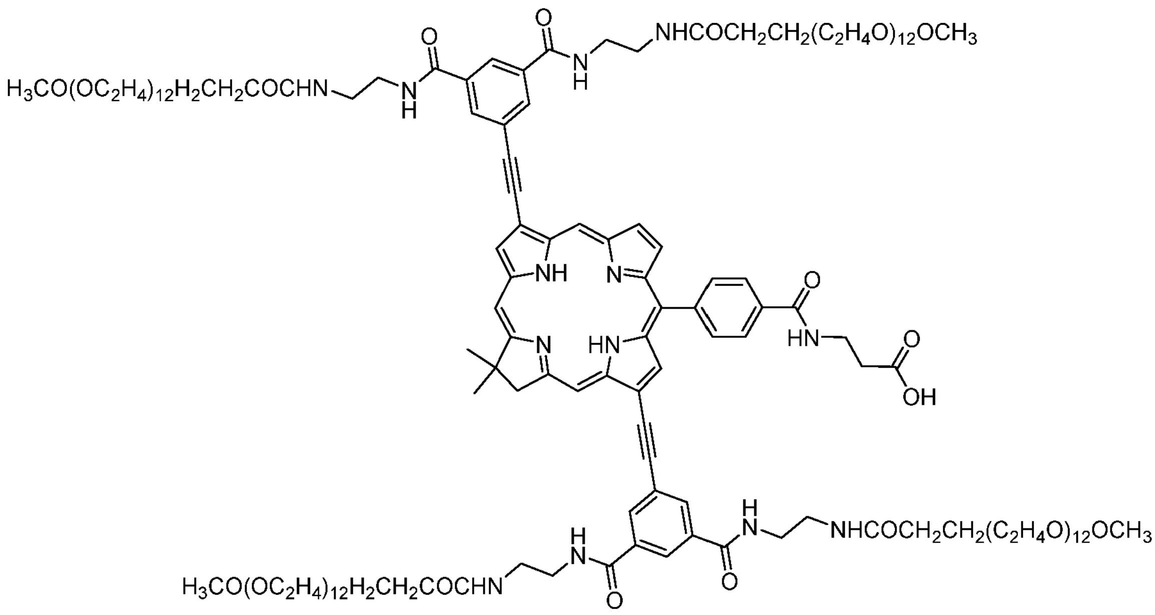

, or a conjugate thereof, wherein the compound is conjugated to one or more of the group consisting of a small molecule, an antigen, a microparticle, a nanoparticle, a polymer, a peptide, a protein, an antibody or an antibody fragment, a nucleic acid, a hormone, and a growth factor.

**7.** A composition comprising a covalent conjugate formed between:

(a) a compound of Formula (I) as defined in claim 1 subject to the proviso that at least one of $R_2$, $R_3$, $R_5$, $R_{10}$, $R_{12}$, $R_{13}$, and $R_{15}$ is a linker group; and
(b) one or more of the group consisting of a small molecule, an antigen, a microparticle, a nanoparticle, a polymer, a peptide, a protein, an antibody or an antibody fragment, a nucleic acid, a hormone, and a growth factor.

**8.** A pharmaceutical composition comprising a compound of claim 1 or a conjugate of claim 7, and a pharmaceutically acceptable carrier.

**9.** A method of detecting a target, wherein said target is a compound, cell or particle, wherein the method comprises labelling the target with a conjugate of claim 7, preferably, wherein the method comprises the use of flow cytometry.

**10.** A method of imaging a cell, a tissue or an organism, wherein the method comprises the use of a compound of claim 1 or a conjugate of claim 7.

**11.** A compound for use in a method of treating a disease in a subject in need of treatment thereof, wherein the compound is a compound of claim 1, a conjugate of claim 7, or a pharmaceutical composition of claim 8, the method comprising:

administering to the subject a compound of claim 1, a conjugate of claim 7, or a pharmaceutical composition of claim 8; and
irradiating at least a portion of the subject with light,
optionally wherein said disease is a hyperproliferative disease, further optionally wherein the disease is cancer.

**12.** The compound of any one of claims 1-6 having a solubility of above about 1 mg/ml in an aqueous solution, optionally having a solubility of about 2.5 mg/ml or more in an aqueous solution; further optionally having a solubility of about 10 mg/ml or more in an aqueous solution.

**13.** A method of preparing a synthetic intermediate of a compound of Formula (I):

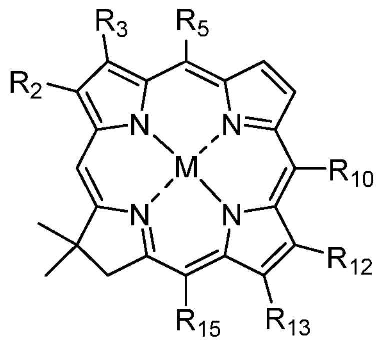

(I)

,

wherein:

M is a metal or is -H, -H;

$R_5$, and $R_{15}$ are independently selected from H, alkoxy, and a linker group having the formula:

$$-L_1-(X_1-L_2)_p-G;$$

wherein p is 0 or 1; $L_1$ is alkylidene; $X_1$ is -C(=O)NH- or -NHC(=O)-; $L_2$ is - $(CH_2CH_2O)_q$-alkylene-, wherein q is an integer between 1 and 24, alkylene, or substituted alkylene, optionally wherein substituted alkylene is alkylene substituted by a one or more groups comprising a polyoxyethylene chain and/or an amide group; and G is a bioconjugatable group;

$R_{10}$ is:

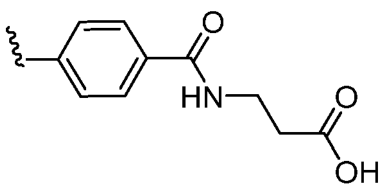

;

and

$R_2$, $R_3$, $R_{12}$, and $R_{13}$ are independently selected from H, halo, cyano, perhaloalkyl, sulfonate, sulfonamide, ester, carboxylic acid, formyl, acetyl, a linker group having the formula $-L_1-(X_1-L_2)_p-G$, and a solubilizing group, wherein the solubilizing group is selected from -aryl-$(R_s)_w$ and -alkynyl-aryl-$(R_s)_w$, wherein w is an integer between 0 and 5 inclusive, and Rs is a group having the formula:

$$-X_2-(L_3)_z-R_{17},$$

wherein: z is 0 or 1; $X_2$ is $-CH_2NHC(=O)-$, -C(=O)NH-alkylene-NH-, or triazolyl; $L_3$ is -C(=O)-alkylene-C(=O)-NH-, and $R_{17}$ is selected from $-(C_2H_4O)_m-R_{18}$, $-C(=O)C_2H_4-(OC_2H_4)_mOR_{18}$, and $-(C_2H_4O)_n-C_2H_4-C(=O)NH-C(R_{19})_3$, wherein m is an integer of 4 or more, optionally wherein m is an integer of 8 or more; n is an integer between 1 and 5; $R_{18}$ is loweralkyl, optionally methyl; and $R_{19}$ is $-CH_2O-C_2H_4-C(=O)NH-(C_2H_4O)_mR_{18}$;

subject to the proviso that at least one of $R_2$, $R_3$, $R_{12}$, and $R_{13}$ is -aryl-$(R_s)_w$ or -alkynyl-aryl-$(R_s)_w$; wherein the method comprises:

(a) providing a compound having the formula (I'):

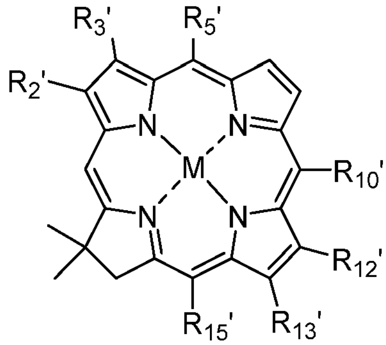,

wherein:

M is a metal or is -H, -H;

$R_5$', and $R_{15}$' are independently selected from H, alkoxy,

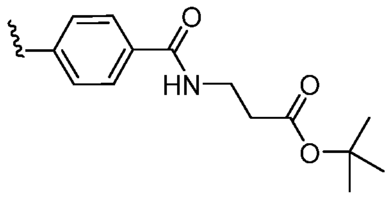, and 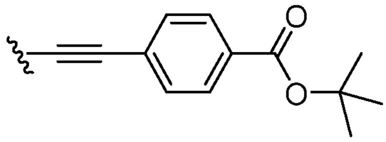;

$R_{10}$' is:

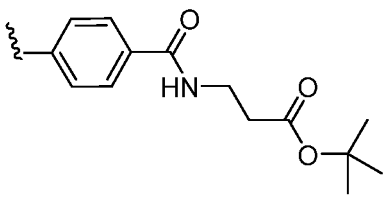;

and

$R_2$', $R_3$', $R_{12}$', and $R_{13}$' are independently selected from H, ester, carboxylic acid, formyl, acetyl,

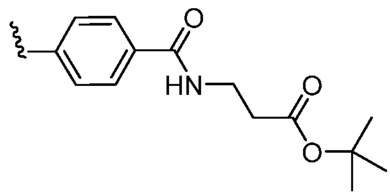, 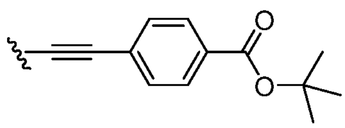,

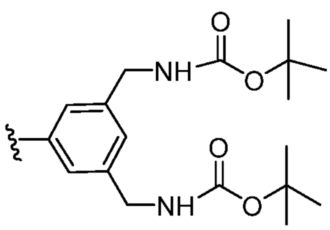 and 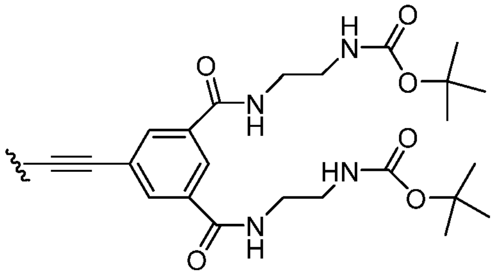;

subject to the proviso that at least one of $R_2$', $R_3$', $R_{12}$', and $R_{13}$' is and

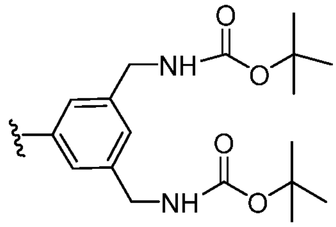

or

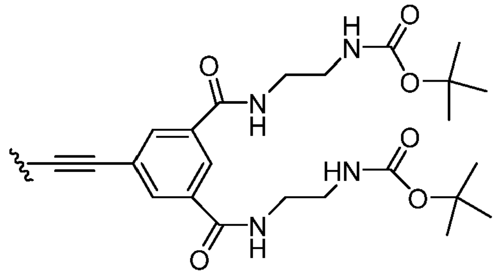

;

(b) contacting the compound provided in step (a) with a solution comprising 4 molar (M) HCl in dioxane to provide a compound of the formula (I"):

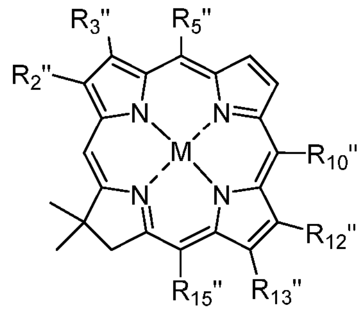

,

wherein:

M is a metal or is -H, -H;
Rs", and $R_{15}$" are independently selected from H, alkoxy,

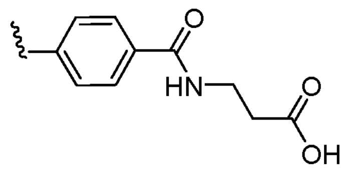

, and

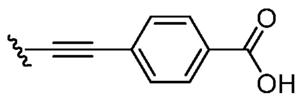

;

R10" is

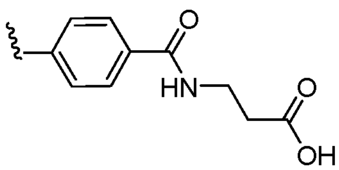

;

and
$R_2$", $R_3$", $R_{12}$", and $R_{13}$" are independently selected from H, ester, carboxylic acid, formyl, acetyl,

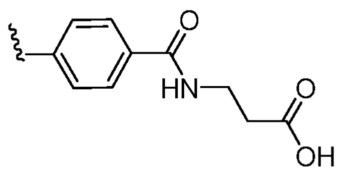

,

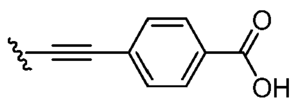

,

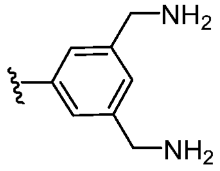 and 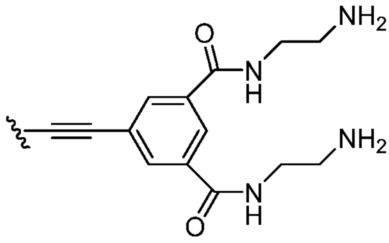 ;

subject to the proviso that at least one of $R_2$", $R_3$", $R_{12}$", and $R_{13}$" is

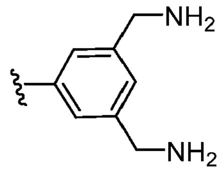 or 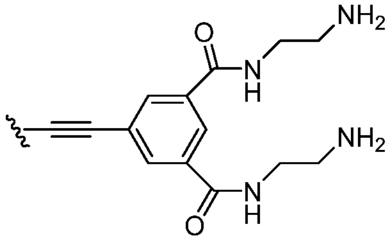 .

**Patentansprüche**

1. Verbindung mit der Formel (I):

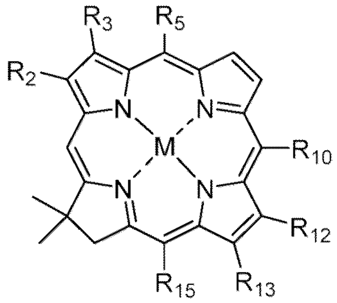

(I)

,

in der:

M ein Metall ist oder zwei Wasserstoffatome (-H, -H);
$R_5$ und $R_{15}$ unabhängig aus H, Alkoxy und einer Verknüpfungsgruppe mit folgender Formel ausgewählt sind:

$$-L_1-(X_1-L_2)_P-G;$$

in der p 0 oder 1 ist; $L_1$ Alkyliden ist; $X_1$ -C(=O)NH- oder -NHC(=O)- ist; $L_2$-$(CH_2CH_2CO)_q$-Alkylen- ist, wobei q eine ganze Zahl zwischen 1 und 24, Alkylen oder ein substituiertes Alkylen ist, wobei optional das substituierte Alkylen ein Alkylen ist, das mit einer oder mehreren Gruppen, die eine Polyoxyethylenkette umfasst bzw umfassen, und/oder einer Amidgruppe substituiert ist; und G eine zur Biokonjugation fähige Gruppe ist;
$R_{10}$

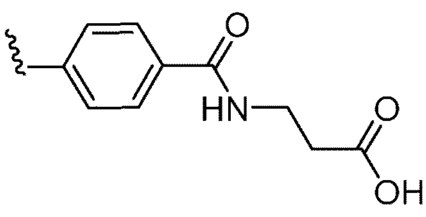 ist;

und

$R_2$, $R_3$, $R_{12}$ und $R_{13}$ unabhängig ausgewählt sind aus H, Cyano, Halogen, Perhaloalkyl, Sulfonat, Sulfonamid, Ester, Carbonsäure, Formyl, Acetyl, einer Verknüpfungsgruppe mit der Formel $-L_1-(X_1-L_2)_P-G$ und einer solubilisierenden Gruppe, wobei die solubilisierende Gruppe ausgewählt ist aus -Aryl-$(R_s)_w$ und -Alkinyl-Aryl-$(R_s)_w$, wobei w eine ganze Zahl zwischen 0 und 5 (jeweils einschließlich) ist und $R_s$ eine Gruppe mit folgender Formel ist:

$$-X_2-(L_3)_z-R_{17},$$

in der: z 0 oder 1 ist; $X_2$ $-CH_2NHC(=O)-$, $-C(=O)NH$-Alkylen-NH- oder Triazolyl ist; $L_3$ $-C(=O)$-Alkylen-$C(=O)-NH-$ ist und $R_{17}$ ausgewählt ist aus $-(CH_2H_4O)_m-R_{18}$, $-C(=O)C_2H_4-(OC_2H_4)_mOR_{18}$ und $-(C_2H_4O)_n-C_2H_4-C(=O)NH-C(R_{19})_3$, wobei m eine ganze Zahl von mindestens 4 ist, wobei m optional eine ganze Zahl von mindestens 8 ist; n eine ganze Zahl zwischen 1 und 5 ist; $R_{18}$ ein niederes Alkyl, optional Methyl ist und $R_{19}$ $-CH_2O-C_2H_4-C(=O)NH-(C_2H_4O)_mR_{18}$ ist;

vorausgesetzt, dass wenigstens einer von $R_2$, $R_3$, $R_{12}$ und $R_{13}$ -Aryl-$(R_s)_w$ oder -Alkinyl-Aryl-$(R_s)_w$ ist.

2. Verbindung nach Anspruch 1, wobei $R_5$ und $R_{15}$ ausgewählt sind aus H, Methoxy und einer Verknüpfungsgruppe mit folgender Formel: $-L_1-(X_1-L_2)_P-G$.

3. Verbindung nach Anspruch 1 oder Anspruch 2, wobei $R_3$ und $R_{13}$ jeweils -Aryl-$(R_s)_w$ sind, wobei optional $R_3$ und $R_{13}$ jeweils -Phenyl-$(R_s)_2$ sind.

4. Verbindung nach Anspruch 3, wobei jeder $R_s$ die Formel $-X_2-(L_3)_z-R_{17}$ aufweist, in der: z 0 ist, $X_2$-$CH_2NHC(=O)$- ist und $R_{17}$ $-(C_2H_4O)_m-R_{18}$ ist, wobei m eine ganze Zahl zwischen 12 und 24 ist.

5. Verbindung nach Anspruch 4, wobei $R_3$ und $R_{13}$ jeweils Folgendes sind:

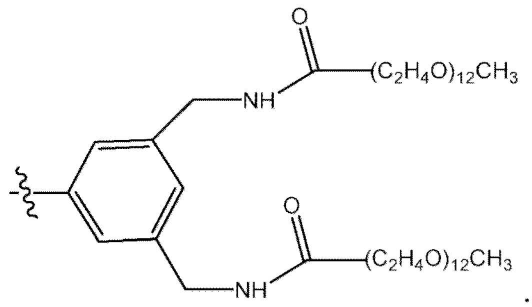

6. Verbindung nach einem der Ansprüche 1 bis 4, wobei die Verbindung:

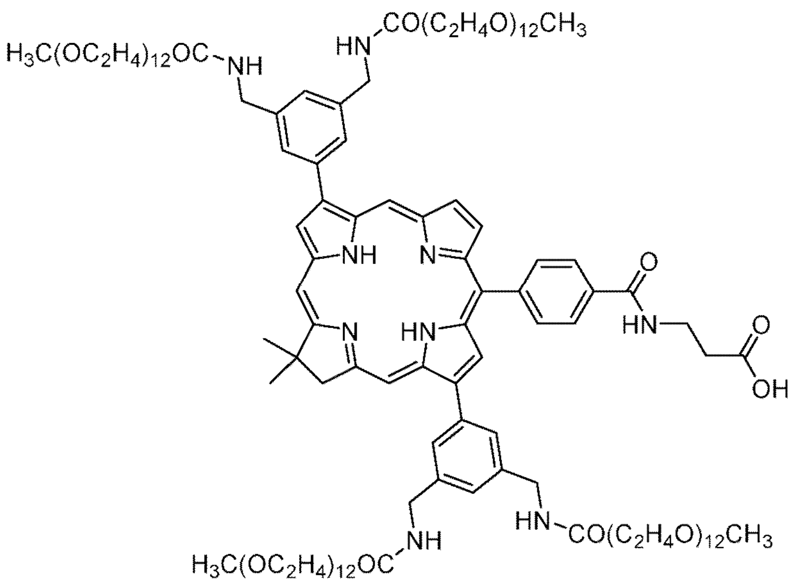

oder

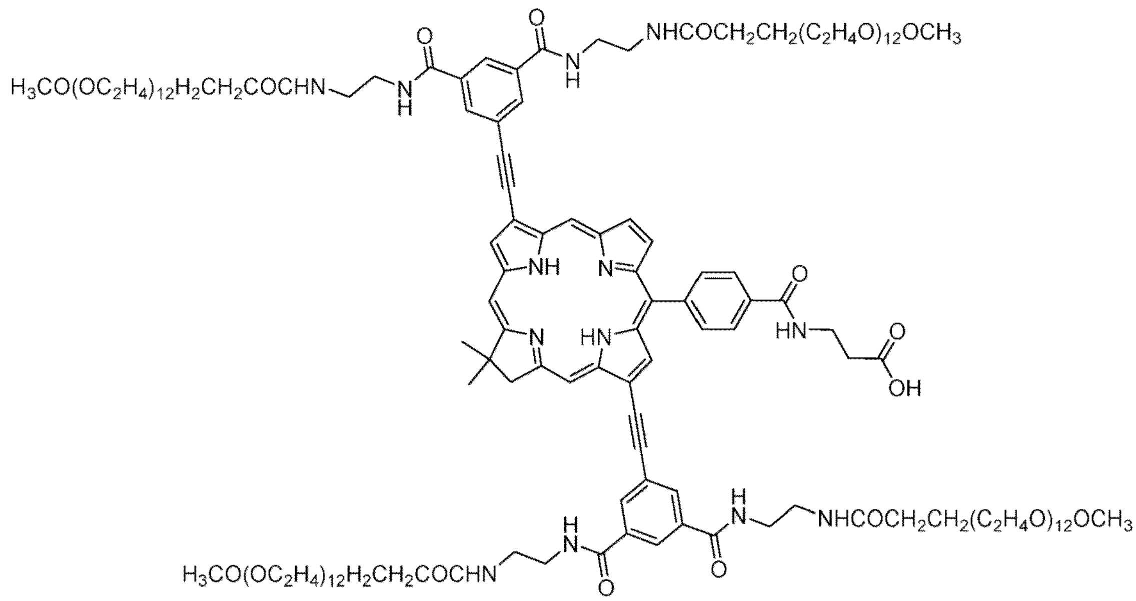

oder ein Konjugat davon ist, wobei die Verbindung an ein oder mehrere Elemente aus der Gruppe konjugiert ist, die aus einem kleinen Molekül, einem Antigen, einem Mikropartikel, einem Nanopartikel, einem Polymer, einem Peptid, einem Protein, einem Antikörper oder einem Antikörperfragment, einer Nukleinsäure, einem Hormon und einem Wachstumsfaktor besteht.

**7.** Zusammensetzung, umfassend ein kovalentes Konjugat, das gebildet ist zwischen:

(a) einer Verbindung mit der Formel (I) nach Anspruch 1, vorausgesetzt, dass wenigstens einer von $R_2$, $R_3$, $R_5$, $R_{10}$, $R_{12}$, $R_{13}$ und $R_{15}$ eine Verknüpfungsgruppe ist; und
(b) einem oder mehreren Elementen aus der Gruppe, die aus einem kleinen Molekül, einem Antigen, einem Mikropartikel, einem Nanopartikel, einem Polymer, einem Peptid, einem Protein, einem Antikörper oder einem Antikörperfragment, einer Nukleinsäure, einem Hormon und einem Wachstumsfaktor besteht.

**8.** Pharmazeutische Zusammensetzung, umfassend eine Verbindung nach Anspruch 1 oder ein Konjugat nach Anspruch 7 und einen pharmazeutisch akzeptablen Träger.

**9.** Verfahren zum Erkennen eines Ziels, wobei das Ziel eine Verbindung, eine Zelle oder ein Partikel ist, wobei das Verfahren ein Markieren des Ziels mit einem Konjugat nach Anspruch 7 umfasst, wobei das Verfahren vorzugsweise die Verwendung der Durchflusszytometrie umfasst.

**10.** Verfahren zum Abbilden einer Zelle, eines Gewebes oder eines Organismus, wobei das Verfahren die Verwendung einer Verbindung nach Anspruch 1 oder eines Konjugats nach Anspruch 7 umfasst.

**11.** Verbindung zur Verwendung in einem Verfahren zur Behandlung einer Krankheit bei einem Patienten, der eine Behandlung dafür benötigt, wobei die Verbindung eine Verbindung nach Anspruch 1, ein Konjugat nach Anspruch 7 oder eine pharmazeutische Zusammensetzung nach Anspruch 8 ist, wobei das Verfahren Folgendes umfasst:

Verabreichen einer Verbindung nach Anspruch 1, eines Konjugats nach Anspruch 7 oder einer pharmazeutischen Zusammensetzung nach Anspruch 8 an den Patienten und
Bestrahlen von wenigstens einem Bereich des Patienten mit Licht,
wobei die Krankheit optional eine Krankheit mit Hyperproliferation ist, wobei die Krankheit optional ferner Krebs ist.

**12.** Verbindung nach einem der Ansprüche 1 bis 6 mit einer Löslichkeit von über ungefähr 1 mg/ml in einer wässrigen Lösung, optional mit einer Löslichkeit von mindestens ungefähr 2,5 mg/ml in einer wässrigen Lösung, optional ferner

mit einer Löslichkeit von mindestens ungefähr 10 mg/ml in einer wässrigen Lösung.

**13.** Verfahren zum Herstellen eines synthetischen Zwischenprodukts einer Verbindung mit der Formel (I):

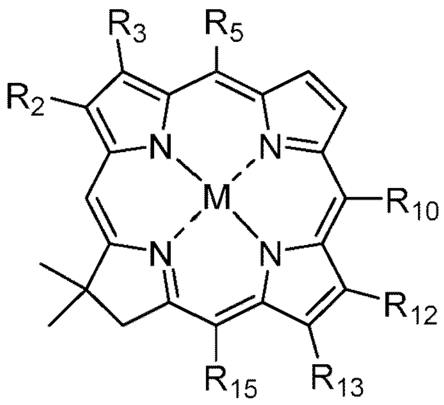

(I)

,

in der:

M ein Metall ist oder-H, -H;
$R_5$ und $R_{15}$ unabhängig aus H, Alkoxy und einer Verknüpfungsgruppe mit folgender Formel ausgewählt sind:

$$-L_1-(X_1-L_2)_P-G;$$

in der p 0 oder 1 ist; $L_1$ Alkyliden ist; $X_1$ -C(=O)NH- oder -NHC(=O)- ist; $L_2$ - $(CH_2CH_2CO)_q$-Alkylen- ist, wobei q eine ganze Zahl zwischen 1 und 24, Alkylen oder ein substituiertes Alkylen ist, wobei optional das substituierte Alkylen ein Alkylen ist, das mit einer oder mehreren Gruppen, die eine Polyoxyethylenkette umfasst bzw umfassen, und/oder einer Amidgruppe substituiert ist; und G eine zur Biokonjugation fähige Gruppe ist;
$R_{10}$

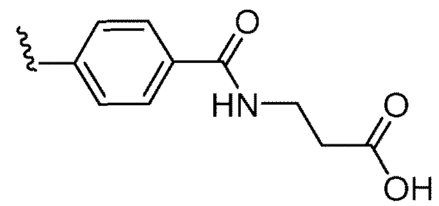

ist; und
$R_2$, $R_3$, $R_{12}$ und $R_{13}$ unabhängig ausgewählt sind aus H, Halogen, Cyano, Perhaloalkyl, Sulfonat, Sulfonamid, Ester, Carbonsäure, Formyl, Acetyl, einer Verknüpfungsgruppe mit der Formel $-L_1-(X_1-L_2)_P-G$ und einer solubilisierenden Gruppe, wobei die solubilisierende Gruppe ausgewählt ist aus -Aryl-$(R_s)_s$ und -Alkinyl-Aryl-$(R_s)_w$, wobei w eine ganze Zahl zwischen 0 und 5 (jeweils einschließlich) ist und $R_s$ eine Gruppe mit folgender Formel ist:

$$-X_2-(L_3)_z-R_{17},$$

in der: z 0 oder 1 ist; $X_2$ $-CH_2NHC(=O)-$, -C(=O)NH-Alkylen-NH- oder Triazolyl ist; $L_3$ -C(=O)-Alkylen-C(=O)-NH- ist und $R_{17}$ ausgewählt ist aus $-(CH_2H_4O)_m-R_{18}$, $-C(=O)C_2H_4-(OC_2H_4)_mOR_{18}$ und - $(C_2H_4O)_n-C_2H_4-C(=O)NH-C(R_{19})_3$, wobei m eine ganze Zahl von mindestens 4 ist, wobei m optional eine ganze Zahl von mindestens 8 ist; n eine ganze Zahl zwischen 1 und 5 ist;
$R_{18}$ ein niederes Alkyl, optional Methyl ist und $R_{19}$ $-CH_2O-C_2H_4-C(=O)NH-(C_2H_4O)_mR_{18}$ ist;
vorausgesetzt, dass wenigstens einer von $R_2$, $R_3$, $R_{12}$ und $R_{13}$ -Aryl-$(R_s)_w$ oder -Alkinyl-Aryl-$(R_s)_w$ ist, wobei das Verfahren Folgendes umfasst:

(a) Bereitstellen einer Verbindung mit der Formel (I'):

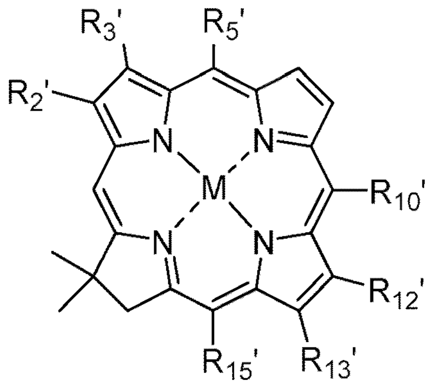

in der:

M ein Metall ist oder -H, -H;

$R_5$, und $R_{15}$' unabhängig ausgewählt sind aus H, Alkoxy,

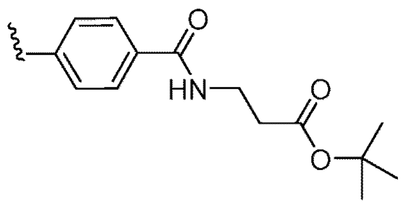, und 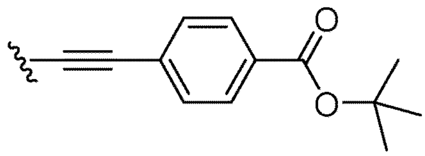;

$R_{10}$'

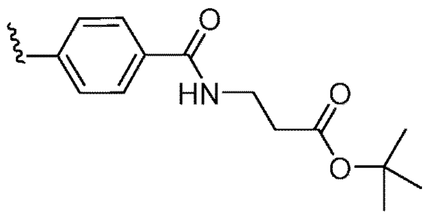

ist; und

$R_2$', $R_3$', $R_{12'}$ und $R_{13}$' unabhängig ausgewählt sind aus H, Ester, Carbonsäure, Formyl, Acetyl,

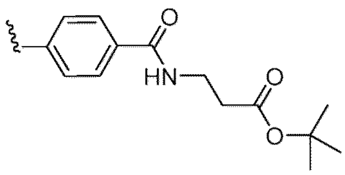, 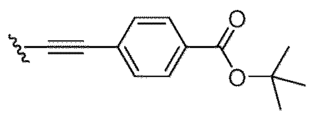,

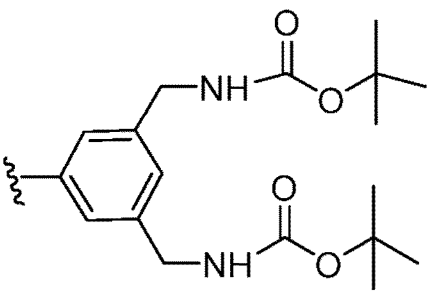 und 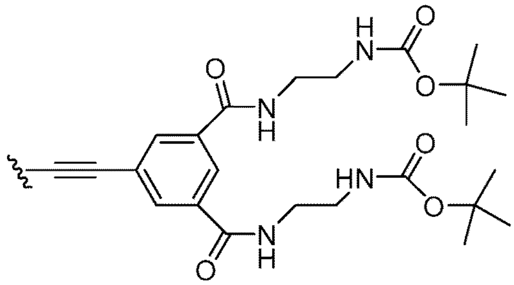;

vorausgesetzt, dass wenigstens einer von $R_2$', $R_3$', $R_{12'}$ und $R_{13}$'

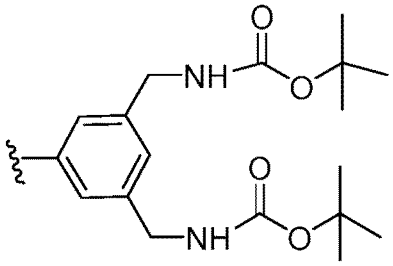

oder

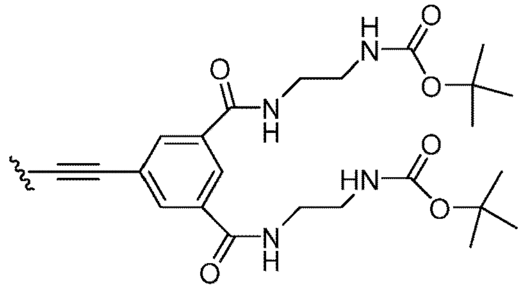

;

ist und

(b) Kontaktieren der Verbindung, die in Schritt (a) bereitgestellt ist, mit einer Lösung, die 4-molare (M) HCl in Dioxan umfasst, und damit Bereitstellen einer Verbindung mit der Formel (I''):

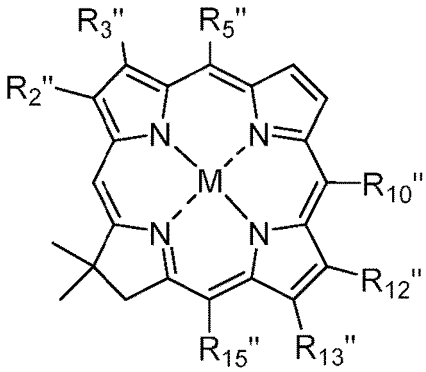

in der:

M ein Metall ist oder -H, -H;
$R_{5''}$ und $R_{15''}$ unabhängig ausgewählt sind aus H, Alkoxy,

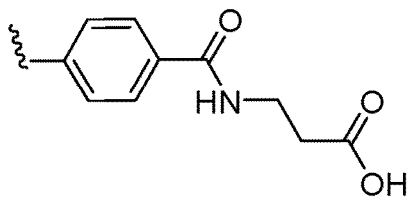

und

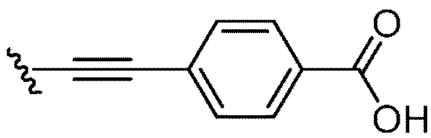

;

$R_{10''}$

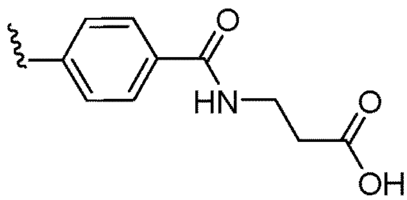

ist; und
$R_2$'', $R_3$'', $R_{12''}$ und $R_{13}$'' unabhängig ausgewählt sind aus H, Ester, Carbonsäure, Formyl, Acetyl,

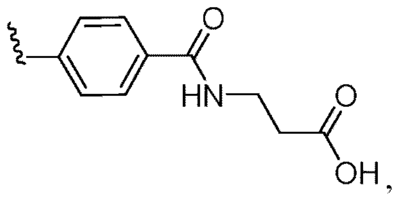
,
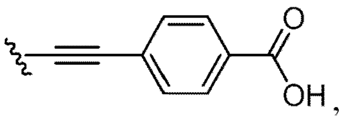
,

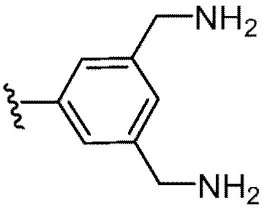
und
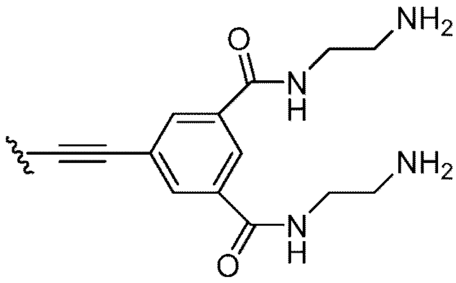
;

vorausgesetzt, dass wenigstens einer von $R_2$", $R_3$", $R_{12"}$ und $R_{13}$"

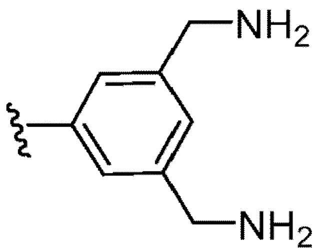
oder
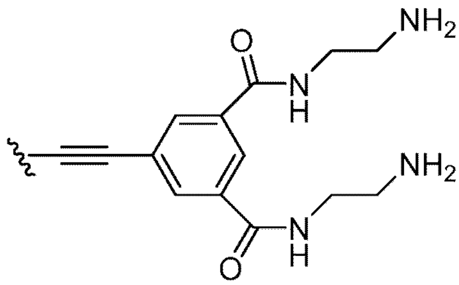
.

ist.

## Revendications

**1.** Composé selon la formule (I) :

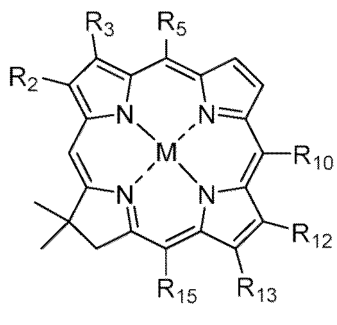
(I)

,

où :

M est un métal ou représente deux atomes d'hydrogène (-H, -H) ;

$R_5$ et $R_{15}$ sont indépendamment choisis parmi un atome H, un groupement alcoxy et un groupement lieur ayant la formule :

$$-L_1-(X_1-L_2)_P-G ;$$

où p est égal à 0 ou à 1 ; $L_1$ est un groupement alcylidène ; $X_1$ est un groupement -C(=O)NH- ou -NHC(=O)- ; $L_2$ est un groupement $-(CH_2CH_2O)q$-alcylène-, où q est un nombre entier entre 1 et 24, un groupement alcylène, ou un groupement alcylène substitué, éventuellement, où le groupement alcylène substitué est un groupement alcylène substitué par un ou plusieurs groupements comprenant une chaine poly oxyéthylène et/ou un groupement amide ; et G est un groupement bioconjugable ; $R_{10}$ est :

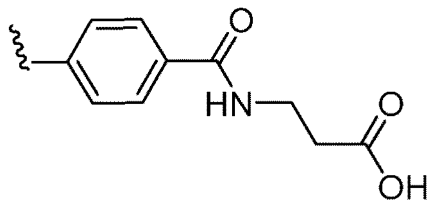

;

et

$R_2$, $R_3$, $R_{12}$ et $R_{13}$ sont indépendamment choisis parmi un atome H, un groupement cyano, halogénure, perhaloalkyle, sulfonate, sulfonamide, ester, acide carboxylique, formyle, acétyle, un groupement lieur ayant la formule $-L_1-(X_1-L_2)_P-G$ et un groupement de solubilisation, où le groupement de solubilisation est choisi parmi des groupements -aryl-$(R_5)_w$ et -alcynyl-aryl-$(R_5)_w$, où w est un nombre entier entre 0 et 5 inclus, et $R_5$ est un groupement ayant la formule :

$$-X_2-(L_3)_z-R_{17},$$

où : z est égal à 0 ou à 1 ; $X_2$ est un groupement $-CH_2NHC(=O)-$, -C(=O)NH-alcylène-NH- ou un groupement triazolyle ; $L_3$ est un groupement -C(=O)-alcylène-C(=O)-NH- et $R_{17}$ est choisi parmi les groupements $-(C_2H_4O)m-R_{18}$, $-C(=O)C_2H_4-(OC_2H_4)_mOR_{18}$ et $-(C_2H_4O)_n-C_2H_4-C(=O)NH-C(R_{19})_3$, où m est un nombre entier égal à 4 ou plus, éventuellement, où m est un nombre entier égal à 8 ou plus ; n est un nombre entier entre 1 et 5 ; $R_{18}$ est un groupement alkyle inférieur, éventuellement méthyle ; et $R_{19}$ est un groupement $-CH_2O-C_2H_4-C(=O)NH-(C_2H_4O)_mR_{18}$ ;

sous réserve qu'au moins un parmi $R_2$, $R_3$, $R_{12}$ et $R_{13}$ soit un groupement -aryl-$(R_5)_w$ ou -alcynyl-aryl-$(R_5)_w$.

**2.** Composé selon la revendication 1, dans lequel $R_5$ et $R_{15}$ sont choisis parmi un atome H, un groupement méthoxy et un groupement lieur ayant la formule :

$$-L_1-(X_1-L_2)_P-G.$$

**3.** Composé selon la revendication 1 ou la revendication 2, dans lequel $R_3$ et $R_{13}$ sont chacun un groupement -aryl-$(R_5)_w$, éventuellement dans lequel $R_3$ et $R_{13}$ sont chacun un groupement -phényl-$(R_5)_2$.

**4.** Composé selon la revendication 3, dans lequel chaque $R_5$ a une formule $-X_2-(L_3)_z-R_{17}$, où : z est égal à 0, $X_2$ est le groupement $-CH_2NHC(=O)-$ et $R_{17}$ est le groupement $-(C_2H_4O)m-R_{18}$, où m est un nombre entier entre 12 et 24.

**5.** Composé selon la revendication 4, dans lequel $R_3$ et $R_{13}$ sont chacun :

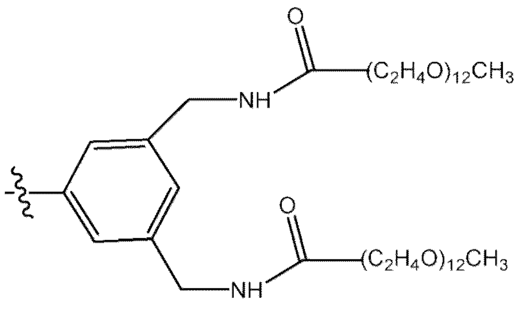

.

**6.** Composé selon l'une quelconque des revendications 1 à 4, le composé étant :

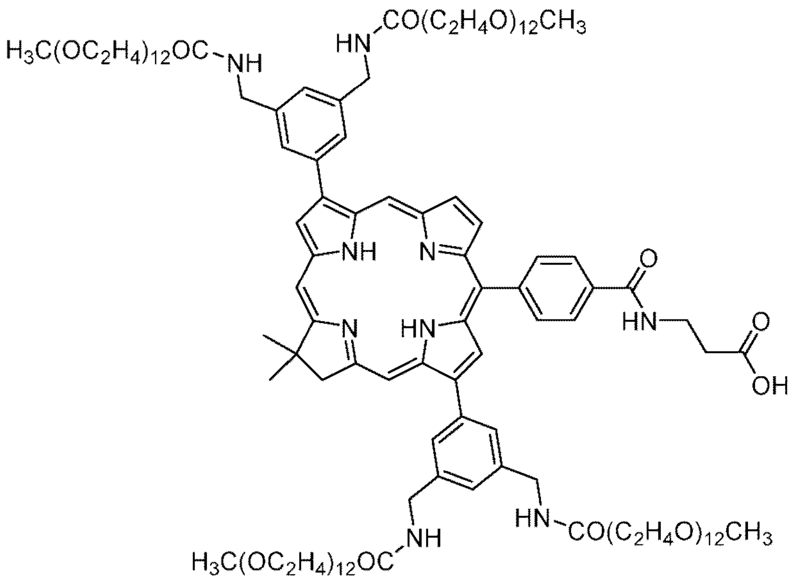

ou

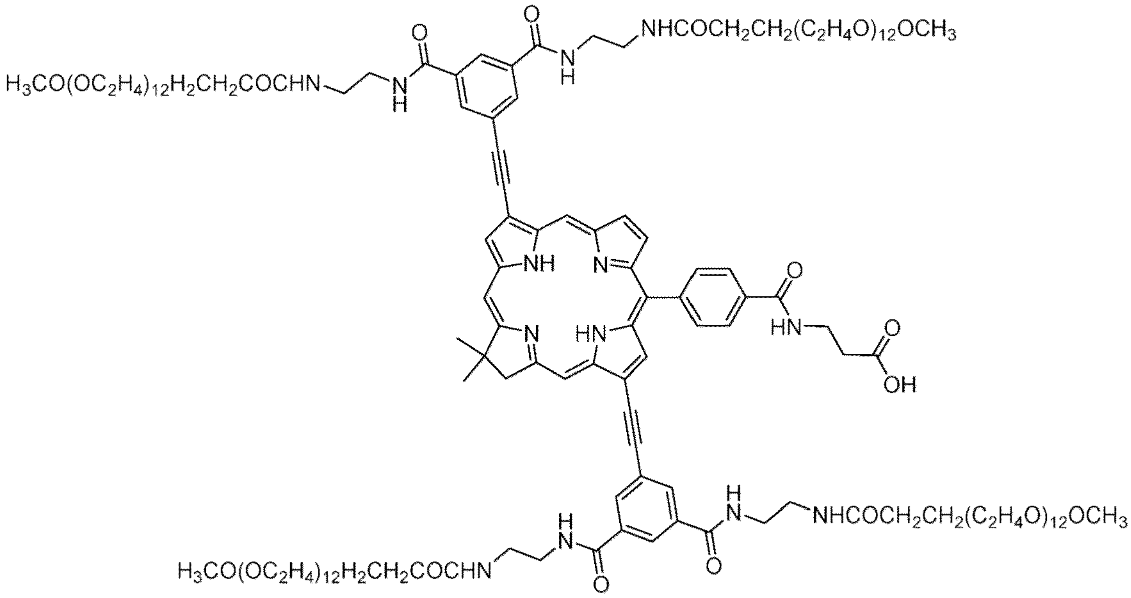

ou un conjugué de celui-ci, le composé étant conjugué avec un ou plusieurs des groupes constitué d'une petite molécule, d'un antigène, d'une microparticule, d'une nanoparticule, d'un polymère, d'un peptide, d'une protéine, d'un anticorps ou d'un fragment d'anticorps, d'un acide nucléique, d'une hormone et d'un facteur de croissance.

**7.** Composition comprenant un conjugué covalent formé entre :

(a) un composé selon la formule (I) tel que défini en revendication 1 sous réserve qu'au moins un parmi $R_2$, $R_3$, R, $R_{10}$, $R_{12}$, $R_{13}$ et $R_{15}$ soit un groupement lieur ; et
(b) un ou plusieurs éléments du groupe constitué d'une petite molécule, d'un antigène, d'une microparticule, d'une nanoparticule, d'un polymère, d'un peptide, d'une protéine, d'un anticorps ou d'un fragment d'anticorps, d'un acide nucléique, d'une hormone et d'un facteur de croissance.

**8.** Composition pharmaceutique comprenant un composé selon la revendication 1 ou un conjugué selon la revendication 7, et un support pharmaceutiquement acceptable.

**9.** Procédé de détection d'une cible, où ladite cible est un composé, une cellule ou une particule, le procédé comprenant un étiquetage de la cible avec un conjugué selon la revendication 7, de préférence, le procédé comprenant l'utilisation de la cytométrie en flux.

**10.** Procédé d'imagerie d'une cellule, d'un tissu ou d'un organisme, le procédé comprenant l'utilisation d'un composé selon la revendication 1 ou d'un conjugué selon la revendication 7.

**11.** Composé destiné à une utilisation dans un procédé de traitement d'une maladie chez un sujet nécessitant le traitement, le composé étant un composé selon la revendication 1, un conjugué selon la revendication 7 ou une composition pharmaceutique selon la revendication 8, le procédé comprenant :

l'administration au sujet d'un composé selon la revendication 1, d'un conjugué selon la revendication 7 ou d'une composition pharmaceutique selon la revendication 8 ;
et l'irradiation d'au moins une partie du sujet avec de la lumière,
éventuellement, où ladite maladie est une maladie hyper proliférative, éventuellement encore où la maladie est le cancer.

**12.** Composé selon l'une quelconque des revendications 1 à 6 ayant une solubilité supérieure à environ 1 mg/ml dans une solution aqueuse, ayant éventuellement une solubilité d'environ 2,5 mg/ml ou plus dans une solution aqueuse ; éventuellement encore ayant une solubilité d'environ 10 mg/ml ou plus dans une solution aqueuse.

**13.** Procédé de préparation d'un intermédiaire synthétique d'un composé selon la formule (I) :

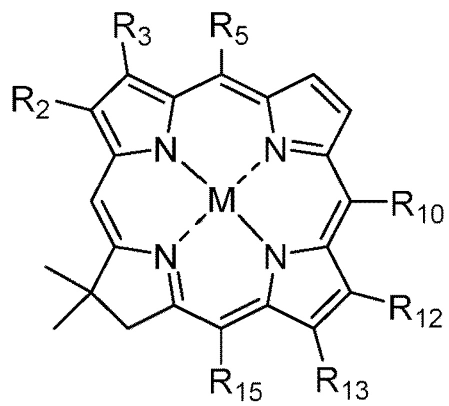
(I)

,

où :

M est un métal ou est un atome -H, -H ;
$R_5$ et $R_{15}$ sont indépendamment choisis parmi un atome H, un groupement alcoxy et un groupement lieur ayant la formule :

$$-L_1-(X_1-L_2)_P-G ;$$

où p est égal à 0 ou à 1 ; $L_1$ est un groupement alcylidène ;
$X_1$ est un groupement -C(=O)NH- ou -NHC(=O)- ; L2 est un groupement $-(CH_2CH_2O)q$-alcylène-, où q est un nombre entier entre 1 et 24, un groupement alcylène ou alcylène substitué, éventuellement où le groupement alcylène substitué est un groupement alcylène substitué par un ou plusieurs groupes comprenant une chaine poly oxyéthylène et/ou un groupement amide ; et G est un groupement bioconjugable ;
$R_{10}$ est :

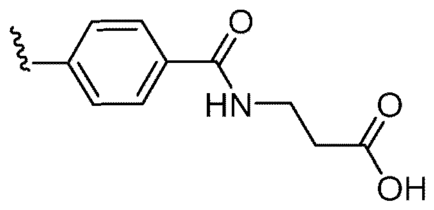

et

$R_2$, $R_3$, $R_{12}$ et $R_{13}$ sont indépendamment choisis parmi un atome H, un groupement halogénure, cyano, perhaloalkyle, sulfonate, sulfonamide, ester, acide carboxylique, formyle, acétyle, un groupement lieur ayant la formule $-L_1-(X_1-L_2)_P-G$ et un groupement de solubilisation, où le groupement de solubilisation est choisi parmi des groupements $-aryl-(R_5)_w$ et $-alcynyl-aryl-(R_5)_w$, où w est un nombre entier entre 0 et 5 inclus, et $R_5$ est un groupement ayant la formule :

$$-X_2-(L_3)_z-R_{17},$$

où : z est égal à 0 ou à 1 ; $X_2$ est un groupement $-CH_2NHC(=O)-$, -C(=O)NH-alcylène-NH-, ou un groupement triazolyle ; $L_3$ est un groupement -C(=O)-alcylène-C(=O)-NH- et $R_{17}$ est choisi parmi les groupements $-(C_2H_4O)m-R_{18}$, $-C(=O)C_2H_4-(OC_2H_4)_mOR_{18}$ et $-(C_2H_4O)"-C_2H_4-C(=O)NH-C\ (R_{19})_3$, où m est un nombre entier égal à 4 ou plus, éventuellement où m est un nombre entier égal à 8 ou plus ; n est un nombre entier entre 1 et 5 ; $R_{18}$ est un groupement alkyle léger, éventuellement méthyle ; et $R_{19}$ est un groupement $-CH_2OC_2H_4-C(=O)NH-(C_2H_4O)_mR_{18}$ ;

sous réserve qu'au moins un parmi $R_2$, $R_3$, $R_{12}$ et $R_{13}$ soit un groupement $-aryl-(R_5)_w$ ou $-alcynyl-aryl-(R_5)_{w;}$ le procédé comprenant :

(a) la fourniture d'un composé ayant la formule (I') :

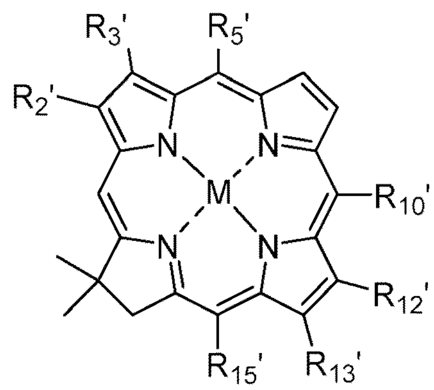

où :

M est un métal ou est un atome -H, -H ;

$R_5$, et $R_{15}$' sont indépendamment choisis parmi un atome H, un groupement alcoxy,

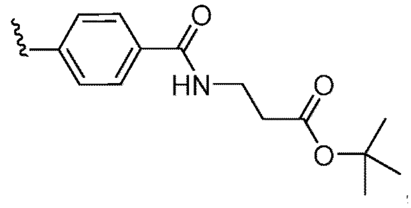

, et

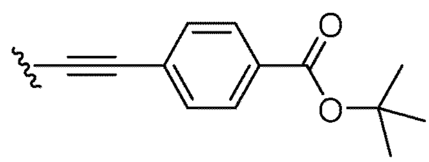

;

$R_{10}$' est :

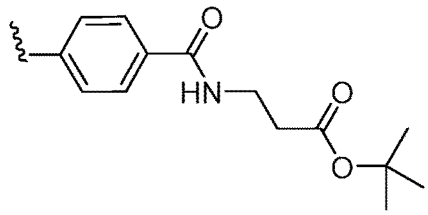

et;

$R_2$', $R_3$', $R_{12'}$ et $R_{13}$' sont indépendamment choisis parmi un atome H, un groupement ester, acide carboxylique, formyle, acétyle,

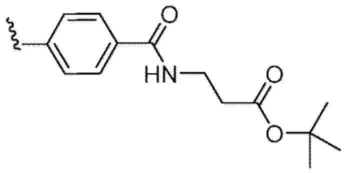,
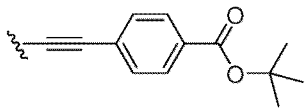,

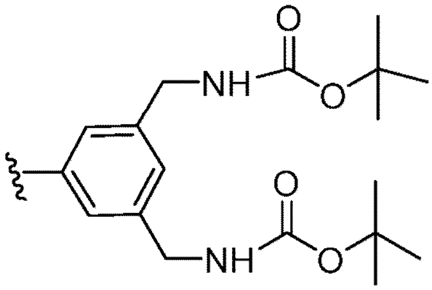
et
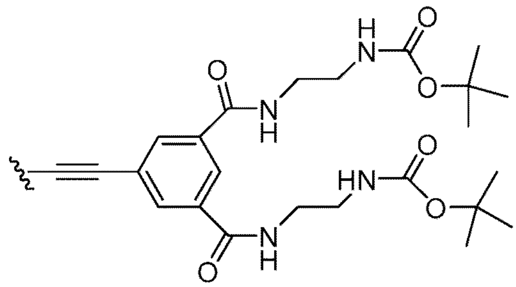
;

sous réserve qu'au moins un parmi $R_2$', $R_3$', $R_{12'}$ et $R_{13'}$ soit

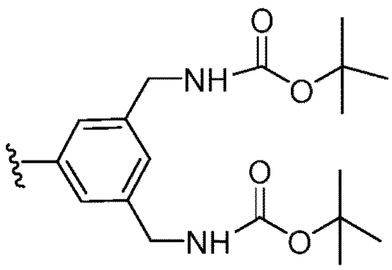
ou
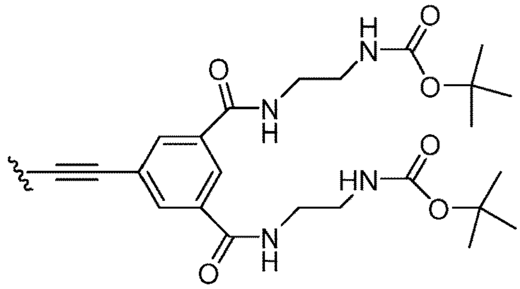
;

et

(b) la mise en contact du composé fourni dans l'étape (a) avec une solution comprenant de l'HCl 4 molaire (M) dans du dioxane pour fournir un composé selon la formule (I") :

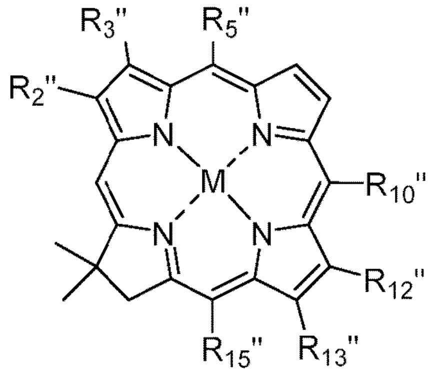

où :

M est un métal ou est un atome -H, -H ;

$R_{5"}$ et $R_{15"}$ sont indépendamment choisis parmi un atome H, un groupement alcoxy,

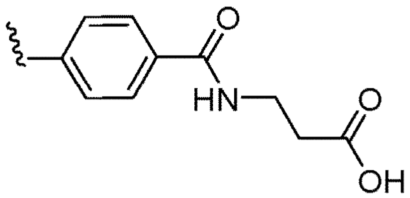 et 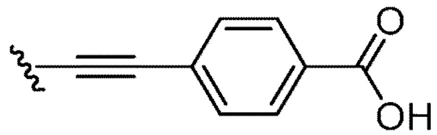 ;

$R_{10"}$ est

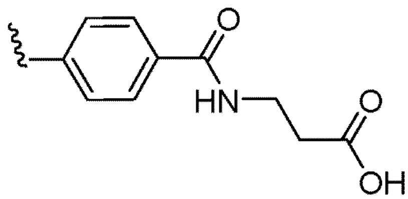 ;

et

$R_2"$, $R_3"$, $R_{12"}$ et $R_{13}"$ sont indépendamment choisis parmi un atome H, un groupement ester, acide carboxylique, formyle, acétyle,

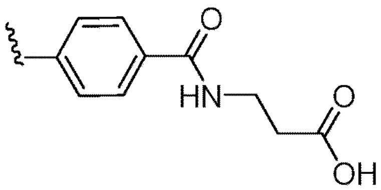, 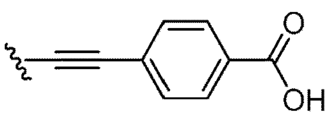,

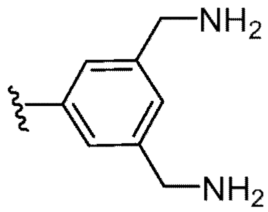 et 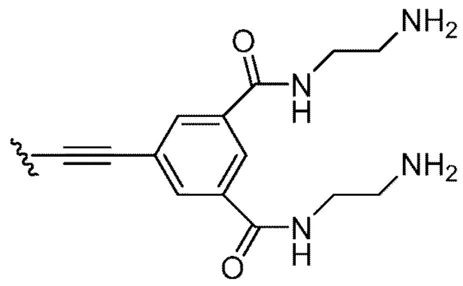 ;

sous réserve qu'au moins parmi $R_2$", $R_3$", $R_{12"}$ et $R_{13}$" soit

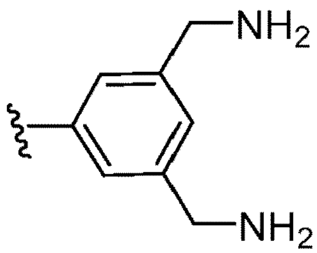 ou 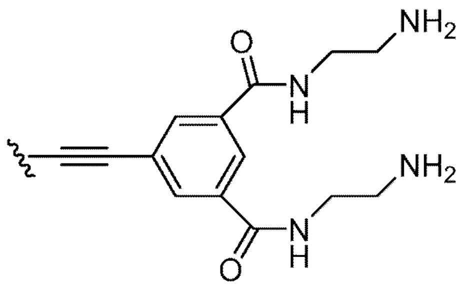 .

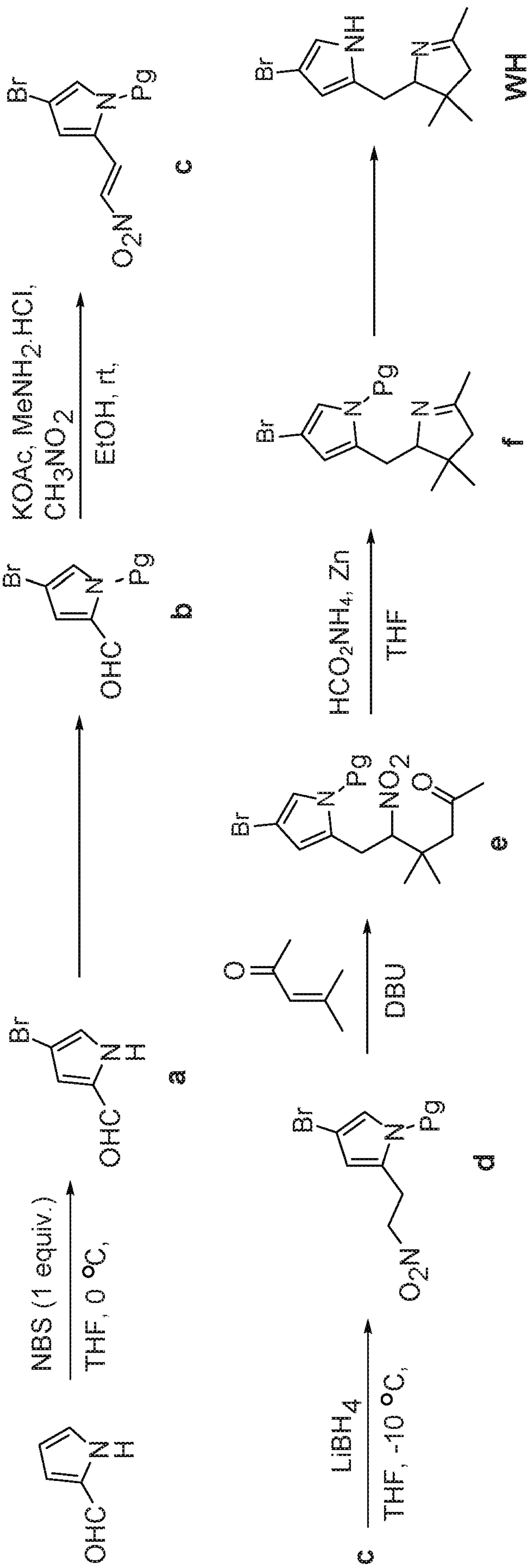
NBS (1 equiv.)
THF, 0 °C,
a
b
KOAc, MeNH2.HCl, CH3NO2
EtOH, rt,
c
c
LiBH4
THF, -10 °C,
d
DBU
e
HCO2NH4, Zn
THF
f
WH

*FIG. 1*

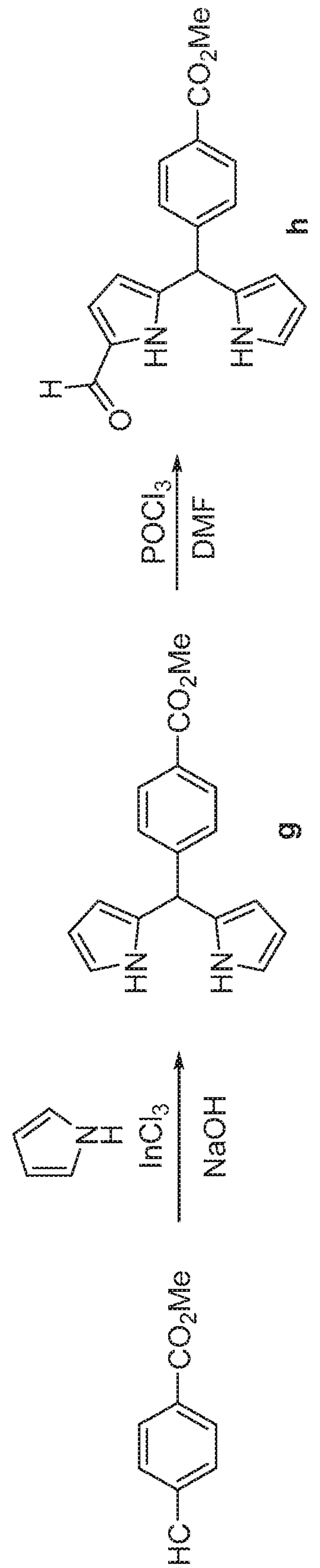


g

h

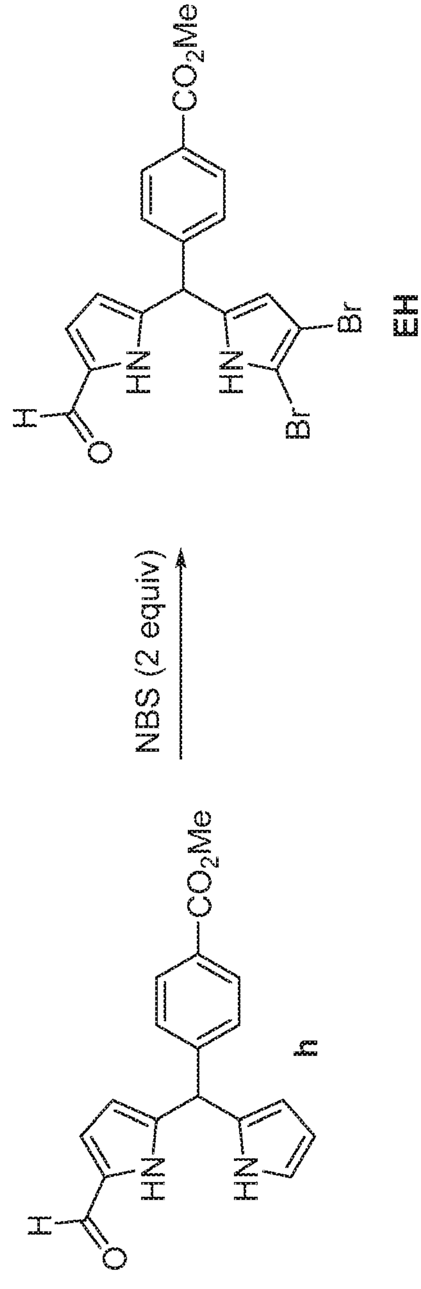


h

EH

*FIG. 2*

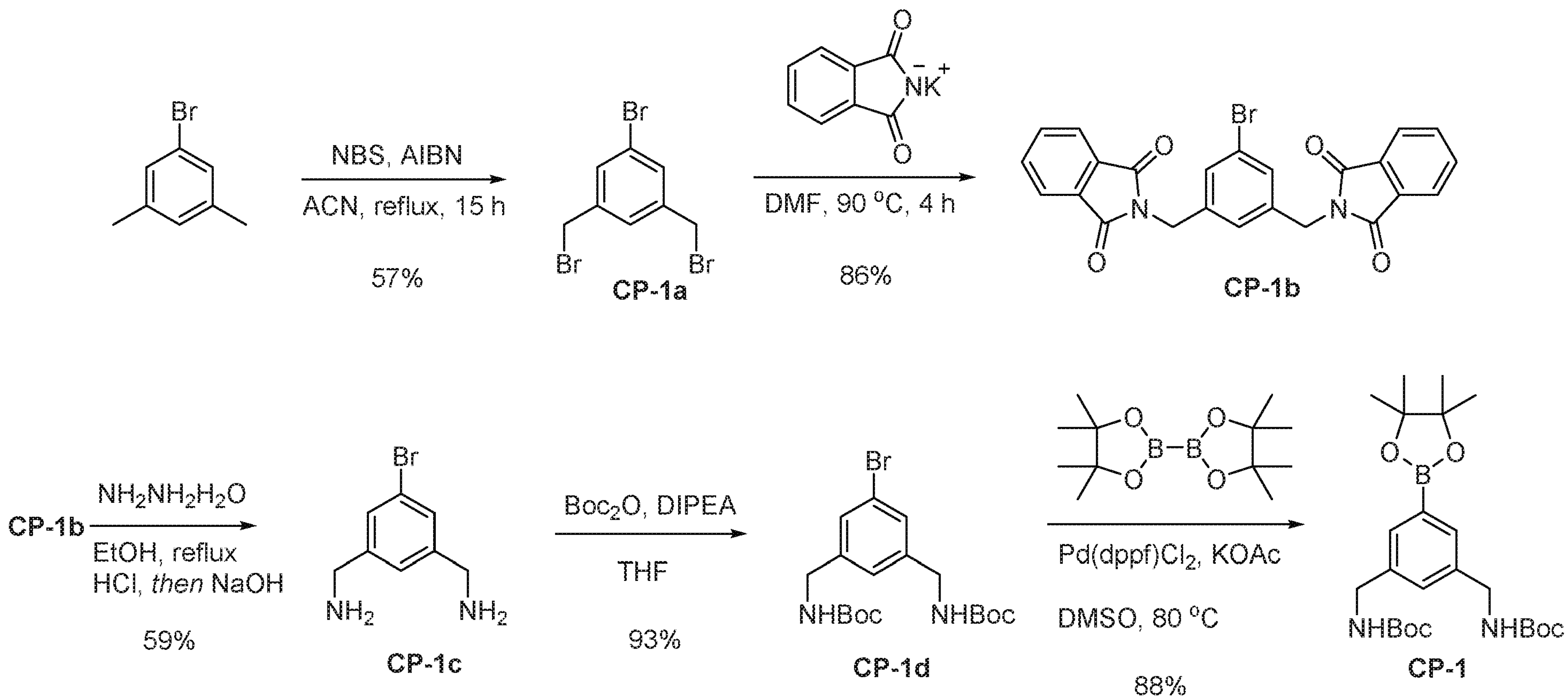


CP-1a

CP-1b

CP-1b

CP-1c

CP-1d

CP-1

*FIG. 3*

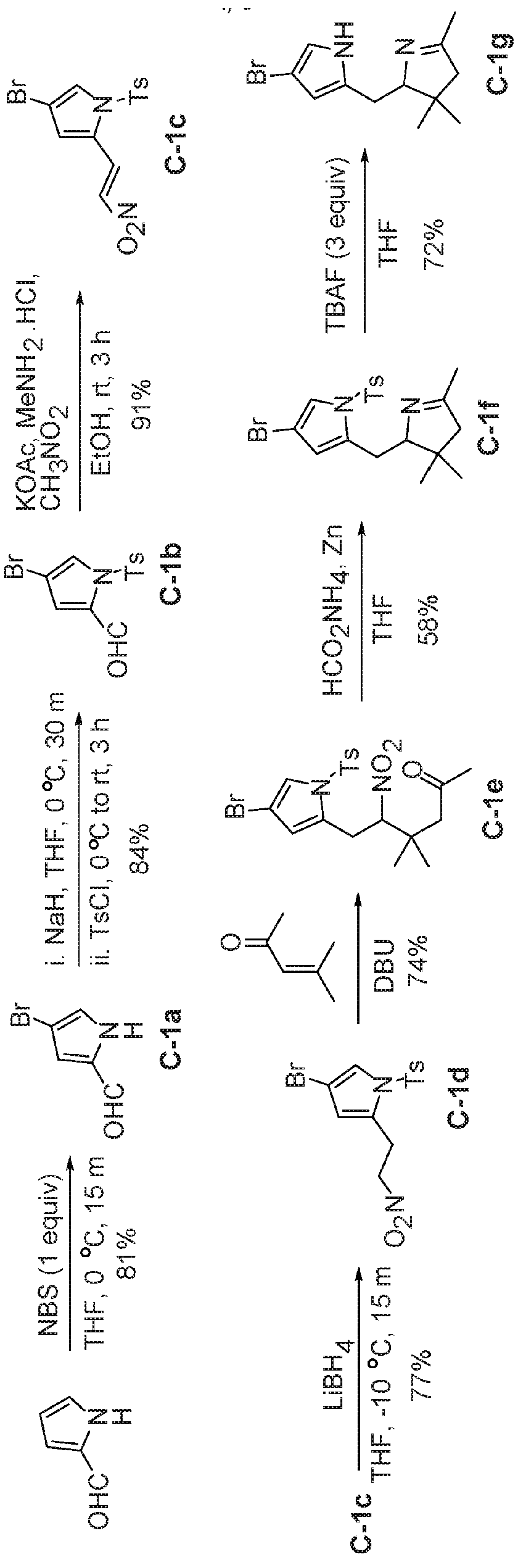
NBS (1 equiv)
THF, 0 °C, 15 m
81%
C-1a
i. NaH, THF, 0 °C, 30 m
ii. TsCl, 0 °C to rt, 3 h
84%
C-1b
KOAc, $MeNH_2$.HCl, $CH_3NO_2$
EtOH, rt, 3 h
91%
C-1c
C-1c
$LiBH_4$
THF, -10 °C, 15 m
77%
C-1d
DBU
74%
C-1e
$HCO_2NH_4$, Zn
THF
58%
C-1f
TBAF (3 equiv)
THF
72%
C-1g

*FIG. 4*

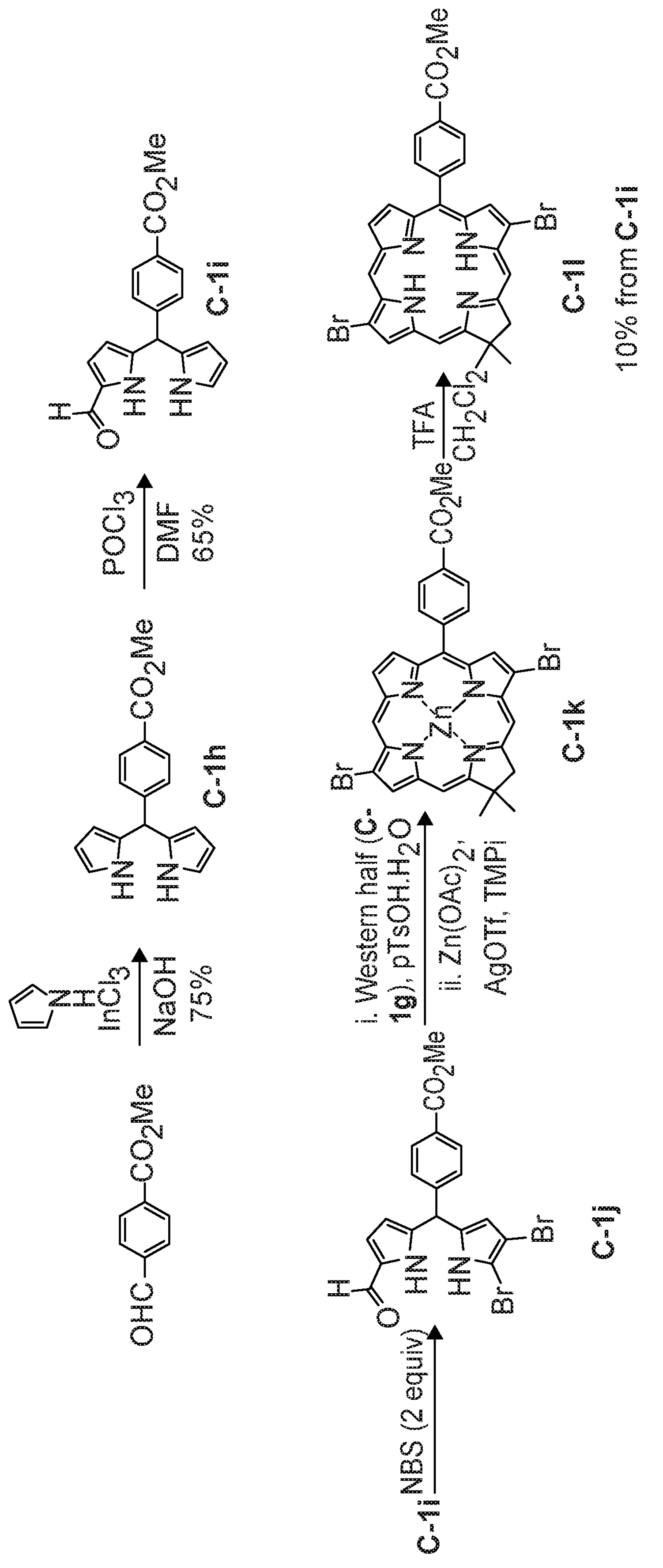
InCl3
NaOH
75%
C-1h
POCl3
DMF
65%
C-1i
C-1i
NBS (2 equiv)
C-1j
i. Western half (C-1g), pTsOH.H2O
ii. Zn(OAc)2, AgOTf, TMPi
C-1k
TFA
CH2Cl2
C-1l
10% from C-1i

*FIG. 5*

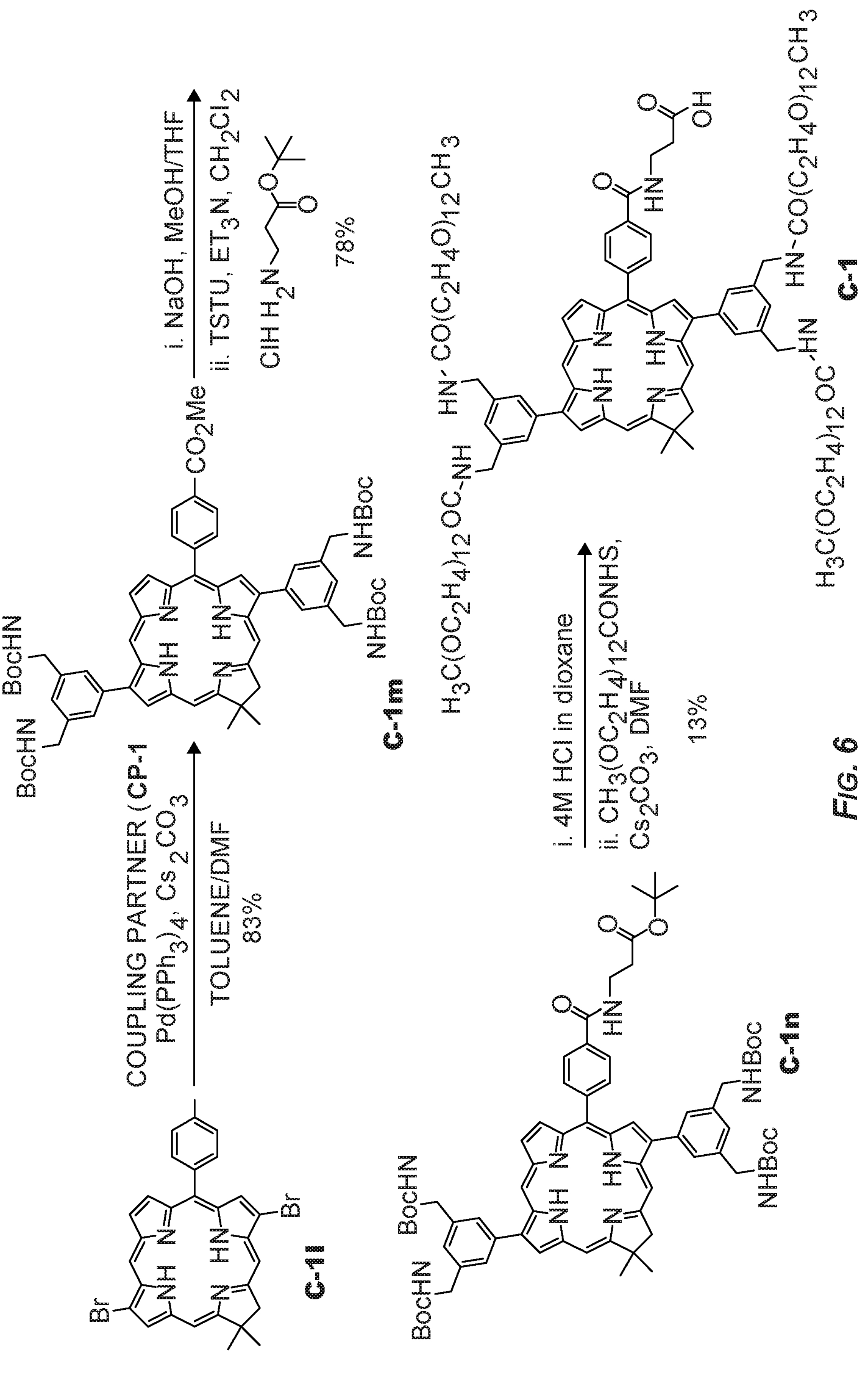


C-1l

C-1m

C-1n

C-1

*FIG. 6*

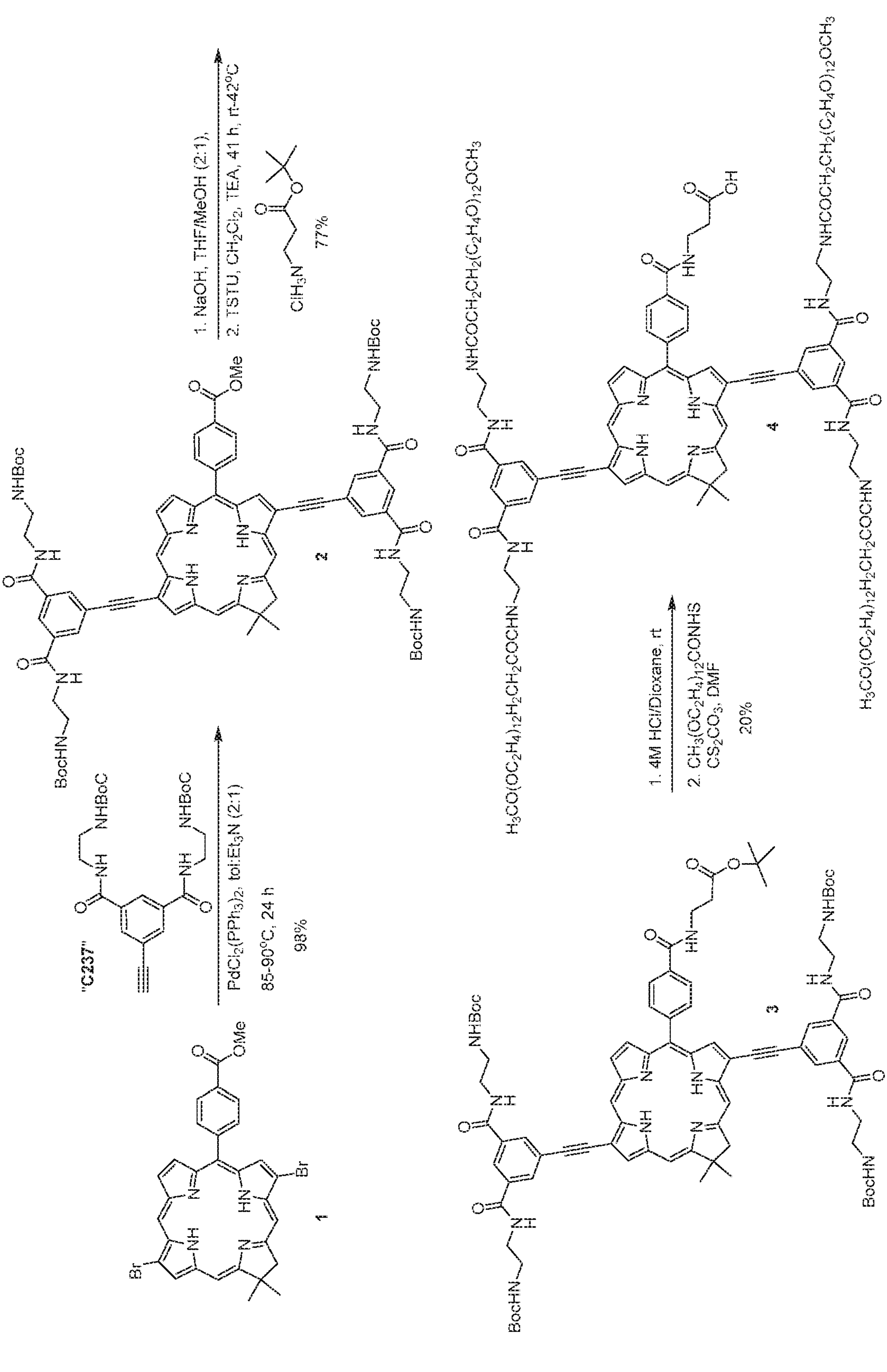
"C237"
PdCl2(PPh3)2, tol:Et3N (2:1)
85-90°C, 24 h
98%
1. NaOH, THF/MeOH (2:1),
2. TSTU, CH2Cl2, TEA, 41 h, rt-42°C
77%
1. 4M HCl/Dioxane, rt
2. CH3(OC2H4)12CONHS
CS2CO3, DMF
20%
H3CO(OC2H4)12H2CH2COCHN
NHCOCH2CH2(C2H4O)12OCH3
1
2
3
4

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017214637 A1
- US 6946533 B
- WO 199222653 A
- US 6273904 B
- US 8212055 B
- US 8980565 B
- US 6559374 B
- US 20040044197, Pandey
- US 5952366 A, Pandey
- US 4328245 A
- US 4409239 A
- US 4410545 A
- US RE28819 E
- US 4358603 A
- US 3710795 A
- US 4044126 A
- US 4414209 A
- US 4364923 A
- US 6267983 B
- US 6261595 B
- US 6256533 B
- US 6167301 A
- US 6024975 A
- US 6010715 A
- US 5985317 A
- US 5983134 A
- US 5948433 A
- US 5860957 A
- US 6316652 B
- US 6274552 B
- US 6271359 B
- US 6253872 B
- US 6139865 A
- US 6131570 A
- US 6120751 A
- US 6071495 A
- US 6060082 A
- US 6048736 A
- US 6039975 A
- US 6004534 A
- US 5985307 A
- US 5972366 A
- US 5900252 A
- US 5840674 A
- US 5759542 A
- US 5709874 A
- US 4522811 A
- US 3927193 A
- US 4331647 A
- US 4348376 A
- US 4361544 A
- US 4468457 A
- US 4444744 A
- US 4818709 A
- US 4624846 A
- US 4474893 A
- US 4479895 A
- US 4946778 A
- US 4374925 A
- US 3817837 A
- EP 0243929 B1
- US 5323907 A
- US 5052558 A
- US 5033252 A
- US 5059415 A
- US 5167926 A
- US 5915925 A
- US 6248590 B
- US 6589792 B
- US 6890487 B


### Non-patent literature cited in the description


- **KEE HOOI LING et al.** *PHOTOCHEMISTRY AND PHOTOBIOLOGY,* 2007, vol. 83 (5), 1110-1124
- **KEE HOOI LING et al.** *PHOTOCHEMISTRY AND PHOTOBIOLOGY,* 2009, vol. 85 (4), 909-920
- **YUMITA et al.** *Cancer Letters,* 1997, vol. 112, 79-86
- **UMEMURA et al.** *Ultrasonics Sonochemistry,* 1996, vol. 3, S187-S191
- **YUMITA et al.** *Japanese Journal of Hyperthermic Oncology,* 1987, vol. 3 (2), 175-182
- **KRAYER et al.** *Journal of Porphyrins and Phthalocyanines,* 2009, vol. 13 (10), 1098-1110
- **JIANG et al.** *Organic & Biomolecular Chemistry,* 2014, vol. 12, 86-103
- **LAHA et al.** *Organic Process Research & Development,* 2003, vol. 7 (6), 799-812
- **YU et al.** *Journal of Organic Chemistry,* 2013, vol. 78 (21), 10678-10691

- **LIU et al.** *New Journal of Chemistry,* 2016, vol. 40 (9), 7721-7740
- **JIANG et al.** *New Journal of Chemistry,* 2015, vol. 39 (7), 5694-5714
- **ZHANG et al.** *New Journal of Chemistry,* 2016, vol. 40 (9), 7750-7767
- **NOGRADY.** Medicinal Chemistry A Biochemical Approach. Oxford University Press, 1985, 388-392
- **ANSEL.** Introduction to Pharmaceutical Dosage Forms,. Lea & Febiger, 1985, 126
- Remington's Pharmaceutical Sciences. Mack Publishing Company, 1975
- **POLIN.** *European Journal of Clinical Microbiology,* 1984, vol. 3 (5), 387-398
- **YOSHIDA et al.** *Science,* 1980, vol. 207, 71-73
- **KASPER et al.** *Journal of Immunology,* 1982, vol. 129, 1694-1699
- **SIMPSON et al.** *Parasitology,* 1981, vol. 83, 163-177
- **SMITH et al.** *Parasitology,* 1982, vol. 84, 83-91
- **GRYZCH et al.** *Journal of Immunology,* 1982, vol. 129, 2739-2743
- **ZODDA et al.** *Journal of Immunology,* 1982, vol. 129, 2326-2328
- **DISSOUS et al.** *Journal of Immunology,* 1982, vol. 129, 2232-2234
- **MILSTEIN et al.** *Immunology Today,* 1984, vol. 5, 299
- **RAKESTRAW et al.** Proceedings of the National Academy of Science of the United States of America. 1990, vol. 87, 4217-4221
- **BODANSKY.** Principles of Peptide Synthesis. Springer & Hermanson, 1993
- Bioconjugate Techniques. Academic Press
- **KARPOVSKY et al.** *Journal of Experimental Medicine,* 1984, vol. 160 (6), 1686-1701
- **LIU et al.** *Proceedings of the National Academy of Science of the United States of America,* 1985, vol. 82 (24), 8648-8652
- **BRENNAN et al.** *Science,* 1985, vol. 229, 81-83
- **GLENNIE et al.** *Journal of Immunology,* 1987, vol. 139, 2367-2375
- **HERMANSON.** Bioconjugate Techniques. Academic Press, 1996
- **MERRIFIELD et al.** *Ciba Foundation Symposium,* 1994, vol. 186, 5-20
- **PYKETT.** *Scientific American,* 1982, vol. 246, 78
- **RUNGE et al.** *American Journal of Radiology,* 1983, vol. 141, 1209
- **PAAJANEN et al.** *Magnetic Resononance in Medicine,* 1990, vol. 13, 38-43
- **LAHA et al.** *J. Org. Chem.,* 2006, vol. 71, 4092-4102
- **MAECKER et al.** *Cytometry A.,* 2004, vol. 62, 169-173